# EUROPEAN PATENT APPLICATION

(11) **EP 4 524 159 A1**
(43) Date of publication of application: **19.03.2025**
(21) Application number: 23826349.5
(22) Date of filing: 19.06.2023
(51) Int. Cl.: C07K 16/28, C12N 15/13, C12N 15/63, A61K 39/395, A61P 35/00

(54) **ANTIBODY AND USE THEREOF**

(30) Priority: 20.06.2022 CN 202210700116
(71) Applicant: Sichuan Huiyu Pharmaceutical Co., Ltd., Neijiang, Sichuan 641000 (CN); Sichuan Huiyu Seacross Pharmaceutical Technology Co., Ltd., Chengdu, Sichuan 610200 (CN)
(72) Inventor: YAN, Wenjun, Neijiang, Sichuan 641000 (CN); ZHANG, Yang, Neijiang, Sichuan 641000 (CN); TANG, Yurui, Neijiang, Sichuan 641000 (CN); BAI, Ju, Neijiang, Sichuan 641000 (CN); DENG, Jian, Neijiang, Sichuan 641000 (CN); WEI, Tao, Neijiang, Sichuan 641000 (CN); DING, Zhao, Neijiang, Sichuan 641000 (CN)
(74) Representative: Murgitroyd & Company
(86) International application number: PCT/CN2023/101086
(87) International publication number: WO 2023/246701

(57) **Abstract**

Disclosed are an anti-CDCP1 antibody or an antigen-binding fragment thereof, a nucleic acid molecule encoding same and a method for preparing same. The anti-CDCP1 antibody or the antigen-binding fragment thereof has a target-binding capacity, a tumor cell-binding capacity, an endocytosis capacity, a capability to inhibit the migration of tumor cells, and druggability. Further disclosed is a conjugate of the antibody or the antigen-binding fragment thereof. Also disclosed are a pharmaceutical composition comprising the antibody or the antigen-binding fragment thereof, and use thereof in preparing a medicament for preventing and/or treating a disease related to CDCP1, such as a tumor.

## Description

The present application claims the priority of Chinese Patent Application 2022107001160 filed on June 20, 2022. This Chinese patent application is incorporated herein by reference in its entirety.

### TECHNICAL FIELD

The present disclosure belongs to the field of biomedicine. In particular, the present disclosure relates to an anti-CDCP1 antibody and a conjugate comprising same and uses thereof, in particular in the treatment and/or prevention of related diseases in which CDCP1 is a target.

### BACKGROUND

CUB domain-containing protein 1 (CDCP1), also known as cluster of differentiation (CD318), is a type I transmembrane glycoprotein that comprises an extracellular domain (ECD) containing 2-3 CUB (Complement protein components C1, Urchin embryonic growth factor and Bone morphogenic protein 1) domains and an intracellular domain (ICD, Y707, Y734, Y743, Y762 and Y806) containing 5 tyrosines. There are two variants of CDCP1 gene, wherein the major variant is 6.4 kb, locates in chromosome 3p21.31, consists of 9 exons, encodes 836 amino acids, has a molecular weight of 90 kDa, and has an actual SDS-PAGE size of 135 kDa or 140 kDa (CDCP1-135 or p140), which may be related to the protein glycosylation. The CDCP1 extracellular domain can be digested by serine proteases near Arg368 to generate two fragments, CDCP1-65 with a molecular weight of 65 kDa and p80 (or CDCP1-70) with a molecular weight 70-80 kDa. The expression of p140 and p80 are different depending on the expression of serine protease in cells. CDCP1 exists mainly in the form of p140 *in vivo* and is cleaved to p80 during tumor development or tissue damage.

The homology of CDCP1 with other proteins in human is very low, and the homology of CUB domain is less than 20%. Other CUB-containing proteins do not contain intracellular tyrosine regions compared to CDCP1. However, it should be noted that CDCP1 has a high homology in mammals. For example, amino acids sequence analysis shows that the homology between human CDCP1 and mouse CDCP1 is up to 81.3%, and which is up to 95.8% between human and cynomolgus monkey.

CDCP1 is widely expressed in human epithelial cells and hematopoietic stem cell precursors, but not in fibroblasts, mesenchymal stem cells and differentiated blood cells. Some studies have shown that overexpression of this protein in tumors is related to relatively poor prognosis in kidney cancer, lung cancer, colorectal cancer, pancreatic cancer, and ovarian cancer. The phosphorylation of CDCP1 mainly occurs in tumor cell mitotic or detachment, and of inhibits cell adhesion signals, destroys adhesion spots, and negatively regulates cell adhesion. The phosphorylation of CDCP1 is related to cell adhesion. The phosphorylation of CDCP1 will increase during tumor cell detachment while the dephosphorylation of CDCP1 will increases during cell adhesion. The phosphorylation of CDCP1 will also increase in cell proliferation during cell culture with the treatment of trypsin or EDTA, and the overexpression of CDCP1 can lead to cell detachment. CDCP1 plays a role in cell migration, that is, both down-regulation and up-regulation of CDCP1 may inhibit cell migration. CDCP1 exerts its effects mainly the regulation of integrin receptors to form a complex after its phosphorylation, and interfered with the aggregation of integrin itself and block this relation ship between the intracellular skeleton and extracellular matrix.

Analysis of clinical samples has found that the expression of CDCP1 is increased in pancreatic cancer, colon cancer, lung cancer, kidney cancer, ovarian cancer and breast cancer, and Ki67 levels were higher in tumor cells from lung cancer patients with high CDCP1 expression than in tumor cells from patients with low expression, indicating that patients with high CDCP1 expression has faster tumor cell proliferation. The level of CDCP1-p-Y734 is significantly increased in patients with lung cancer and gastric cancer. And CDCP1 showed moderate to high expression in an analysis with the number of 60 or 200 patients with lung cancer. Patients with high CDCP1 expression are more likely to experience lymph node metastasis and recurrence, and a lower 5-year survival rate; A study of 230 patients with kidney cancer showed that CDCP1 expression was difficult to detect in normal tissues, while high expression was detected in 33.5% of the of the patient tissues. In addition, CDCP1 was highly expressed in lymphoma cells.

The high expression of CDCP1 in tumor tissue makes it a potential target for tumor therapy. But so far, there are no drugs made from anti-CDCP1 antibodies available on the market. Therefore, it is important to develop related drugs of anti-CDCP1 antibodies, which will provide cancer patients with more medication choices.

### CONTENT OF THE PRESENT INVENTION

### Antibody of the present disclosure

In one aspect, the present disclosure provides an antibody or an antigen-binding fragment thereof that specifically binds to CDCP1,
the antibody or the antigen-binding fragment thereof comprises the following heavy chain variable region (VH) and light chain variable region (VL), wherein the CDR is defined according to the IMGT numbering system:
(1) a heavy chain variable region (VH) comprising the following 3 CDRs: CDR-H1 comprising the sequence as set forth in SEQ ID NO: 11, CDR-H2 comprising the sequence as set forth in SEQ ID NO: 48, and CDR-H3 comprising the sequence as set forth in SEQ ID NO: 90; and
   a light chain variable region (VL) comprising the following 3 CDRs: CDR-L1 comprising the sequence as set forth in SEQ ID NO: 136, CDR-L2 comprising the sequence as set forth in SEQ ID NO: 171, and CDR-L3 comprising the sequence as set forth in SEQ ID NO: 207;
   or
(2) a heavy chain variable region (VH) comprising the following 3 CDRs: CDR-H1 comprising the sequence as set forth in SEQ ID NO: 14, CDR-H2 comprising the sequence as set forth in SEQ ID NO: 52, and CDR-H3 comprising the sequence as set forth in SEQ ID NO: 95; and
   a light chain variable region (VL) comprising the following 3 CDRs: CDR-L1 comprising the sequence as set forth in SEQ ID NO: 140, CDR-L2 comprising the sequence as set forth in SEQ ID NO: 175, and CDR-L3 comprising the sequence as set forth in SEQ ID NO: 211;
   or
(3) a heavy chain variable region (VH) comprising the following 3 CDRs: CDR-H1 comprising the sequence as set forth in SEQ ID NO: 2, CDR-H2 comprising the sequence as set forth in SEQ ID NO: 39, and CDR-H3 comprising the sequence as set forth in SEQ ID NO: 81; and
   a light chain variable region (VL) comprising the following 3 CDRs: CDR-L1 comprising the sequence as set forth in SEQ ID NO: 125, CDR-L2 comprising the sequence as set forth in SEQ ID NO: 166, and CDR-L3 comprising the sequence as set forth in SEQ ID NO: 199;
   or
(4) a heavy chain variable region (VH) comprising the following 3 CDRs: CDR-H1 comprising the sequence as set forth in SEQ ID NO: 1, CDR-H2 comprising the sequence as set forth in SEQ ID NO: 38, and CDR-H3 comprising the sequence as set forth in SEQ ID NO: 80; and
   a light chain variable region (VL) comprising the following 3 CDRs: CDR-L1 comprising the sequence as set forth in SEQ ID NO: 124, CDR-L2 comprising the sequence as set forth in SEQ ID NO: 164, and CDR-L3 comprising the sequence as set forth in SEQ ID NO: 197;
   or
(5) a heavy chain variable region (VH) comprising the following 3 CDRs: CDR-H1 comprising the sequence as set forth in SEQ ID NO: 1, CDR-H2 comprising the sequence as set forth in SEQ ID NO: 38, and CDR-H3 comprising the sequence as set forth in SEQ ID NO: 80; and
   a light chain variable region (VL) comprising the following 3 CDRs: CDR-L1 comprising the sequence as set forth in SEQ ID NO: 125, CDR-L2 comprising the sequence as set forth in SEQ ID NO: 165, and CDR-L3 comprising the sequence as set forth in SEQ ID NO: 198;
   or
(6) a heavy chain variable region (VH) comprising the following 3 CDRs: CDR-H1 comprising the sequence as set forth in SEQ ID NO: 3, CDR-H2 comprising the sequence as set forth in SEQ ID NO: 39, and CDR-H3 comprising the sequence as set forth in SEQ ID NO: 81; and
   a light chain variable region (VL) comprising the following 3 CDRs: CDR-L1 comprising the sequence as set forth in SEQ ID NO: 126, CDR-L2 comprising the sequence as set forth in SEQ ID NO: 167, and CDR-L3 comprising the sequence as set forth in SEQ ID NO: 200;
   or
(7) a heavy chain variable region (VH) comprising the following 3 CDRs: CDR-H1 comprising the sequence as set forth in SEQ ID NO: 4, CDR-H2 comprising the sequence as set forth in SEQ ID NO: 40, and CDR-H3 comprising the sequence as set forth in SEQ ID NO: 82; and
   a light chain variable region (VL) comprising the following 3 CDRs: CDR-L1 comprising the sequence as set forth in SEQ ID NO: 127, CDR-L2 comprising the sequence as set forth in SEQ ID NO: 168, and CDR-L3 comprising the sequence as set forth in SEQ ID NO: 201;
   or
(8) a heavy chain variable region (VH) comprising the following 3 CDRs: CDR-H1 comprising the sequence as set forth in SEQ ID NO: 5, CDR-H2 comprising the sequence as set forth in SEQ ID NO: 41, and CDR-H3 comprising the sequence as set forth in SEQ ID NO: 83; and
   a light chain variable region (VL) comprising the following 3 CDRs: CDR-L1 comprising the sequence as set forth in SEQ ID NO: 128, CDR-L2 comprising the sequence as set forth in SEQ ID NO: 169, and CDR-L3 comprising the sequence as set forth in SEQ ID NO: 202;
   or
(9) a heavy chain variable region (VH) comprising the following 3 CDRs: CDR-H1 comprising the sequence as set forth in SEQ ID NO: 6, CDR-H2 comprising the sequence as set forth in SEQ ID NO: 42, and CDR-H3 comprising the sequence as set forth in SEQ ID NO: 84; and
   a light chain variable region (VL) comprising the following 3 CDRs: CDR-L1 comprising the sequence as set forth in SEQ ID NO: 129, CDR-L2 comprising the sequence as set forth in SEQ ID NO: 170, and CDR-L3 comprising the sequence as set forth in SEQ ID NO: 203;
   or
(10) a heavy chain variable region (VH) comprising the following 3 CDRs: CDR-H1 comprising the sequence as set forth in SEQ ID NO: 6, CDR-H2 comprising the sequence as set forth in SEQ ID NO: 43, and CDR-H3 comprising the sequence as set forth in SEQ ID NO: 85; and
   a light chain variable region (VL) comprising the following 3 CDRs: CDR-L1 comprising the sequence as set forth in SEQ ID NO: 130, CDR-L2 comprising the sequence as set forth in SEQ ID NO: 170, and CDR-L3 comprising the sequence as set forth in SEQ ID NO: 203;
   or
(11) a heavy chain variable region (VH) comprising the following 3 CDRs: CDR-H1 comprising the sequence as set forth in SEQ ID NO: 7, CDR-H2 comprising the sequence as set forth in SEQ ID NO: 44, and CDR-H3 comprising the sequence as set forth in SEQ ID NO: 86; and
   a light chain variable region (VL) comprising the following 3 CDRs: CDR-L1 comprising the sequence as set forth in SEQ ID NO: 131, CDR-L2 comprising the sequence as set forth in SEQ ID NO: 171, and CDR-L3 comprising the sequence as set forth in SEQ ID NO: 204;
   or
(12) a heavy chain variable region (VH) comprising the following 3 CDRs: CDR-H1 comprising the sequence as set forth in SEQ ID NO: 7, CDR-H2 comprising the sequence as set forth in SEQ ID NO: 44, and CDR-H3 comprising the sequence as set forth in SEQ ID NO: 86; and

   a light chain variable region (VL) comprising the following 3 CDRs: CDR-L1 comprising the sequence as set forth in SEQ ID NO: 132, CDR-L2 comprising the sequence as set forth in SEQ ID NO: 171, and CDR-L3 comprising the sequence as set forth in SEQ ID NO: 204;
   or
(13) a heavy chain variable region (VH) comprising the following 3 CDRs: CDR-H1 comprising the sequence as set forth in SEQ ID NO: 7, CDR-H2 comprising the sequence as set forth in SEQ ID NO: 44, and CDR-H3 comprising the sequence as set forth in SEQ ID NO: 86; and
   a light chain variable region (VL) comprising the following 3 CDRs: CDR-L1 comprising the sequence as set forth in SEQ ID NO: 130, CDR-L2 comprising the sequence as set forth in SEQ ID NO: 170, and CDR-L3 comprising the sequence as set forth in SEQ ID NO: 203;
   or
(14) a heavy chain variable region (VH) comprising the following 3 CDRs: CDR-H1 comprising the sequence as set forth in SEQ ID NO: 8, CDR-H2 comprising the sequence as set forth in SEQ ID NO: 45, and CDR-H3 comprising the sequence as set forth in SEQ ID NO: 87; and
   a light chain variable region (VL) comprising the following 3 CDRs: CDR-L1 comprising the sequence as set forth in SEQ ID NO: 133, CDR-L2 comprising the sequence as set forth in SEQ ID NO: 170, and CDR-L3 comprising the sequence as set forth in SEQ ID NO: 203;
   or
(15) a heavy chain variable region (VH) comprising the following 3 CDRs: CDR-H1 comprising the sequence as set forth in SEQ ID NO: 9, CDR-H2 comprising the sequence as set forth in SEQ ID NO: 46, and CDR-H3 comprising the sequence as set forth in SEQ ID NO: 88; and
   a light chain variable region (VL) comprising the following 3 CDRs: CDR-L1 comprising the sequence as set forth in SEQ ID NO: 134, CDR-L2 comprising the sequence as set forth in SEQ ID NO: 164, and CDR-L3 comprising the sequence as set forth in SEQ ID NO: 205;
   or
(16) a heavy chain variable region (VH) comprising the following 3 CDRs: CDR-H1 comprising the sequence as set forth in SEQ ID NO: 10, CDR-H2 comprising the sequence as set forth in SEQ ID NO: 47, and CDR-H3 comprising the sequence as set forth in SEQ ID NO: 89; and
   a light chain variable region (VL) comprising the following 3 CDRs: CDR-L1 comprising the sequence as set forth in SEQ ID NO: 135, CDR-L2 comprising the sequence as set forth in SEQ ID NO: 172, and CDR-L3 comprising the sequence as set forth in SEQ ID NO: 206;
   or
(17) a heavy chain variable region (VH) comprising the following 3 CDRs: CDR-H1 comprising the sequence as set forth in SEQ ID NO: 12, CDR-H2 comprising the sequence as set forth in SEQ ID NO: 48, and CDR-H3 comprising the sequence as set forth in SEQ ID NO: 91; and
   a light chain variable region (VL) comprising the following 3 CDRs: CDR-L1 comprising the sequence as set forth in SEQ ID NO: 136, CDR-L2 comprising the sequence as set forth in SEQ ID NO: 171, and CDR-L3 comprising the sequence as set forth in SEQ ID NO: 207;
   or
(18) a heavy chain variable region (VH) comprising the following 3 CDRs: CDR-H1 comprising the sequence as set forth in SEQ ID NO: 11, CDR-H2 comprising the sequence as set forth in SEQ ID NO: 48, and CDR-H3 comprising the sequence as set forth in SEQ ID NO: 90; and
   a light chain variable region (VL) comprising the following 3 CDRs: CDR-L1 comprising the sequence as set forth in SEQ ID NO: 136, CDR-L2 comprising the sequence as set forth in SEQ ID NO: 171, and CDR-L3 comprising the sequence as set forth in SEQ ID NO: 207;
   or
(19) a heavy chain variable region (VH) comprising the following 3 CDRs: CDR-H1 comprising the sequence as set forth in SEQ ID NO: 13, CDR-H2 comprising the sequence as set forth in SEQ ID NO: 49, and CDR-H3 comprising the sequence as set forth in SEQ ID NO: 92; and
   a light chain variable region (VL) comprising the following 3 CDRs: CDR-L1 comprising the sequence as set forth in SEQ ID NO: 137, CDR-L2 comprising the sequence as set forth in SEQ ID NO: 173, and CDR-L3 comprising the sequence as set forth in SEQ ID NO: 208;
   or
(20) a heavy chain variable region (VH) comprising the following 3 CDRs: CDR-H1 comprising the sequence as set forth in SEQ ID NO: 13, CDR-H2 comprising the sequence as set forth in SEQ ID NO: 50, and CDR-H3 comprising the sequence as set forth in SEQ ID NO: 93; and
   a light chain variable region (VL) comprising the following 3 CDRs: CDR-L1 comprising the sequence as set forth in SEQ ID NO: 138, CDR-L2 comprising the sequence as set forth in SEQ ID NO: 170, and CDR-L3 comprising the sequence as set forth in SEQ ID NO: 209;
   or
(21) a heavy chain variable region (VH) comprising the following 3 CDRs: CDR-H1 comprising the sequence as set forth in SEQ ID NO: 13, CDR-H2 comprising the sequence as set forth in SEQ ID NO: 51, and CDR-H3 comprising the sequence as set forth in SEQ ID NO: 94; and
   a light chain variable region (VL) comprising the following 3 CDRs: CDR-L1 comprising the sequence as set forth in SEQ ID NO: 139, CDR-L2 comprising the sequence as set forth in SEQ ID NO: 170, and CDR-L3 comprising the sequence as set forth in SEQ ID NO: 210;
   or
(22) a heavy chain variable region (VH) comprising the following 3 CDRs: CDR-H1 comprising the sequence as set forth in SEQ ID NO: 15, CDR-H2 comprising the sequence as set forth in SEQ ID NO: 53, and CDR-H3 comprising the sequence as set forth in SEQ ID NO: 96; and
   a light chain variable region (VL) comprising the following 3 CDRs: CDR-L1 comprising the sequence as set forth in SEQ ID NO: 124, CDR-L2 comprising the sequence as set forth in SEQ ID NO: 164, and CDR-L3 comprising the sequence as set forth in SEQ ID NO: 212;
   or
(23) a heavy chain variable region (VH) comprising the following 3 CDRs: CDR-H1 comprising the sequence as set forth in SEQ ID NO: 16, CDR-H2 comprising the sequence as set forth in SEQ ID NO: 54, and CDR-H3 comprising the sequence as set forth in SEQ ID NO: 97; and
   a light chain variable region (VL) comprising the following 3 CDRs: CDR-L1 comprising the sequence as set forth in SEQ ID NO: 141, CDR-L2 comprising the sequence as set forth in SEQ ID NO: 172, and CDR-L3 comprising the sequence as set forth in SEQ ID NO: 213;
   or
(24) a heavy chain variable region (VH) comprising the following 3 CDRs: CDR-H1 comprising the sequence as set forth in SEQ ID NO: 17, CDR-H2 comprising the sequence as set forth in SEQ ID NO: 55, and CDR-H3 comprising the sequence as set forth in SEQ ID NO: 98; and
   a light chain variable region (VL) comprising the following 3 CDRs: CDR-L1 comprising the sequence as set forth in SEQ ID NO: 126, CDR-L2 comprising the sequence as set forth in SEQ ID NO: 174, and CDR-L3 comprising the sequence as set forth in SEQ ID NO: 214;
   or
(25) a heavy chain variable region (VH) comprising the following 3 CDRs: CDR-H1 comprising the sequence as set forth in SEQ ID NO: 18, CDR-H2 comprising the sequence as set forth in SEQ ID NO: 56, and CDR-H3 comprising the sequence as set forth in SEQ ID NO: 99; and
   a light chain variable region (VL) comprising the following 3 CDRs: CDR-L1 comprising the sequence as set forth in SEQ ID NO: 142, CDR-L2 comprising the sequence as set forth in SEQ ID NO: 176, and CDR-L3 comprising the sequence as set forth in SEQ ID NO: 215;
   or
(26) a heavy chain variable region (VH) comprising the following 3 CDRs: CDR-H1 comprising the sequence as set forth in SEQ ID NO: 19, CDR-H2 comprising the sequence as set forth in SEQ ID NO: 57, and CDR-H3 comprising the sequence as set forth in SEQ ID NO: 100; and
   a light chain variable region (VL) comprising the following 3 CDRs: CDR-L1 comprising the sequence as set forth in SEQ ID NO: 143, CDR-L2 comprising the sequence as set forth in SEQ ID NO: 177, and CDR-L3 comprising the sequence as set forth in SEQ ID NO: 216;
   or
(27) a heavy chain variable region (VH) comprising the following 3 CDRs: CDR-H1 comprising the sequence as set forth in SEQ ID NO: 20, CDR-H2 comprising the sequence as set forth in SEQ ID NO: 58, and CDR-H3 comprising the sequence as set forth in SEQ ID NO: 101; and
   a light chain variable region (VL) comprising the following 3 CDRs: CDR-L1 comprising the sequence as set forth in SEQ ID NO: 144, CDR-L2 comprising the sequence as set forth in SEQ ID NO: 176, and CDR-L3 comprising the sequence as set forth in SEQ ID NO: 215;
   or
(28) a heavy chain variable region (VH) comprising the following 3 CDRs: CDR-H1 comprising the sequence as set forth in SEQ ID NO: 21, CDR-H2 comprising the sequence as set forth in SEQ ID NO: 59, and CDR-H3 comprising the sequence as set forth in SEQ ID NO: 102; and
   a light chain variable region (VL) comprising the following 3 CDRs: CDR-L1 comprising the sequence as set forth in SEQ ID NO: 127, CDR-L2 comprising the sequence as set forth in SEQ ID NO: 168, and CDR-L3 comprising the sequence as set forth in SEQ ID NO: 217;
   or
(29) a heavy chain variable region (VH) comprising the following 3 CDRs: CDR-H1 comprising the sequence as set forth in SEQ ID NO: 21, CDR-H2 comprising the sequence as set forth in SEQ ID NO: 60, and CDR-H3 comprising the sequence as set forth in SEQ ID NO: 102; and
   a light chain variable region (VL) comprising the following 3 CDRs: CDR-L1 comprising the sequence as set forth in SEQ ID NO: 127, CDR-L2 comprising the sequence as set forth in SEQ ID NO: 168, and CDR-L3 comprising the sequence as set forth in SEQ ID NO: 217;
   or
(30) a heavy chain variable region (VH) comprising the following 3 CDRs: CDR-H1 comprising the sequence as set forth in SEQ ID NO: 13, CDR-H2 comprising the sequence as set forth in SEQ ID NO: 49, and CDR-H3 comprising the sequence as set forth in SEQ ID NO: 103; and
   a light chain variable region (VL) comprising the following 3 CDRs: CDR-L1 comprising the sequence as set forth in SEQ ID NO: 144, CDR-L2 comprising the sequence as set forth in SEQ ID NO: 169, and CDR-L3 comprising the sequence as set forth in SEQ ID NO: 218;
   or
(31) a heavy chain variable region (VH) comprising the following 3 CDRs: CDR-H1 comprising the sequence as set forth in SEQ ID NO: 22, CDR-H2 comprising the sequence as set forth in SEQ ID NO: 61, and CDR-H3 comprising the sequence as set forth in SEQ ID NO: 104; and
   a light chain variable region (VL) comprising the following 3 CDRs: CDR-L1 comprising the sequence as set forth in SEQ ID NO: 145, CDR-L2 comprising the sequence as set forth in SEQ ID NO: 178, and CDR-L3 comprising the sequence as set forth in SEQ ID NO: 219;
   or
(32) a heavy chain variable region (VH) comprising the following 3 CDRs: CDR-H1 comprising the sequence as set forth in SEQ ID NO: 17, CDR-H2 comprising the sequence as set forth in SEQ ID NO: 62, and CDR-H3 comprising the sequence as set forth in SEQ ID NO: 105; and
   a light chain variable region (VL) comprising the following 3 CDRs: CDR-L1 comprising the sequence as set forth in SEQ ID NO: 141, CDR-L2 comprising the sequence as set forth in SEQ ID NO: 172, and CDR-L3 comprising the sequence as set forth in SEQ ID NO: 220;
   or
(33) a heavy chain variable region (VH) comprising the following 3 CDRs: CDR-H1 comprising the sequence as set forth in SEQ ID NO: 17, CDR-H2 comprising the sequence as set forth in SEQ ID NO: 63, and CDR-H3 comprising the sequence as set forth in SEQ ID NO: 106; and
   a light chain variable region (VL) comprising the following 3 CDRs: CDR-L1 comprising the sequence as set forth in SEQ ID NO: 126, CDR-L2 comprising the sequence as set forth in SEQ ID NO: 179, and CDR-L3 comprising the sequence as set forth in SEQ ID NO: 221;
   or
(34) a heavy chain variable region (VH) comprising the following 3 CDRs: CDR-H1 comprising the sequence as set forth in SEQ ID NO: 23, CDR-H2 comprising the sequence as set forth in SEQ ID NO: 64, and CDR-H3 comprising the sequence as set forth in SEQ ID NO: 107; and
   a light chain variable region (VL) comprising the following 3 CDRs: CDR-L1 comprising the sequence as set forth in SEQ ID NO: 146, CDR-L2 comprising the sequence as set forth in SEQ ID NO: 180, and CDR-L3 comprising the sequence as set forth in SEQ ID NO: 222;
   or
(35) a heavy chain variable region (VH) comprising the following 3 CDRs: CDR-H1 comprising the sequence as set forth in SEQ ID NO: 24, CDR-H2 comprising the sequence as set forth in SEQ ID NO: 65, and CDR-H3 comprising the sequence as set forth in SEQ ID NO: 108; and
   a light chain variable region (VL) comprising the following 3 CDRs: CDR-L1 comprising the sequence as set forth in SEQ ID NO: 147, CDR-L2 comprising the sequence as set forth in SEQ ID NO: 181, and CDR-L3 comprising the sequence as set forth in SEQ ID NO: 223;
   or
(36) a heavy chain variable region (VH) comprising the following 3 CDRs: CDR-H1 comprising the sequence as set forth in SEQ ID NO: 25, CDR-H2 comprising the sequence as set forth in SEQ ID NO: 65, and CDR-H3 comprising the sequence as set forth in SEQ ID NO: 108; and
   a light chain variable region (VL) comprising the following 3 CDRs: CDR-L1 comprising the sequence as set forth in SEQ ID NO: 148, CDR-L2 comprising the sequence as set forth in SEQ ID NO: 181, and CDR-L3 comprising the sequence as set forth in SEQ ID NO: 224;
   or
(37) a heavy chain variable region (VH) comprising the following 3 CDRs: CDR-H1 comprising the sequence as set forth in SEQ ID NO: 26, CDR-H2 comprising the sequence as set forth in SEQ ID NO: 66, and CDR-H3 comprising the sequence as set forth in SEQ ID NO: 109; and
   a light chain variable region (VL) comprising the following 3 CDRs: CDR-L1 comprising the sequence as set forth in SEQ ID NO: 149, CDR-L2 comprising the sequence as set forth in SEQ ID NO: 182, and CDR-L3 comprising the sequence as set forth in SEQ ID NO: 225;
   or
(38) a heavy chain variable region (VH) comprising the following 3 CDRs: CDR-H1 comprising the sequence as set forth in SEQ ID NO: 27, CDR-H2 comprising the sequence as set forth in SEQ ID NO: 67, and CDR-H3 comprising the sequence as set forth in SEQ ID NO: 110; and
   a light chain variable region (VL) comprising the following 3 CDRs: CDR-L1 comprising the sequence as set forth in SEQ ID NO: 150, CDR-L2 comprising the sequence as set forth in SEQ ID NO: 183, and CDR-L3 comprising the sequence as set forth in SEQ ID NO: 226;
   or
(39) a heavy chain variable region (VH) comprising the following 3 CDRs: CDR-H1 comprising the sequence as set forth in SEQ ID NO: 28, CDR-H2 comprising the sequence as set forth in SEQ ID NO: 68, and CDR-H3 comprising the sequence as set forth in SEQ ID NO: 111; and
   a light chain variable region (VL) comprising the following 3 CDRs: CDR-L1 comprising the sequence as set forth in SEQ ID NO: 151, CDR-L2 comprising the sequence as set forth in SEQ ID NO: 184, and CDR-L3 comprising the sequence as set forth in SEQ ID NO: 227;
   or
(40) a heavy chain variable region (VH) comprising the following 3 CDRs: CDR-H1 comprising the sequence as set forth in SEQ ID NO: 29, CDR-H2 comprising the sequence as set forth in SEQ ID NO: 69, and CDR-H3 comprising the sequence as set forth in SEQ ID NO: 112; and
   a light chain variable region (VL) comprising the following 3 CDRs: CDR-L1 comprising the sequence as set forth in SEQ ID NO: 152, CDR-L2 comprising the sequence as set forth in SEQ ID NO: 185, and CDR-L3 comprising the sequence as set forth in SEQ ID NO: 222;
   or
(41) a heavy chain variable region (VH) comprising the following 3 CDRs: CDR-H1 comprising the sequence as set forth in SEQ ID NO: 30, CDR-H2 comprising the sequence as set forth in SEQ ID NO: 70, and CDR-H3 comprising the sequence as set forth in SEQ ID NO: 113; and
   a light chain variable region (VL) comprising the following 3 CDRs: CDR-L1 comprising the sequence as set forth in SEQ ID NO: 153, CDR-L2 comprising the sequence as set forth in SEQ ID NO: 186, and CDR-L3 comprising the sequence as set forth in SEQ ID NO: 228;
   or
(42) a heavy chain variable region (VH) comprising the following 3 CDRs: CDR-H1 comprising the sequence as set forth in SEQ ID NO: 31, CDR-H2 comprising the sequence as set forth in SEQ ID NO: 71, and CDR-H3 comprising the sequence as set forth in SEQ ID NO: 114; and
   a light chain variable region (VL) comprising the following 3 CDRs: CDR-L1 comprising the sequence as set forth in SEQ ID NO: 154, CDR-L2 comprising the sequence as set forth in SEQ ID NO: 187, and CDR-L3 comprising the sequence as set forth in SEQ ID NO: 229;
   or
(43) a heavy chain variable region (VH) comprising the following 3 CDRs: CDR-H1 comprising the sequence as set forth in SEQ ID NO: 32, CDR-H2 comprising the sequence as set forth in SEQ ID NO: 72, and CDR-H3 comprising the sequence as set forth in SEQ ID NO: 115; and
   a light chain variable region (VL) comprising the following 3 CDRs: CDR-L1 comprising the sequence as set forth in SEQ ID NO: 155, CDR-L2 comprising the sequence as set forth in SEQ ID NO: 188, and CDR-L3 comprising the sequence as set forth in SEQ ID NO: 230;
   or
(44) a heavy chain variable region (VH) comprising the following 3 CDRs: CDR-H1 comprising the sequence as set forth in SEQ ID NO: 33, CDR-H2 comprising the sequence as set forth in SEQ ID NO: 73, and CDR-H3 comprising the sequence as set forth in SEQ ID NO: 116; and
   a light chain variable region (VL) comprising the following 3 CDRs: CDR-L1 comprising the sequence as set forth in SEQ ID NO: 156, CDR-L2 comprising the sequence as set forth in SEQ ID NO: 189, and CDR-L3 comprising the sequence as set forth in SEQ ID NO: 231;
   or
(45) a heavy chain variable region (VH) comprising the following 3 CDRs: CDR-H1 comprising the sequence as set forth in SEQ ID NO: 34, CDR-H2 comprising the sequence as set forth in SEQ ID NO: 74, and CDR-H3 comprising the sequence as set forth in SEQ ID NO: 117; and
   a light chain variable region (VL) comprising the following 3 CDRs: CDR-L1 comprising the sequence as set forth in SEQ ID NO: 157, CDR-L2 comprising the sequence as set forth in SEQ ID NO: 190, and CDR-L3 comprising the sequence as set forth in SEQ ID NO: 232;
   or
(46) a heavy chain variable region (VH) comprising the following 3 CDRs: CDR-H1 comprising the sequence as set forth in SEQ ID NO: 35, CDR-H2 comprising the sequence as set forth in SEQ ID NO: 75, and CDR-H3 comprising the sequence as set forth in SEQ ID NO: 118; and
   a light chain variable region (VL) comprising the following 3 CDRs: CDR-L1 comprising the sequence as set forth in SEQ ID NO: 158, CDR-L2 comprising the sequence as set forth in SEQ ID NO: 191, and CDR-L3 comprising the sequence as set forth in SEQ ID NO: 233;
   or
(47) a heavy chain variable region (VH) comprising the following 3 CDRs: CDR-H1 comprising the sequence as set forth in SEQ ID NO: 26, CDR-H2 comprising the sequence as set forth in SEQ ID NO: 76, and CDR-H3 comprising the sequence as set forth in SEQ ID NO: 119; and
   a light chain variable region (VL) comprising the following 3 CDRs: CDR-L1 comprising the sequence as set forth in SEQ ID NO: 159, CDR-L2 comprising the sequence as set forth in SEQ ID NO: 192, and CDR-L3 comprising the sequence as set forth in SEQ ID NO: 234;
   or
(48) a heavy chain variable region (VH) comprising the following 3 CDRs: CDR-H1 comprising the sequence as set forth in SEQ ID NO: 24, CDR-H2 comprising the sequence as set forth in SEQ ID NO: 77, and CDR-H3 comprising the sequence as set forth in SEQ ID NO: 120; and
   a light chain variable region (VL) comprising the following 3 CDRs: CDR-L1 comprising the sequence as set forth in SEQ ID NO: 160, CDR-L2 comprising the sequence as set forth in SEQ ID NO: 193, and CDR-L3 comprising the sequence as set forth in SEQ ID NO: 235;
   or
(49) a heavy chain variable region (VH) comprising the following 3 CDRs: CDR-H1 comprising the sequence as set forth in SEQ ID NO: 36, CDR-H2 comprising the sequence as set forth in SEQ ID NO: 78, and CDR-H3 comprising the sequence as set forth in SEQ ID NO: 121; and
   a light chain variable region (VL) comprising the following 3 CDRs: CDR-L1 comprising the sequence as set forth in SEQ ID NO: 161, CDR-L2 comprising the sequence as set forth in SEQ ID NO: 194, and CDR-L3 comprising the sequence as set forth in SEQ ID NO: 236;
   or
(50) a heavy chain variable region (VH) comprising the following 3 CDRs: CDR-H1 comprising the sequence as set forth in SEQ ID NO: 32, CDR-H2 comprising the sequence as set forth in SEQ ID NO: 79, and CDR-H3 comprising the sequence as set forth in SEQ ID NO: 122; and
   a light chain variable region (VL) comprising the following 3 CDRs: CDR-L1 comprising the sequence as set forth in SEQ ID NO: 162, CDR-L2 comprising the sequence as set forth in SEQ ID NO: 195, and CDR-L3 comprising the sequence as set forth in SEQ ID NO: 237;
   or
(51) a heavy chain variable region (VH) comprising the following 3 CDRs: CDR-H1 comprising the sequence as set forth in SEQ ID NO: 37, CDR-H2 comprising the sequence as set forth in SEQ ID NO: 79, and CDR-H3 comprising the sequence as set forth in SEQ ID NO: 123; and
   a light chain variable region (VL) comprising the following 3 CDRs: CDR-L1 comprising the sequence as set forth in SEQ ID NO: 163, CDR-L2 comprising the sequence as set forth in SEQ ID NO: 196, and CDR-L3 comprising the sequence as set forth in SEQ ID NO: 238.

In some embodiments of the present disclosure, the CDRs may also be defined according to the Kabat numbering system:
(1) a heavy chain variable region (VH) comprising the following 3 CDRs: CDR-H1 comprising the sequence as set forth in SEQ ID NO: 392, CDR-H2 comprising the sequence as set forth in SEQ ID NO: 393, and CDR-H3 comprising the sequence as set forth in SEQ ID NO: 394; and
   a light chain variable region (VL) comprising the following 3 CDRs: CDR-L1 comprising the sequence as set forth in SEQ ID NO: 395, CDR-L2 comprising the sequence as set forth in SEQ ID NO: 396, and CDR-L3 comprising the sequence as set forth in SEQ ID NO: 207;
   or
(2) a heavy chain variable region (VH) comprising the following 3 CDRs: CDR-H1 comprising the sequence as set forth in SEQ ID NO: 377, CDR-H2 comprising the sequence as set forth in SEQ ID NO: 378, and CDR-H3 comprising the sequence as set forth in SEQ ID NO: 379; and
   a light chain variable region (VL) comprising the following 3 CDRs: CDR-L1 comprising the sequence as set forth in SEQ ID NO: 380, CDR-L2 comprising the sequence as set forth in SEQ ID NO: 381, and CDR-L3 comprising the sequence as set forth in SEQ ID NO: 211;
   or
(3) a heavy chain variable region (VH) comprising the following 3 CDRs: CDR-H1 comprising the sequence as set forth in SEQ ID NO: 362, CDR-H2 comprising the sequence as set forth in SEQ ID NO: 363, and CDR-H3 comprising the sequence as set forth in SEQ ID NO: 364; and
   a light chain variable region (VL) comprising the following 3 CDRs: CDR-L1 comprising the sequence as set forth in SEQ ID NO: 365, CDR-L2 comprising the sequence as set forth in SEQ ID NO: 366, and CDR-L3 comprising the sequence as set forth in SEQ ID NO: 199.

In some embodiments of the present disclosure, the CDRs may also be defined according to the AbM numbering system:
(1) a heavy chain variable region (VH) comprising the following 3 CDRs: CDR-H1 comprising the sequence as set forth in SEQ ID NO: 397, CDR-H2 comprising the sequence as set forth in SEQ ID NO: 398, and CDR-H3 comprising the sequence as set forth in SEQ ID NO: 394; and
   a light chain variable region (VL) comprising the following 3 CDRs: CDR-L1 comprising the sequence as set forth in SEQ ID NO: 395, CDR-L2 comprising the sequence as set forth in SEQ ID NO: 396, and CDR-L3 comprising the sequence as set forth in SEQ ID NO: 207;
   or
(2) a heavy chain variable region (VH) comprising the following 3 CDRs: CDR-H1 comprising the sequence as set forth in SEQ ID NO: 382, CDR-H2 comprising the sequence as set forth in SEQ ID NO: 383, and CDR-H3 comprising the sequence as set forth in SEQ ID NO: 379; and
   a light chain variable region (VL) comprising the following 3 CDRs: CDR-L1 comprising the sequence as set forth in SEQ ID NO: 380, CDR-L2 comprising the sequence as set forth in SEQ ID NO: 381, and CDR-L3 comprising the sequence as set forth in SEQ ID NO: 211;
   or
(3) a heavy chain variable region (VH) comprising the following 3 CDRs: CDR-H1 comprising the sequence as set forth in SEQ ID NO: 367, CDR-H2 comprising the sequence as set forth in SEQ ID NO: 368, and CDR-H3 comprising the sequence as set forth in SEQ ID NO: 364; and
   a light chain variable region (VL) comprising the following 3 CDRs: CDR-L1 comprising the sequence as set forth in SEQ ID NO: 365, CDR-L2 comprising the sequence as set forth in SEQ ID NO: 366, and CDR-L3 comprising the sequence as set forth in SEQ ID NO: 199.

In some embodiments of the present disclosure, the CDRs may also be defined according to the Chothia numbering system:
(1) a heavy chain variable region (VH) comprising the following 3 CDRs: CDR-H1 comprising the sequence as set forth in SEQ ID NO: 399, CDR-H2 comprising the sequence as set forth in SEQ ID NO: 400, and CDR-H3 comprising the sequence as set forth in SEQ ID NO: 394; and
   a light chain variable region (VL) comprising the following 3 CDRs: CDR-L1 comprising the sequence as set forth in SEQ ID NO: 395, CDR-L2 comprising the sequence as set forth in SEQ ID NO: 396, and CDR-L3 comprising the sequence as set forth in SEQ ID NO: 207;
   or
(2) a heavy chain variable region (VH) comprising the following 3 CDRs: CDR-H1 comprising the sequence as set forth in SEQ ID NO: 384, CDR-H2 comprising the sequence as set forth in SEQ ID NO: 385, and CDR-H3 comprising the sequence as set forth in SEQ ID NO: 379; and
   a light chain variable region (VL) comprising the following 3 CDRs: CDR-L1 comprising the sequence as set forth in SEQ ID NO: 380, CDR-L2 comprising the sequence as set forth in SEQ ID NO: 381, and CDR-L3 comprising the sequence as set forth in SEQ ID NO: 211;
(3) a heavy chain variable region (VH) comprising the following 3 CDRs: CDR-H1 comprising the sequence as set forth in SEQ ID NO: 369, CDR-H2 comprising the sequence as set forth in SEQ ID NO: 370, and CDR-H3 comprising the sequence as set forth in SEQ ID NO: 364; and
   a light chain variable region (VL) comprising the following 3 CDRs: CDR-L1 comprising the sequence as set forth in SEQ ID NO: 365, CDR-L2 comprising the sequence as set forth in SEQ ID NO: 366, and CDR-L3 comprising the sequence as set forth in SEQ ID NO: 199;

In the present disclosure, the CDRs may also be defined according to the Contact numbering system:
(1) a heavy chain variable region (VH) comprising the following 3 CDRs: CDR-H1 comprising the sequence as set forth in SEQ ID NO: 401, CDR-H2 comprising the sequence as set forth in SEQ ID NO: 402, and CDR-H3 comprising the sequence as set forth in SEQ ID NO: 403; and
   a light chain variable region (VL) comprising the following 3 CDRs: CDR-L1 comprising the sequence as set forth in SEQ ID NO: 404, CDR-L2 comprising the sequence as set forth in SEQ ID NO: 405, and CDR-L3 comprising the sequence as set forth in SEQ ID NO: 406;
   or
(2) a heavy chain variable region (VH) comprising the following 3 CDRs: CDR-H1 comprising the sequence as set forth in SEQ ID NO: 386, CDR-H2 comprising the sequence as set forth in SEQ ID NO: 387, and CDR-H3 comprising the sequence as set forth in SEQ ID NO: 388; and
   a light chain variable region (VL) comprising the following 3 CDRs: CDR-L1 comprising the sequence as set forth in SEQ ID NO: 389, CDR-L2 comprising the sequence as set forth in SEQ ID NO: 390, and CDR-L3 comprising the sequence as set forth in SEQ ID NO: 391;
   or
(3) a heavy chain variable region (VH) comprising the following 3 CDRs: CDR-H1 comprising the sequence as set forth in SEQ ID NO: 371, CDR-H2 comprising the sequence as set forth in SEQ ID NO: 372, and CDR-H3 comprising the sequence as set forth in SEQ ID NO: 373; and
   a light chain variable region (VL) comprising the following 3 CDRs: CDR-L1 comprising the sequence as set forth in SEQ ID NO: 374, CDR-L2 comprising the sequence as set forth in SEQ ID NO: 375, and CDR-L3 comprising the sequence as set forth in SEQ ID NO: 376.

In certain preferred embodiments, the antibody or the antigen-binding fragment thereof of the present disclosure comprises a heavy chain variable region (VH) and a light chain variable region (VL) selected from any one of the following groups:
(1) VH of the sequence as set forth in SEQ ID NO: 239 and VL of the sequence as set forth in SEQ ID NO: 289;
(2) VH of the sequence as set forth in SEQ ID NO: 239 and VL of the sequence as set forth in SEQ ID NO: 290;
(3) VH of the sequence as set forth in SEQ ID NO: 240 and VL of the sequence as set forth in SEQ ID NO: 291;
(4) VH of the sequence as set forth in SEQ ID NO: 241 and VL of the sequence as set forth in SEQ ID NO: 292;
(5) VH of the sequence as set forth in SEQ ID NO: 242 and VL of the sequence as set forth in SEQ ID NO: 293;
(6) VH of the sequence as set forth in SEQ ID NO: 243 and VL of the sequence as set forth in SEQ ID NO: 294;
(7) VH of the sequence as set forth in SEQ ID NO: 244 and VL of the sequence as set forth in SEQ ID NO: 295;
(8) VH of the sequence as set forth in SEQ ID NO: 245 and VL of the sequence as set forth in SEQ ID NO: 296;
(9) VH of the sequence as set forth in SEQ ID NO: 246 and VL of the sequence as set forth in SEQ ID NO: 297;
(10) VH of the sequence as set forth in SEQ ID NO: 247 and VL of the sequence as set forth in SEQ ID NO: 298;
(11) VH of the sequence as set forth in SEQ ID NO: 248 and VL of the sequence as set forth in SEQ ID NO: 296;
(12) VH of the sequence as set forth in SEQ ID NO: 249 and VL of the sequence as set forth in SEQ ID NO: 299;
(13) VH of the sequence as set forth in SEQ ID NO: 250 and VL of the sequence as set forth in SEQ ID NO: 300;
(14) VH of the sequence as set forth in SEQ ID NO: 251 and VL of the sequence as set forth in SEQ ID NO: 301;
(15) VH of the sequence as set forth in SEQ ID NO: 252 and VL of the sequence as set forth in SEQ ID NO: 302;
(16) VH of the sequence as set forth in SEQ ID NO: 253 and VL of the sequence as set forth in SEQ ID NO: 303;
(17) VH of the sequence as set forth in SEQ ID NO: 254 and VL of the sequence as set forth in SEQ ID NO: 304;
(18) VH of the sequence as set forth in SEQ ID NO: 255 and VL of the sequence as set forth in SEQ ID NO: 305;
(19) VH of the sequence as set forth in SEQ ID NO: 256 and VL of the sequence as set forth in SEQ ID NO: 306;
(20) VH of the sequence as set forth in SEQ ID NO: 257 and VL of the sequence as set forth in SEQ ID NO: 307;
(21) VH of the sequence as set forth in SEQ ID NO: 258 and VL of the sequence as set forth in SEQ ID NO: 308;
(22) VH of the sequence as set forth in SEQ ID NO: 259 and VL of the sequence as set forth in SEQ ID NO: 309;
(23) VH of the sequence as set forth in SEQ ID NO: 260 and VL of the sequence as set forth in SEQ ID NO: 310;
(24) VH of the sequence as set forth in SEQ ID NO: 261 and VL of the sequence as set forth in SEQ ID NO: 311;
(25) VH of the sequence as set forth in SEQ ID NO: 262 and VL of the sequence as set forth in SEQ ID NO: 312;
(26) VH of the sequence as set forth in SEQ ID NO: 263 and VL of the sequence as set forth in SEQ ID NO: 313;
(27) VH of the sequence as set forth in SEQ ID NO: 264 and VL of the sequence as set forth in SEQ ID NO: 314;
(28) VH of the sequence as set forth in SEQ ID NO: 265 and VL of the sequence as set forth in SEQ ID NO: 315;
(29) VH of the sequence as set forth in SEQ ID NO: 266 and VL of the sequence as set forth in SEQ ID NO: 316;
(30) VH of the sequence as set forth in SEQ ID NO: 267 and VL of the sequence as set forth in SEQ ID NO: 317;
(31) VH of the sequence as set forth in SEQ ID NO: 268 and VL of the sequence as set forth in SEQ ID NO: 318;
(32) VH of the sequence as set forth in SEQ ID NO: 269 and VL of the sequence as set forth in SEQ ID NO: 319;
(33) VH of the sequence as set forth in SEQ ID NO: 270 and VL of the sequence as set forth in SEQ ID NO: 320;
(34) VH of the sequence as set forth in SEQ ID NO: 271 and VL of the sequence as set forth in SEQ ID NO: 321;
(35) VH of the sequence as set forth in SEQ ID NO: 272 and VL of the sequence as set forth in SEQ ID NO: 322;
(36) VH of the sequence as set forth in SEQ ID NO: 273 and VL of the sequence as set forth in SEQ ID NO: 323;
(37) VH of the sequence as set forth in SEQ ID NO: 274 and VL of the sequence as set forth in SEQ ID NO: 324;
(38) VH of the sequence as set forth in SEQ ID NO: 275 and VL of the sequence as set forth in SEQ ID NO: 325;
(39) VH of the sequence as set forth in SEQ ID NO: 276 and VL of the sequence as set forth in SEQ ID NO: 326;
(40) VH of the sequence as set forth in SEQ ID NO: 277 and VL of the sequence as set forth in SEQ ID NO: 327;
(41) VH of the sequence as set forth in SEQ ID NO: 278 and VL of the sequence as set forth in SEQ ID NO: 328;
(42) VH of the sequence as set forth in SEQ ID NO: 279 and VL of the sequence as set forth in SEQ ID NO: 329;
(43) VH of the sequence as set forth in SEQ ID NO: 280 and VL of the sequence as set forth in SEQ ID NO: 330;
(44) VH of the sequence as set forth in SEQ ID NO: 281 and VL of the sequence as set forth in SEQ ID NO: 331;
(45) VH of the sequence as set forth in SEQ ID NO: 282 and VL of the sequence as set forth in SEQ ID NO: 332;
(46) VH of the sequence as set forth in SEQ ID NO: 283 and VL of the sequence as set forth in SEQ ID NO: 333;
(47) VH of the sequence as set forth in SEQ ID NO: 284 and VL of the sequence as set forth in SEQ ID NO: 334;
(48) VH of the sequence as set forth in SEQ ID NO: 285 and VL of the sequence as set forth in SEQ ID NO: 335;
(49) VH of the sequence as set forth in SEQ ID NO: 286 and VL of the sequence as set forth in SEQ ID NO: 336;
(50) VH of the sequence as set forth in SEQ ID NO: 287 and VL of the sequence as set forth in SEQ ID NO: 337;
(51) VH of the sequence as set forth in SEQ ID NO: 288 and VL of the sequence as set forth in SEQ ID NO: 338;
(52) VH of the sequence as set forth in SEQ ID NO: 339 and VL of the sequence as set forth in SEQ ID NO: 351;
(53) VH of the sequence as set forth in SEQ ID NO: 339 and VL of the sequence as set forth in SEQ ID NO: 352;
(54) VH of the sequence as set forth in SEQ ID NO: 339 and VL of the sequence as set forth in SEQ ID NO: 353;
(55) VH of the sequence as set forth in SEQ ID NO: 340 and VL of the sequence as set forth in SEQ ID NO: 351;
(56) VH of the sequence as set forth in SEQ ID NO: 340 and VL of the sequence as set forth in SEQ ID NO: 352;
(57) VH of the sequence as set forth in SEQ ID NO: 340 and VL of the sequence as set forth in SEQ ID NO: 353;
(58) VH of the sequence as set forth in SEQ ID NO: 341 and VL of the sequence as set forth in SEQ ID NO: 351;
(59) VH of the sequence as set forth in SEQ ID NO: 341 and VL of the sequence as set forth in SEQ ID NO: 352;
(60) VH of the sequence as set forth in SEQ ID NO: 341 and VL of the sequence as set forth in SEQ ID NO: 353;
(61) VH of the sequence as set forth in SEQ ID NO: 342 and VL of the sequence as set forth in SEQ ID NO: 351;
(62) VH of the sequence as set forth in SEQ ID NO: 342 and VL of the sequence as set forth in SEQ ID NO: 352;
(63) VH of the sequence as set forth in SEQ ID NO: 342 and VL of the sequence as set forth in SEQ ID NO: 353;
(64) VH of the sequence as set forth in SEQ ID NO: 343 and VL of the sequence as set forth in SEQ ID NO: 354;
(65) VH of the sequence as set forth in SEQ ID NO: 343 and VL of the sequence as set forth in SEQ ID NO: 355;
(66) VH of the sequence as set forth in SEQ ID NO: 343 and VL of the sequence as set forth in SEQ ID NO: 356;
(67) VH of the sequence as set forth in SEQ ID NO: 344 and VL of the sequence as set forth in SEQ ID NO: 354;
(68) VH of the sequence as set forth in SEQ ID NO: 344 and VL of the sequence as set forth in SEQ ID NO: 355;
(69) VH of the sequence as set forth in SEQ ID NO: 344 and VL of the sequence as set forth in SEQ ID NO: 356;
(70) VH of the sequence as set forth in SEQ ID NO: 345 and VL of the sequence as set forth in SEQ ID NO: 354;
(71) VH of the sequence as set forth in SEQ ID NO: 345 and VL of the sequence as set forth in SEQ ID NO: 355;
(72) VH of the sequence as set forth in SEQ ID NO: 345 and VL of the sequence as set forth in SEQ ID NO: 356;
(73) VH of the sequence as set forth in SEQ ID NO: 346 and VL of the sequence as set forth in SEQ ID NO: 354;
(74) VH of the sequence as set forth in SEQ ID NO: 346 and VL of the sequence as set forth in SEQ ID NO: 355;
(75) VH of the sequence as set forth in SEQ ID NO: 346 and VL of the sequence as set forth in SEQ ID NO: 356;
(76) VH of the sequence as set forth in SEQ ID NO: 347 and VL of the sequence as set forth in SEQ ID NO: 357;
(77) VH of the sequence as set forth in SEQ ID NO: 347 and VL of the sequence as set forth in SEQ ID NO: 358;
(78) VH of the sequence as set forth in SEQ ID NO: 347 and VL of the sequence as set forth in SEQ ID NO: 359;
(79) VH of the sequence as set forth in SEQ ID NO: 348 and VL of the sequence as set forth in SEQ ID NO: 357;
(80) VH of the sequence as set forth in SEQ ID NO: 348 and VL of the sequence as set forth in SEQ ID NO: 358;
(81) VH of the sequence as set forth in SEQ ID NO: 348 and VL of the sequence as set forth in SEQ ID NO: 359;
(82) VH of the sequence as set forth in SEQ ID NO: 349 and VL of the sequence as set forth in SEQ ID NO: 357;
(83) VH of the sequence as set forth in SEQ ID NO: 349 and VL of the sequence as set forth in SEQ ID NO: 358;
(84) VH of the sequence as set forth in SEQ ID NO: 349 and VL of the sequence as set forth in SEQ ID NO: 359;
(85) VH of the sequence as set forth in SEQ ID NO: 350 and VL of the sequence as set forth in SEQ ID NO: 357;
(86) VH of the sequence as set forth in SEQ ID NO: 350 and VL of the sequence as set forth in SEQ ID NO: 358;
(87) VH of the sequence as set forth in SEQ ID NO: 350 and VL of the sequence as set forth in SEQ ID NO: 359.

Optionally, for the VH and the VL when compared to any one of groups from (1) to (87), the VH has at least 70%, at least 80%, at least 85%, at least 90%, at least 91%, at least 92%, at least 93%, at least 94%, at least 95%, at least 96%, at least 97%, at least 98%, at least 99%, or 100% sequence identity with the VH in any one of groups from (1) to (87); and/or, the VL has at least 70%, at least 80%, at least 85%, at least 90%, at least 91%, at least 92%, at least 93%, at least 94%, at least 95%, at least 96%, at least 97%, at least 98%, at least 99%, or 100% sequence identity with the VL in any one of groups from (1) to (87).

In certain preferred embodiments, the antibody or the antigen-binding fragment of the present disclosure is provided, wherein the antibody or the antigen-binding fragment thereof is selected from an Fab fragment, an Fab' fragment, an F(ab)'₂ fragment, a single chain antibody, a disulfide-stabilized Fv protein (dsFv).

In certain preferred embodiments, the antibody or the antigen-binding fragment thereof of the present disclosure is a single-chain antibody, such as scFv, di-scFv or (scFv)₂.

In certain preferred embodiments, the antibody or the antigen-binding fragment of the present disclosure is provided, wherein the antibody or the antigen-binding fragment thereof is a murine antibody, a chimeric antibody, a humanized antibody, or an antibody derived from other species (e.g., rabbits, camels, or sharks).

In certain preferred embodiments, the antibody or the antigen-binding fragment thereof of the present disclosure further comprises a heavy chain constant region (CH) and a light chain constant region (CL).

In certain preferred embodiments, the heavy chain constant region is selected from the heavy chain constant region or a variant thereof of IgG, IgM, IgE, IgD and IgA.

In certain preferred embodiments, the light chain constant region is selected from a light chain constant region or a variant thereof of kappa or lambda.

In certain preferred embodiments, the variant has a substitution, deletion, or addition of one or more amino acid(s) (e.g., a substitution, deletion, or addition of up to 20, up to 15, up to 10, or up to 5 amino acids; e.g., a substitution, deletion, or addition of 1, 2, 3, 4, or 5 amino acid(s)) compared to the wild-type sequence from which it is derived.

In certain preferred embodiments, the antibody or the antigen-binding fragment thereof of the present disclosure comprises a heavy chain constant region and a light chain constant region selected from the following:
(1) the heavy chain constant region of human IgG1;
(2) the light chain constant region of human IgG1.

In certain preferred embodiments, the heavy chain constant region (CH) of the antibody or the antigen-binding fragment thereof of the present disclosure comprises the amino acid sequence as set forth in SEQ ID NO: 360.

In certain preferred embodiments, the light chain constant region is the kappa light chain constant region of human IgG1; the light chain constant region (CL) comprises the amino acid as set forth in SEQ ID NO: 361.

In certain preferred embodiments, the antibody or antigen-binding fragment thereof comprises a light chain constant region (CL) as set forth in SEQ ID NO: 361 or a variant thereof, the variant has a conservative substitution of up to 20 amino acids compared to SEQ ID NO: 361 (e.g., a conservative substitution of up to 15, up to 10, or up to 5 amino acids; e.g., a conservative substitution of 1, 2, 3, 4, or 5 amino acid(s)).

In certain preferred embodiments, the antibody or antigen-binding fragment thereof of the present disclosure binds to CDCP1 (e.g., human CDCP1) at a KD of less than about 500 nM, such as less than about 100 nM, 50 nM, 40 nM, 40 nM, 20 nM, 10 nM, 1 nM, 0.1 nM, 0.01 nM or less.

In certain preferred embodiments, the antibody or the antigen-binding fragment thereof of the present disclosure binds to tumor cells expressing CDCP1 at an EC₅₀ of less than about 500 nM, such as less than about 100 nM, 10 nM, 1 nM, 0.9 nM, 0.8 nM, 0.7 nM, 0.6 nM, 0.5 nM, 0.4 nM, 0.3 nM, 0.2 nM, 0.1 nM, 0.01 nM or less; Preferably, the EC₅₀ is measured by flow cytometry or cell competitive ELISA.

In certain preferred embodiments, the antibody or the antigen-binding fragment thereof can or cannot induce endocytosis.

In certain preferred embodiments, the antibody or the antigen-binding fragment thereof cannot promote tumor cell migration.

In certain preferred embodiments, the antibody or the antigen-binding fragment thereof can inhibit tumor metastasis.

### Derived antibody

The antibody or the antigen-binding fragment thereof of the present disclosure may be derived, for example, by being linked to another molecule (e.g., another polypeptide or protein). Typically, derivatization (e.g., labeling) of the antibody or the antigen-binding fragment thereof does not adversely affect its binding to CDCP1 (particularly human CDCP1). Accordingly, the antibody or the antigen-binding fragment thereof of the present disclosure are also intended to comprise such derived forms. For example, the antibody or the antigen-binding fragment thereof according to the present disclosure can be functionally linked (by chemical coupling, gene fusion, non-covalent linkage, or other means) to one or more other molecular group(s), such as another antibody (e.g., to form a bispecific antibody), a detection reagent, a pharmaceutical reagent, and/or a protein or a polypeptide (e.g., anti-biotin protein or polyhistidine tag) capable of mediating the antibody or the antigen-binding fragment to bind to another molecule.

A type of derived antibody (for example, a bispecific antibody) is created by crosslinking 2 or more antibodies (belonging to the same type or different types). Methods for obtaining bispecific antibodies are well known in the art, examples of which include, but are not limited to, chemical crosslinking methods, cell engineering methods (hybridoma methods), or genetic engineering methods.

Another type of derived antibody is an antibody that is linked to a therapeutic moiety. The therapeutic moiety of the present disclosure may be bacterial toxins, cytotoxic drugs or radiotoxins, examples of which include, but are not limited to, taxol, cytochalasin B, mitomycin, etoposide, vincristine or other antimetabolites, alkylating agents, antibiotics or antimitotic agents.

Another type of derived antibody is a labeled antibody. For example, the antibody or the antigen-binding fragment thereof of the present disclosure may be linked to a detectable label. The detectable label of the present disclosure may be any substance detectable by fluorescent, spectral, photochemical, biochemical, immunological, electrical, optical or chemical means. Such labels are well known in the art, examples of which include, but are not limited to, an enzyme (e.g., horseradish peroxidase, alkaline phosphatase, β-galactosidase, urease, glucose oxidase, etc.), a radioactive nuclide (e.g., ³H, ¹²5I, ³⁵S, ¹⁴C, or ³²P), a fluorescent dye (e.g., fluorescein isothiocyanate (FITC), fluorescein, tetramethylrhodamine isothiocyanate (TRITC), phycoerythrin (PE), Texas red, rhodamine, quantum dots, or cyanine dye derivatives (e.g., Cy7, Alexa 750)), acridine ester compound, magnetic beads, calorimetric marker such as colloidal gold or colored glass or plastics (e.g., polystyrene, polypropylene, latex, etc.), and biotin for binding avidin (e.g., streptavidin) modified by the above labels. Patents teaching the use of the label include, but are not limited to, U.S. Pat. No. 3,817,837; 3,850,752; 3,939,350; 3,996,345; 4,277,437; 4,275,149; and 4,366,241 (all incorporated herein by reference). Detectable labels as described above can be detected by methods known in the art. For example, radioactive labels may be detected using photographic film or scintillation counters, and fluorescent labels may be detected using photodetectors to detect emitted light. Enzyme labels are generally detected by supplying the enzyme with a substrate and detecting the reaction products produced by the enzyme's action on the substrate, and calorimetric labels are detected by simply visualizing colored markers. In certain embodiments, such labels can be adapted for immunological assays (e.g., enzyme-linked immunoassay, radioimmunoassay, fluorescent immunoassay, chemiluminescent immunoassay, etc.). In certain embodiments, detectable labels as described above may be linked to the antibody or the antigen-binding fragment thereof of the present disclosure by linkers of different lengths to reduce potential steric hindrance.

In addition, the antibody or the antigen-binding fragment thereof of the present disclosure may also be derived from chemical groups, such as polyethylene glycol (PEG), methyl or ethyl, or sugar groups. These groups can be used to improve the biological properties of antibodies, such as increasing serum half-life.

### Multispecific antibodies

In another aspect, the present disclosure provides a multispecific antibody comprising the antibody or the antigen-binding fragment thereof of the present disclosure. In certain preferred embodiments, the multispecific antibody is a bispecific antibody or a trispecific antibody or a tetraspecific antibody.

### Isolated nucleic acid molecule

In another aspect, the present disclosure provides a nucleic acid molecule encoding the antibody or the antigen-binding fragment thereof of the present disclosure or the multispecific antibody of the present disclosure.

### Vector

In another aspect, the present disclosure provides a vector comprising a nucleic acid molecule encoding the antibody or the antigen-binding fragment thereof of the present disclosure or the multispecific antibody of the present disclosure.

In certain preferred embodiments, the vector is a clone vector or an expression vector.

In certain preferred embodiments, the vector of the present disclosure is, for example, a plasmid, a cosmid, a bacteriophage, and the like. In certain preferred embodiments, the vector is capable of expressing the antibody or the antigen-binding fragment thereof of the present disclosure in a subject (e.g., a mammal, e.g., a human).

### Host cell

In another aspect, the present disclosure provides a host cell comprising the nucleic acid molecule of the present disclosure or the vector of the present disclosure. Such host cells include, but are not limited to, prokaryotic cells such as Escherichia coli cells, and eukaryotic cells such as yeast cells, insect cells, plant cells, and animal cells (such as mammalian cells, such as mouse cells, human cells, etc.). In certain preferred embodiments, the host cell of the present disclosure is a mammalian cell, such as a CHO (e.g., CHO-K1, CHO-S, CHO DG44).

### Preparation of antibody

In another aspect, the present disclosure provides a method of preparing an antibody or an antigen-binding fragment thereof, or a multispecific antibody, that specifically binds to CDCP1, and the method comprises culturing the host cell of the present disclosure under conditions that allow expression of the antibody or the antigen-binding fragment thereof, or the multispecific antibody, and recovering the antibody or the antigen-binding fragment thereof, or the multispecific antibody from the cultured host cell culture.

Antibodies of the present disclosure can be prepared by various methods known in the art, such as by genetic engineering recombinant technology. For example, DNA molecules encoding heavy and light chain genes of antibodies of the present disclosure are obtained by chemical synthesis or PCR amplification. The resulting DNA molecules are inserted into the expression vector and then transfected into host cells. Then, the transfected host cells are cultured under specific conditions and the antibodies of the present disclosure are expressed.

Antigen-binding fragments of the present disclosure can be obtained by hydrolyzing intact antibody molecules (see Morimoto et al., J. Biochem. Biophys. Methods 24: 107-117 (1992) and Brennan et al., Science 229: 81 (1985)). In addition, these antigen-binding fragments can also be generated directly from recombinant host cells (reviewed in Hudson, Curr. Opin. Immunol. 11: 548-557 (1999); Little et al., Immunol. Today, 21:364-370 (2000)). For example, Fab' fragments can be obtained directly from host cells; Fab' fragments can be chemically coupled to form F(ab')₂ fragment (Carter et al., Bio/Technology, 10: 163-167 (1992)). Also, Fv, Fab, or F(ab')₂ fragments can also be directly isolated from the culture fluid of recombinant host cells. Other techniques for preparing these antigen-binding fragments are well known to those of ordinary skill in the art.

### Conjugate

In another aspect, the present disclosure provides a conjugate or a pharmaceutically acceptable salt thereof, wherein the conjugate comprises the antibody or the antigen-binding fragment thereof of the present disclosure, or the multispecific antibody of the present disclosure, and a coupling moiety; the antibody or the antigen-binding fragment thereof, or the multispecific antibody is linked to the coupling moiety directly or by a linker.

In certain preferred embodiments, the coupling moiety is selected from: a detectable label (e.g., radioisotope, fluorescent substance, luminescent substance, colored substance or enzyme) or a therapeutic agent (e.g., nuclide, immune agonist, immunosuppressant, cytokine, toxin, and other active substance that inhibits tumor cell growth, promotes tumor cell apoptosis or necrosis).

In certain preferred embodiments, the structure of the conjugate is as shown in formula (I):
A-(L-D)ₚ (I), L is present or absent;
where A is an antibody or an antigen-binding fragment thereof of the present disclosure, or a multispecific antibody of the present disclosure; L is the linker, preferably the linker is a vc linker, the structure of which is: 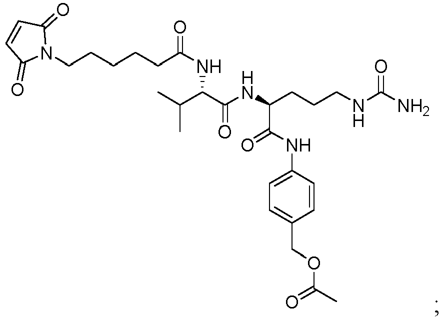
D is the coupling moiety;

In certain preferred embodiments, the coupling moiety is an immune activator comprising a TLR, STING, ONDs, CpG, LPS, SEB, SEA, or SEC activator.

In certain preferred embodiments, the coupling moiety is a cytokine comprising IL-6, IL-15, IL-12, IL-1b or IL-23.

In certain preferred embodiments, the coupling moiety is a toxin, which comprises a cytotoxic agent; more preferably, the cytotoxic agent is selected from camptothecins (e.g., SN-38), maytansines (e.g., maytansine DM1/4), pyrrolozobenzodiazepines (PBDs), or auristatin (e.g., Monomethyl auristatin E/F).

In certain preferred embodiments, the value of p is more than or equal to 0;

In certain preferred embodiments, p is 0-8.

### Pharmaceutical composition

In another aspect, the present disclosure provides a pharmaceutical composition comprising the antibody or the antigen-binding fragment thereof or a precursor drug thereof (e.g., a probody), the conjugate, the multispecific antibody of the present disclosure, and a pharmaceutically acceptable carrier and/or excipient.

In certain preferred embodiments, the pharmaceutical composition further comprises an additional pharmaceutically active agent. In certain preferred embodiments, the additional pharmaceutically active agent is a drug having anti-tumor activity, such as a platinum-based drug, a small molecule inhibitor, or the like. In certain preferred embodiments, the additional pharmaceutically active agent is an immunity-enhancing drug, e.g. interferon, interleukin. In certain preferred embodiments, the additional pharmaceutically active agent is an immunotherapy-related drug, such as an immune checkpoint inhibitor (e.g., a PD-1, PD-L1 or CTLA-4 inhibitor), an immunity-enhancing drug (e.g., interferon, interleukin) or an immune system activator (e.g., a TLR, ONDs, CpG, LPS, SEB, SEA, STING or SEC activator). In certain preferred embodiments, the additional pharmaceutically active agent is an oncolytic virus, an immune cell, or an engineered immune cell. In certain preferred embodiments, the additional pharmaceutically active agent is a radioactive substance or a chromogenic agent.

In certain preferred embodiments, the antibody or the antigen-binding fragment thereof is provided as a separate component or as a component of the same composition with the additional pharmaceutically active agent.

In certain preferred embodiments, the antibody or the antigen-binding fragment thereof, the conjugate, the multispecific antibody or the pharmaceutical composition of the present disclosure is used in a subject for: (a) inducing apoptosis of tumor cells; (b) inhibiting tumor cell proliferation; (c) inducing and/or increasing T cell infiltration; (d) inducing and/or enhancing an immune response; (e) inducing and/or increasing complement-dependent cytotoxic activity; (f) inducing and/or increasing antibody-dependent cytotoxic activity; (g) increasing NK cell activity; (h) inhibiting the expression and activation of CDCP1; (i) inhibiting CDCP1-mediated signaling; or (j) any combination of (a)-(i).

In certain preferred embodiments, wherein the tumor is selected from a solid tumor or a hematological tumor.

In certain preferred embodiments, the tumor is a CDCP1 positive tumor.

In certain preferred embodiments, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, glioblastoma; The hematological tumor is selected from lymphoma, myeloma (e.g., multiple myeloma) or leukemia.

### Use

In another aspect, the present disclosure provides the use of the antibody or the antigen-binding fragment thereof, the multispecific antibody, the conjugate or the pharmaceutical composition of the present disclosure in the preparation of a medicament for use in the prevention and/or treatment and/or adjuvant treatment and/or neoadjuvant treatment of a disease related to CDCP1.

In certain preferred embodiments, the disease related to CDCP1 is a tumor.

In certain preferred embodiments, the tumor is selected from a solid tumor or a hematological tumor.

In certain preferred embodiments, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; The hematological tumor is selected from lymphoma, myeloma (e.g., multiple myeloma) or leukemia.

In certain preferred embodiments, the antibody or the antigen-binding fragment thereof, the conjugate, the multispecific antibody or the pharmaceutical composition of the present disclosure is administered separately, in combination, simultaneously or sequentially with an additional pharmaceutically active agent.

In certain preferred embodiments, the additional pharmaceutically active agent is a drug having anti-tumor activity, such as a platinum drug, a small molecule inhibitor, or the like.

In certain preferred embodiments, the additional pharmaceutically active agent is an immunity-enhancing drug, e.g. interferon, interleukin.

In certain preferred embodiments, the additional pharmaceutically active agent is an immune system activator (e.g., a TLR, ONDs, CpG, LPS, SEB, STING, SEA, or SEC activator).

In certain preferred embodiments, the additional pharmaceutically active agent is an immune checkpoint inhibitor (e.g., PD-1, PD-L1 or CTLA-4 inhibitor);
In certain preferred embodiments, the additional pharmaceutically active agent is an oncolytic virus, an immune cell, or an engineered immune cell.

In another aspect, the present disclosure provides a method of prevention and/or treatment and/or adjuvant treatment and/or neoadjuvant treatment of a diseases related to CDCP1 in a subject, and the method comprises administering an effective amount of the antibody or the antigen-binding fragment thereof, the conjugate, the multispecific antibody or pharmaceutical composition of the present disclosure to a subject in need thereof.

In certain preferred embodiments, the method further comprises administering to the subject a second therapy selected from surgery, chemotherapy, radiotherapy, immunotherapy, gene therapy, DNA therapy, RNA therapy, nanotherapy, viral therapy, adjuvant therapy, cell therapy, and any combination thereof, the second therapy may be applied separately or in combination with the method of the present disclosure; the second therapy may be applied separately or in combination with the method of the present disclosure simultaneously or sequentially.

In certain preferred embodiments, the disease related to CDCP1 is a tumor.

In certain preferred embodiments, the tumor is selected from a solid tumor or a hematological tumor.

In certain preferred embodiments, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; The hematological tumor is selected from lymphoma, myeloma (e.g., multiple myeloma) or leukemia.

In another aspect, the present disclosure provides a method of detecting the presence or level of CDCP1 in a sample, and the method comprises bringing the sample into contact with the antibody or the antigen-binding fragment thereof, the multispecific antibody, the conjugate or the pharmaceutical composition under conditions that allow the antibody or the antigen-binding fragment thereof, the multispecific antibody, the conjugate or the pharmaceutical composition of the present disclosure to form a complex with CDCP1, and detecting the formation of the complex.

In another aspect, the present disclosure provides the antibody or the antigen-binding fragment thereof, the conjugate, the multispecific antibody or the pharmaceutical composition of the present disclosure for use in the prevention and/or treatment and/or adjuvant treatment and/or neoadjuvant treatment of a disease related to CDCP1.

In certain preferred embodiments, the disease related to CDCP1 is a tumor.

In certain preferred embodiments, the tumor is selected from a solid tumor or a hematological tumor.

In certain preferred embodiments, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; The hematological tumor is selected from lymphoma, myeloma (e.g., multiple myeloma) or leukemia.

In another aspect, the present disclosure provides the antibody or the antigen-binding fragment thereof, the conjugate, the multispecific antibody or the pharmaceutical composition of the present disclosure for the diagnosis or differential diagnosis of a disease related to CDCP1.

In certain preferred embodiments, the disease related to CDCP1 is a tumor.

In certain preferred embodiments, the tumor is selected from a solid tumor or a hematological tumor.

In certain preferred embodiments, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; The hematological tumor is selected from lymphoma, myeloma (e.g., multiple myeloma) or leukemia.

The antibody or the antigen-binding fragment thereof of the present disclosure, the pharmaceutical composition of the present disclosure, or the immunogenic composition of the present disclosure may be formulated into any dosage form known in the medical field, for example, tablets, pills, suspensions, emulsions, solutions, gels, capsules, powders, granules, elixirs, lozenges, suppositories, injections (including injection solutions, sterile powders for injection and concentrated solutions for injection), inhalants, sprays, etc. The preferred dosage form depends on the intended mode of administration and therapeutic use. The pharmaceutical composition of the present disclosure should be sterile and stable under production and storage conditions. One preferred dosage form is an injection. Such an injection may be a sterile injection solution. For example, the sterile injection solution may be prepared by: Into a suitable solvent, the recombinant protein of the present disclosure is incorporated at a necessary dose and optionally, other expected ingredients (including, but not limited to, a pH regulator, a surfactant, an adjuvant, an ionic strength enhancer, an isotonic agent, a preservative, a diluent, or any combination thereof) are incorporated, followed by filtration and sterilization. In addition, the sterile injection solution may be prepared as a sterile lyophilized powder (e.g., by vacuum drying or freeze drying) for easy storage and use. Such a sterile lyophilized powder can be dispersed in a suitable carrier, such as sterile pyrogenic water, before use.

Further, the antibody or the antigen-binding fragment thereof of the present disclosure may be present in a unit dose form in a pharmaceutical composition or an immunogenic composition to facilitate administration.

The antibody or the antigen-binding fragment thereof, the pharmaceutical composition or the immunogenic composition of the present disclosure may be administered by any suitable method known in the art, including, but not limited to, oral, buccal, sublingual, ocular, topical, parenteral, rectal, intrathecal, intracytoplasmic reticulum, inguinal, intravesical, topical (e.g., powder, ointment or drops), or nasal route. However, for many therapeutic uses, the preferred route/mode of administration is parenteral administration (e.g., intravenous injection, subcutaneous injection, intraperitoneal injection, intramuscular injection). The skilled person should understand that the route and/or manner of administration will change according to the intended purpose. In a preferred embodiment, the antibody or the antigen-binding fragment thereof, the pharmaceutical composition or the immunogenic composition of the present disclosure is administered by intravenous infusion or injection.

The pharmaceutical composition or the immunogenic composition of the present disclosure may comprise an "effective amount" ("therapeutically effective amount" or "prophylactically effective amount") of the antibody or the antigen-binding fragment thereof of the present disclosure. "Prophylactically effective amount" means an amount sufficient to prevent, stop, or delay the occurrence of a disease. "Therapeutically effective amount" means an amount sufficient to cure or at least partially stop a disease and its complications in a patient who already has the disease. The therapeutically effective amount of the antibody or the antigen-binding fragment thereof of the present disclosure may vary according to the following factors: the severity of the disease to be treated, the general state of the patient's own immune system, the general condition of the patient such as age, weight and sex, the manner in which the drug is administered, other treatments administered at the same time, etc.

In the present disclosure, the dosing regimen may be adjusted to obtain the best desired response (e.g., therapeutic or prophylactic response). For example, it can be administered in a single dose, it can be administered multiple times over a period of time, or it can be decreased or increased proportionally based on the urgency of the treatment situation.

It should be noted that the dose may vary depending on the type and severity of the symptom requiring treatment. Further, one skilled in the art understands that for any particular patient, the particular dosing regimen should be adjusted over time according to the needs of the patient and the professional evaluation of the physician. The dose ranges given herein are for illustrative purposes only and do not limit the use or range of the pharmaceutical composition or the immunogenic composition of the present disclosure.

In the present disclosure, the subject may be a mammal, such as a human.

The present invention provides a container (e.g. a plastic or glass vial, e.g. having caps or chromatographic columns, hollow needles or syringe cylinders) that contains any antibody or antigen-binding fragment, or pharmaceutical composition of the present disclosure. The invention also provides an injection device comprising any antibody or antigen-binding fragment, or pharmaceutical composition of the present disclosure.

### Kit

The antibody or the antigen-binding fragment thereof of the present disclosure is capable of specifically binding to CDCP1 and thus can be used to detect the presence or level of CDCP1 in a sample.

Thus, in another aspect, the present disclosure provides a kit comprising the antibody or the antigen-binding fragment thereof, the multispecific antibody, the conjugate or the pharmaceutical composition of the present disclosure. In certain preferred embodiments, the antibody or the antigen-binding fragment thereof of the present disclosure carries a detectable label. In a preferred embodiment, the kit further comprises a second antibody that specifically recognizes the antibody or the antigen-binding fragment thereof of the present disclosure. Preferably, the second antibody further comprises a detectable label.

The detectable label of the present disclosure may be any substance detectable by fluorescent, spectral, photochemical, biochemical, immunological, electrical, optical or chemical means. Particularly preferably, such labels can be adapted for immunological assays (e.g., enzyme-linked immunoassay, radioimmunoassay, fluorescent immunoassay, chemiluminescent immunoassay, etc.). Such labels are well known in the art and include, but are not limited to, an enzyme (e.g., horseradish peroxidase, alkaline phosphatase, β-galactosidase, urease, glucose oxidase, etc.), a radioactive nuclide (e.g., ³H, ¹²5I, ³⁵S, ¹⁴C, or ³²P), a fluorescent dye (e.g., fluorescein isothiocyanate (FITC), fluorescein, tetramethylrhodamine isothiocyanate (TRITC), phycoerythrin (PE), Texas red, rhodamine, quantum dots, or cyanine dye derivatives (e.g., Cy7, Alexa 750)), acridine ester compound, magnetic beads, calorimetric marker such as colloidal gold or colored glass or plastics (e.g., polystyrene, polypropylene, latex, etc.), and biotin for binding avidin (e.g., streptavidin) modified by the above labels. Patents teaching the use of the label include, but are not limited to, U.S. Pat. No. 3,817,837; 3,850,752; 3,939,350; 3,996,345; 4,277,437; 4,275,149; and 4,366,241 (all incorporated herein by reference). Labels encompassed in the present disclosure can be detected by methods known in the art. For example, radioactive labels may be detected using photographic film or scintillation counters, and fluorescent labels may be detected using photodetectors to detect emitted light. Enzyme labels are generally detected by supplying the enzyme with a substrate and detecting the reaction products produced by the enzyme's action on the substrate, and calorimetric labels are detected by simply visualizing colored markers. In certain embodiments, detectable labels as described above may be linked to the recombinant protein of the present disclosure by linkers of different lengths to reduce potential steric hindrance.

In another aspect, the present disclosure provides the use of the antibody or the antigen-binding fragment thereof, the conjugate, the multispecific antibody or the pharmaceutical composition of the present disclosure in the preparation of a diagnostic kit for the diagnosis or differential diagnosis of a diseases related to CDCP1.

In certain preferred embodiments, the disease related to CDCP1 is a tumor.

In certain preferred embodiments, the tumor is selected from a solid tumor or a hematological tumor.

In certain preferred embodiments, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; The hematological tumor is selected from lymphoma, myeloma (e.g., multiple myeloma) or leukemia.

### Definition of terms

In the present disclosure, all scientific and technical terms used herein have the meanings commonly understood by a person skilled in the art unless specified otherwise. In addition, the laboratory operation steps related to virology, biochemistry, and immunology as used herein are conventional steps that are widely used in the corresponding art. To better understand the present disclosure, definitions and explanations of related terms are provided below.

When the terms "such as", "as", "for example", "comprise", "include" or variations thereof are used herein, such terms will not be considered as limiting terms, but will be interpreted to mean "but not limited to" or "not limited to."

Unless otherwise indicated herein or clearly contradicted by context, the terms "a" and "an" and "the" and similar referents should be interpreted to cover both singular and plural forms in the context of describing the present disclosure, especially in the context of the following claims.

As used herein, the term "antibody" refers to an immunoglobulin molecule typically consisting of two pairs of polypeptide chains, each pair having a light chain (LC) and a heavy chain (HC). Antibody light chains can be classified into κ (kappa) and λ (lambda) light chains. Heavy chains can be classified into µ, δ, γ, α or ε, and the isotypes of antibodies are defined as IgM, IgD, IgG, IgA and IgE, respectively. In the light chain and heavy chain, the variable region and constant region are linked by the "J" region of about 12 or more amino acids, and the heavy chain also comprises the "D" region of about 3 or more amino acids. Each heavy chain consists of a heavy chain variable region (VH) and a heavy chain constant region (CH). For example, the heavy chain constant region of IgG consists of 3 domains (CH1, CH2 and CH3). Each light chain consists of a light chain variable region (VL) and a light chain constant region (CL). The light chain constant region consists of one domain CL. Constant domains do not directly participate in the binding of antigens and antibodies, but exhibit a variety of effector functions, such as being capable of mediating the binding of immunoglobulins to host tissues or factors, including various cells of the immune system (e.g., effector cells) and the first component of the classical complement system (C1q). The VH region and VL region can also be subdivided into regions with high variability called complementary determining region (CDR), among which more conservative regions called framework regions (FRs) are interspersed. Each V_{H} or V_{L} consists of 3 CDRs and 4 FRs arranged in the following order: FR1, CDR1, FR2, CDR2, FR3, CDR3, and FR4 from amino terminus to carboxy terminus. The variable regions (VH and VL) of each heavy chain and light chain pair form antigen binding sites respectively. The assignment of amino acids in each region or domain may follow definitions of Kabat, Sequences of Proteins of Immunological Interest (National Institutes of Health, Bethesda, Md. (1987 and 1991)), or Chothia & Lesk (1987) J. Mol. Biol. 196: 901-917; Chothia et al. (1989) Nature 342: 878-883.

In this article, unless the context expressly indicates, when the term "antibody" is mentioned, it includes not only the complete antibody, but also the antigen-binding fragment of the antibody.

As used herein, the term "complementarity determining region" or "CDR" refers to the amino acid residues in the variable region of an antibody responsible for antigen binding. The variable region of the heavy chain and the variable region of the light chain each contain three CDRs, designated CDR1, CDR2, and CDR3. The precise boundaries of these CDRs may be defined in accordance with various numbering systems known in the art, for example, defined in accordance with the Kabat numbering system (Kabat et al., Sequences of Proteins of Immunological Interest, 5th Ed., Public Health Service, National Institutes of Health, Bethesda, Md., 1991), the Chothia numbering system (Chothia & Lesk (1987) J. Mol. Biol. 196: 901-917; Chothia et al. (1989) Nature 342: 878-883), the IMGT numbering system (Lefranc et al., Dev. Comparat. Immunol. 27: 55-77, 2003), the AbM numbering system (Martin ACR, Cheetham JC, Rees AR (1989) Modelling antibody hypervariable loops: A combined algorithm. Proc Natl Acad Sci USA 86: 9268-9272) or the Contact numbering system (MacCallum, R. M., Martin, A. C. R., & Thornton, J. M. (1996). Antibody-antigen Interactions: Contact Analysis and Binding Site Topography. Journal of Molecular Biology, 262 (5), 732-745.). For a given antibody, one skilled in the art will easily identify the CDR defined according to each numbering system. Moreover, correspondence between different numbering systems is well known to those skilled in the art (see, for example, Lefranc et al., Dev. Comparat. Immunol. 27: 55-77, 2003).

In the present disclosure, the CDR contained in the antibody or the antigen-binding fragment thereof of the present disclosure may be determined according to various numbering systems known in the art, such as by Kabat, Chothia, IMGT, AbM or Contact numbering systems. In certain embodiments, the CDR contained in the antibody or the antigen-binding fragment thereof of the present disclosure is determined by the Kabat, Chothia, IMGT, AbM or Contact numbering system.

As used herein, the terms "framework region" or "FR" residues refer to those amino acid residues in the variable region of an antibody other than the CDR residues as defined above.

The term "antibody" is not limited by any particular method of producing antibodies. For example, it includes recombinant antibodies, monoclonal antibodies and polyclonal antibodies. Antibodies may be antibodies of different isotypes, for example, IgG (e.g., IgG1, IgG2, IgG3 or IgG4 subtypes), IgA1, IgA2, IgD, IgE or IgM antibodies.

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody to specifically bind to the antigen, and is also referred to as "antigen-binding portion". Usually see, Fundamental Immunology, Ch. 7 (Paul, W., ed., 2nd ed., Raven Press, N.Y. (1989), which is incorporated herein by reference in its entirety for all purposes. Antigen-binding fragments of antibodies can be produced by recombinant DNA technology or by enzymatic or chemical cleavage of intact antibodies. Non-limiting examples of antigen-binding fragments include Fab, Fab', F(ab')₂, Fd, Fv, complementarity determining region (CDR) fragments, scFv, diabodies, single domain antibodies, chimeric antibodies, linear antibodies, nanobodies (technology from Domantis), probody and polypeptides that contain at least a portion of an antibody sufficient to confer specific antigen binding capacity to the polypeptide.

As used herein, the term "full-length antibody" means an antibody consisting of two "full-length heavy chains" and two "full-length light chains". Among them, "full-length heavy chain" refers to a polypeptide chain that consists of, in a direction from the N-terminus to the C-terminus, a heavy chain variable region (VH), a heavy chain constant region CH1 domain, a hinge region (HR), a heavy chain constant region CH2 domain, and a heavy chain constant region CH3 domain; moreover, when the full-length antibody is an IgE isotype, it optionally also comprises a heavy chain constant region CH4 domain. Preferably, the "full-length heavy chain" is a polypeptide chain consisting of VH, CH1, HR, CH2 and CH3 in the N-terminal to C-terminal direction. "Full-length light chain" is a polypeptide chain that consists of, in a direction from the N-terminus to the C-terminus, a light chain variable region (VL) and a light chain constant region (CL). The two pairs of full-length antibody chains are linked together by a disulfide bond between CL and CH1 and a disulfide bond between the HRs of the two full-length heavy chains. The full-length antibody contains two antigen-binding sites formed by VH and VL pairs, respectively, the two antigen-binding sites specifically recognize/bind the same antigen.

As used herein, the term "Fab fragment" means an antibody fragment consisting of the VL, VH, CL and CH1 domains. The term "F(ab')₂ fragment "means an antibody fragment comprising two Fab fragments linked by a disulfide bridge on the hinge region. The term "Fab' fragment" means a fragment obtained by reducing disulfide bonds connecting two heavy chain fragments in the F(ab')₂ fragment and consisted of an intact light chain and the Fd fragment (consisting of the VH and CH1 domains) of the heavy chain.

As used herein, the term "Fv" means an antibody fragment consisting of the VL and VH domains of the antibody's single arm. The Fv fragment is generally considered to be the smallest antibody fragment that can form an intact antigen binding site. It is generally believed that the six CDRs confer antigen binding specificity to an antibody. However, even one variable region (such as a Fd fragment, which contains only three antigen-specific CDRs) is able to recognize and bind to an antigen, although it may have a lower affinity than an intact binding site.

As used herein, the term "Fc domain" or "Fc region" means a portion of the constant region of the heavy chain comprising CH2 and CH3. The Fc fragment of an antibody has a number of different functions, but is not involved in antigen binding. "Effector function" mediated by the Fc region includes Fc receptor binding; Clq binding and complement-dependent cytotoxicity (CDC); antibody-dependent cell-mediated cytotoxicity (ADCC); phagocytosis; down-regulation of cell surface receptors, such as B cell receptors; B cell activation, etc. In some embodiments, the Fc region comprises a hinge, CH2, and CH3. When the Fc region contains a hinge, the hinge regulates the dimerization between two Fc-containing polypeptides. The Fc region may be any antibody chain constant region isotype, such as IgG1, IgG2, IgG3 or IgG4.

The Fc domain can include both the natural Fc region and the variant Fc region. The natural Fc region contains amino acid sequences consistent with the amino acid sequences of the Fc region found in nature, for example, the natural human Fc region sequence includes the natural human IgG1 Fc region sequence (non-A and A allotypes); natural human IgG2 Fc region sequence; natural human IgG3 Fc region sequence; and the natural human IgG4 Fc region sequence, and naturally occurring variants thereof. The variant Fc region comprises an amino acid sequence that differs from the amino acid sequence of the natural Fc region sequence due to at least one amino acid modification. In some embodiments, the variant Fc region may have effector functions (e.g., Fc receptor binding, antibody glycosylation, number of cysteine residues, effector cell function, or complement function) that are altered compared to the native Fc region.

As used herein, the term "scFv" refers to a single polypeptide chain comprising the VL and VH domains, wherein the VL and VH are linked by a linker. Such scFv molecules can have a general structure: NH₂-VL-linker-VH-COOH or NH₂-VH-linker-VL-COOH. Suitable linkers in the prior art composed of repeated GGGGS amino acid sequences or variants thereof. For example, a linker having amino acid sequence (GGGGS)₄ can be used, and variants thereof can also be used. In some cases, a disulfide bond may also exist between VH and VL of the scFv.

As used herein, the term "diabody" means that the VH and VL domains thereof are expressed on a single polypeptide chain, but the used linker is too short to allow pairing between the two domains of the same chain, thereby forcing one domain to pair with the complementary domain of another chain and generating two antigen-binding sites (see, e.g., Holliger P. et al., Proc. Natl. Acad. Sci. USA 90: 6444-6448 (1993), and Poljak R. J. et al., Structure 2: 1121-1123 (1994)).

Each of the above antibody fragments maintains the ability to specifically bind the same antigen bound by the full-length antibody, and/or competes with the full-length antibody for specific binding to the antigen.

As used herein, "bispecific antibody" refers to a conjugate formed from a first antibody (fragment) and a second antibody (fragment) or an antibody analog via a coupling arm. Coupling methods include but are not limited to chemical reaction, gene fusion, protein fusion, polypeptide fusion and enzyme catalysis. "Multispecific antibody" includes, for example: trispecific antibody and tetraspecific antibody, the former is an antibody with three different antigen binding specificities, while the latter is an antibody with four different antigen binding specificities.

Herein, with regard to techniques for obtaining antibodies, conventional techniques known to those skilled in the art (for example, recombinant DNA technology or enzymatic or chemical fragmentation) can be used to obtain from a given antibody (for example, the antibody provided by the present disclosure) the antigen-binding fragments of the antibody (for example, the above-mentioned antibody fragments) which can be screened for specificity in the same manner by which intact antibodies are screened.

As used herein, the terms "monoclonal antibody", "McAb", "mAb" have the same meaning and can be used interchangeably, and refer to an antibody or a fragment of an antibody from a population of highly homologous antibody molecules, that is, a population of completely identical antibody molecules except for natural mutation that may occur spontaneously. Monoclonal antibodies are highly specific for single epitopes on antigens. Polyclonal antibodies are relative to monoclonal antibodies and typically contain at least two or more different antibodies that typically recognize different epitopes on antigens. Furthermore, the modifier "monoclonal" only indicates that the antibody is characterized as being obtained from a highly homologous antibody population and cannot be understood as requiring the preparation of the antibody by any particular method.

As used herein, the term "murine antibody" refers to a antibody obtained by fusing B cells from immunized mice with myeloma cells, and then screening mouse hybridoma cells that can both proliferate infinitely and secrete antibodies, followed by screening, antibody preparation and antibody purification, or obtained by B cells differentiating and proliferating to form plasma cells after antigens invade mice, which plasma cells can produce secretory antibodies. The production of antibodies stimulated by specific antigens is due to the interaction of various immune cells caused by antigens invading the human body, so that B cells in lymphocytes differentiate and proliferate to form plasma cells, which can produce secretory antibodies.

As used herein, the term "humanized antibody" herein refers to a genetically engineered non-human antibody whose amino acid sequence has been modified to increase sequence homology with a human antibody. Generally speaking, all or part of the CDR regions of a humanized antibody are derived from a non-human antibody (donor antibody), and all or part of the non-CDR regions (for example, variable region FR and/or constant region) are derived from human Immunoglobulin (recipient antibody). A humanized antibody generally retains the expected properties of a donor antibody, including but not limited to antigen specificity, affinity, reactivity, ability to improve immune cell activities, ability to enhance immune responses, etc. The donor antibody may be a mouse, rat, rabbit or non-human primate (e.g., cynomolgus monkey) antibody having the desired properties (e.g., antigen specificity, affinity, reactivity, ability to enhance immune cell activity and/or ability to enhance immune response).

Humanized antibodies are particularly advantageous because they can both retain the expected properties of non-human donor antibodies (e.g., murine antibodies) and effectively reduce the immunogenicity of non-human donor antibodies (e.g., murine antibodies) in human subjects. However, due to matching issues between the CDRs of the donor antibody and the FRs of the receptor antibody, the expected properties of humanized antibodies (e.g., antigen specificity, affinity, reactivity, ability to enhance immune cell activity and/or ability to enhance immune response) are generally lower than those of non-human donor antibodies (e.g., murine antibodies).

Therefore, although researchers in the art have conducted in-depth studies on the humanization of antibodies and have made some progress (see e.g., Jones et al., Nature, 321: 522 525 (1986); Reichmann et al., Nature, 332:323 329 (1988); Presta, Curr. Op. Struct. Biol., 2:593 596 (1992); and Clark, Immunol. Today 21: 397 402 (2000)), the prior art does not provide detailed guidance on how to sufficiently humanize a certain donor antibody so that the resulting humanized antibody has the highest possible degree of humanization while retaining the expected properties of the donor antibody as much as possible. Technicians need to explore, study and modify specific donor antibodies, and can possibly obtain humanized antibodies that not only have a high degree of humanization (e.g., at least 75%, at least 80%, at least 85%, at least 90%, at least 91%, at least 92%, at least 93%, at least 94%, at least 95%, at least 96%, at least 97%, at least 98%, at least 99% degree of humanization), but also retain the expected properties of specific donor antibodies only after undertaking substantial creative work.

In the present disclosure, in order to allow the humanized antibodies to retain the properties of the donor antibodies as much as possible (including, e.g., antigen specificity, affinity, reactivity, ability to enhance immune cell activity and/or ability to enhance immune response), the framework regions (FR) of the humanized antibodies of the present disclosure may comprise both amino acid residues of the human receptor antibodies and amino acid residues of the corresponding non-human donor antibodies.

As used herein, the term "degree of humanization" is an indicator used to evaluate the number of non-human amino acid residues in humanized antibodies. The degree of humanization of the humanized antibody can be predicted, for example, by the IMGT website DomainGapAlign, for the homology of the variable region sequence to the human V domain.

As used herein, the term "specific binding" refers to a non-random binding reaction between two molecules, such as the reaction between an antibody and the antigen it targets. The strength or affinity of a specific binding interaction may be expressed by the equilibrium dissociation constant (KD) or the half maximum effect concentration (EC₅₀) of the interaction.

The specific binding properties between the two molecules can be determined using methods known in the art. One method involves measuring the rate at which antigen binding sites or antigen complexes form and dissociate. Both "binding rate constant" (kₐ or kₒₙ) and "dissociation rate constant" (k_{dis} or k_{off}) can be calculated by concentration and actual rates of association and dissociation (see Malmqvist M, Nature, 1993, 361: 186-187). The ratio of kdis or kon is equal to the dissociation constant KD (see Davies et al., Annual Rev Biochem, 1990; 59: 439-473). KD, kₒₙ and k_{dis} values can be measured by any effective method. In certain embodiments, the dissociation constant may be measured using bioluminescence interferometry, such as the ForteBio Octet method. In addition, surface plasmon resonance techniques (e.g. Biacore) or Kinexa can be used to measure the dissociation constant.

As used herein, the term "vector" refers to a nucleic acid delivery vehicle in which polynucleotides can be inserted. When the vector enables a protein encoded by the inserted polynucleotide to be expressed, the vector is called an expression vector. Vectors can be transformed, transduced or transfected into host cells, and the genetic material elements carried by them can be expressed in host cells. Vectors are well known to those skilled in the art and include, but are not limited to: plasmid; phasmid; Cos plasmid; artificial chromosomes, such as yeast artificial chromosomes (YAC), bacterial artificial chromosomes (BAC) or P1-derived artificial chromosomes (PAC); bacteriophages such as lambda phage or M13 phage, animal viruses, etc. Animal viruses that may be used as vectors include, but are not limited to, retroviruses (including lentiviruses), adenoviruses, adeno-associated viruses, herpes viruses (such as herpes simplex virus), poxviruses, baculoviruses, papillomavirus, papovaviruses (such as SV40). A vector may contain a plurality of elements controlling expression, including but not limited to promoter sequences, transcription start sequences, enhancer sequences, selection elements, and reporter genes. In addition, the vector may also contain a replication initiation site.

As used herein, the term "host cell" refers to cells that can be used to introduce vectors, and includes but not limited to prokaryotic cells such as *Escherichia coli* or *Bacillus subtilis,* fungal cells such as yeast cells or *Aspergillus,* insect cells such as S2 *Drosophila* cells or Sf9 cells, or animal cells such as fibroblasts, CHO cells, COS cells, NSO cells, HeLa cells, BHK cells, HEK 293 cells or human cells. As used herein, the term "identity" is used to refer to the matching of sequences between two polypeptides or between two nucleic acids. In order to determine the percentage identity of two amino acid sequences or two nucleic acid sequences, the sequences are aligned for optimal comparison purposes (e.g., a gap may be introduced in the first amino acid sequence or nucleic acid sequence for optimal alignment with the second amino acid or nucleic acid sequence). The amino acid residues or nucleotides at corresponding amino acid positions or nucleotide positions are then compared. When a position in the first sequence is occupied by an amino acid residue or nucleotide that is the same as the corresponding position in the second sequence, the molecules are identical in that position. The percentage identity between two sequences is a function of the number of identical positions shared by the sequences (i.e., percentage identity = number of the identical overlapping positions/total number of positions × 100%). In certain embodiments, both sequences are of the same length.

The determination of percentage identity between the two sequences can also be achieved using mathematical algorithms. One non-limiting example of a mathematical algorithm for the comparison of two sequences is the algorithm of Karlin and Altschul, 1990, Proc. Natl. Acad. Sci. U.S.A. 87: 2264-2268, as modified in Karlin and Altschul, 1993, Proc. Natl. Acad. Sci. U.S.A. 90: 5873-5877. This algorithm is integrated into the NBLAST and XBLAST programs of Altschul et al., 1990, J. Mol. Biol. 215:403.

As used herein, the term "variant", in the context of polypeptides (including polypeptides), also refers to polypeptides or peptides containing amino acid sequences that have been altered by the introduction of amino acid residue substitution, deletion or addition. In certain cases, the term "variant" also refers to a polypeptide or peptide that has been modified (i.e., by covalently linking any type of molecule to the polypeptide or peptide). For example, but not by way of limitation, the polypeptide can be modified, such as by glycosylation, acetylation, pegylation, phosphorylation, amidation, derivatization by known protective blocking groups, proteolytic cleavage, attachment to cell ligands or other proteins, etc. Derived polypeptides or peptides may be produced by chemical modification using techniques known to those skilled in the art, and the techniques include but not limited to specific chemical cleavage, acetylation, formylation, metabolic synthesis of tunicamycin, and the like. In addition, variants have similar, identical or improved functions to the polypeptide or peptide from which they are derived.

As used herein, the term "conservative substitution" means an amino acid substitution that does not adversely affect or alter the expected properties of proteins or peptides containing amino acid sequences. For example, conserved substitutions may be introduced by standard techniques known in the art, such as site-directed mutagenesis and PCR-mediated mutagenesis. Conservative amino acid substitution includes substitution of amino acid residues with amino acid residues having similar side chains, for example, substitution with residues that are physically or functionally similar to the corresponding amino acid residues (e.g., having similar size, shape, charge, chemical properties, including the ability to form covalent or hydrogen bonds, etc.). Families of amino acid residues with similar side chains have been defined in the art. These families include amino acids with basic side chains (e.g., lysine, arginine and histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Therefore, it is preferable to replace the corresponding amino acid residue with another amino acid residue from the same side chain family. In addition, amino acid residues can be divided into categories defined according to optional physical and functional properties. For example, alcohol-containing residues (S and T), aliphatic residues (I, L, V and M), cycloalkenyl-related residues (F, H, W and Y), hydrophobic residues (A, C, F, G, H, I, L, M, R, T, V, W and Y), negatively charged residues (D and E), polar residues (C, D, E, H, K, N, Q, R, S, and T), positively charged residues (H, K and R), small residues (A, C, D, G, N, P, S, T and V), minimal residues (A, G and S), residues related to corner formation (A, C, D, E, G, H, K, N, Q, R, S, P and T), and flexible residues (Q, T, K, S, G, P, D, E, and R). Methods for identifying conservative substitutions of amino acids are well known in the art (see, e.g., Brummell et al., Biochem. 32: 1180-1187 (1993); Kobayashi et al. Protein Eng. 12 (10): 879-884 (1999); and Burks et al. Proc. Natl Acad. Set USA 94: 412-417 (1997), which are incorporated herein by reference).

The twenty conventional amino acids involved in the present disclosure are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present disclosure, the terms "polypeptide" and "protein" have the same meaning and are used interchangeably. In the present disclosure, amino acids are typically denoted by one-letter and three-letter abbreviations known in the art. For example, alanine can be represented by A or Ala.

As used herein, the term "linker" refers to the molecular moiety used to link an antibody targeting a specific antigen or a antigen-binding fragment thereof with a coupling moiety to form a conjugate. In some cases, the linker further comprises a spacer to change the steric hindrance of the coupling moiety. In some conjugates, linkers are essential. In other conjugates, linkers are not essential. The linker is divided into a non-cleavable linker and a cleavable linker. A conjugate containing the non-cleavable linker needs to degrade the antibody or the antigen-binding fragment thereof by internalization to release the coupling moiety; The conjugate containing the cleavable linker may release the coupling moiety by degrading the antibody or the antigen-binding fragment thereof by internalization, or may release the coupling moiety by disrupting the structure of the linker by enzymatically catalytic or chemically catalytic cleavage occurring at the cleavage site without internalization. Examples of the non-cleavable linker include N-succinimido-4-(N-maleimidomethyl)cyclohexane-1-carboxylate (SMCC). Examples of the cleavable linker include linkers with disulfide bonds as cleavage sites, hydrazone linkers with acyl groups as cleavage sites, linkers with enzymatically catalytic sites as cleavage sites (e.g., dipeptide linkers Val-Cit, Val-Ala; tetrapeptide linkers Gly-Gly-Phe-Gly; linkers containing phosphoric acid and pyrophosphoric acid).

The linker can link the antibody or the antigen-binding fragment thereof to the coupling moiety by covalently linking through a chemical functional group reaction by conjugation techniques known in the art. The coupling techniques may be chemically based specific in situ antibody modifications, for example, using the reaction between lysine residues and electrophilic groups to achieve linking, using the reaction of disulfide bonds to achieve linking, using thiol-rebridging to achieve linking, and using glycanosylation to achieve linking. The conjugation technique may also be site-specific bioconjugation based on engineered antibodies, such as the addition of enzyme-specifically recognized natural amino acids or non-natural amino acids to the antibody to enable the linking by enzymatic catalysis.

As used herein, the term "pharmaceutically acceptable carrier and/or excipient" refers to a carrier and/or excipient that is pharmacologically and/or physiologically compatible with the subject and the active ingredient and that is well known in the art (see, for example, Remington's Pharmaceutical Sciences. Edited by Gennaro AR, 19th ed. Pennsylvania: Mack Publishing Company, 1995), and includes but not limited to: pH regulators, surfactants, adjuvants, ionic strength enhancers, diluents, reagents for maintaining osmotic pressure, reagents for delayed absorption, preservatives. For example, the pH regulators include but are not limited to phosphate buffer. Surfactants include, but are not limited to, cationic, anionic, or nonionic surfactants, such as Tween-80. Ion strength enhancers include, but are not limited to, sodium chloride. Preservatives include, but are not limited to, various antibacterial agents and antifungal agents, such as araben, trichlorotert-butanol, phenol, sorbic acid, and the like. Reagents for maintaining osmotic pressure include, but are not limited to, sugars, NaCl and analogues thereof. Reagents for delayed absorption include, but are not limited to, monostearate and gelatin. Diluents include, but are not limited to, water, aqueous buffers (such as buffered saline), alcohols and polyols (such as glycerol), and the like. Preservatives include, but are not limited to, various antibacterial agents and antifungal agents, such as thimerosal, 2-phenoxyethanol, paraben, trichlorotert-butanol, phenol, sorbic acid, and the like. Stabilizers have a meaning commonly understood by those skilled in the art, are capable of stabilizing the expected activity of active ingredients in a drug. They include but not limited to, sodium glutamate, gelatin, SPGA, saccharides (e.g., sorbitol, mannitol, starch, sucrose, lactose, dextran, or glucose), amino acids (e.g., glutamic acid, glycine), proteins (e.g., dry whey, albumin or casein) or degradation products thereof (e.g., lactalbumin hydrolysates), etc.

As used herein, the term "prevention" refers to a method implemented to prevent or delay the onset of a disease or condition or symptom in a subject's body. As used herein, the term "treatment" refers to a method implemented in order to obtain a beneficial or desired clinical outcome. For the objective of the present disclosure, beneficial or necessary clinical outcomes include, but are not limited to, alleviating symptoms, narrowing the extent of the disease, stabilizing (i.e., preventing from getting worse) the state of the disease, delaying or slowing down disease progression, ameliorating or alleviating the state of the disease, reducing or suppressing the recurrence of the disease and relieving symptoms (whether partially or fully), whether detectable or undetectable. In addition, "treatment" can also refer to an extended survival period compared to the expected survival period (if not treated).

As used herein, the term "subject" refers to mammals, such as primates, such as humans. In certain embodiments, the subject (e.g., a person) has a tumor (e.g., a tumor expressing MSLN), an inflammatory disease, or an autoimmune disease, or is at risk of suffering from the above diseases.

As used herein, the term "effective amount" refers to an amount sufficient to obtain, or at least partially obtain, the desired effect. For example, an effective amount for preventing a disease (e.g., a tumor, an inflammatory disease, or an autoimmune disease) refers to an amount sufficient to prevent, stop, or delay the occurrence of a disease (e.g., a tumor, an inflammatory disease, or an autoimmune disease); Therapeutically effective amount refers to an amount sufficient to cure or at least partially stop a disease and its complications in a patient who already has the disease. Determining such an effective amount is well within the capabilities of the skilled person. For example, the effective amount for therapeutic use will depend on the severity of the disease to be treated, the general state of the patient's own immune system, the general condition of the patient such as age, weight and sex, the manner in which the drug is administered, other treatments administered at the same time, etc.

The terms "cancer" and "tumor" are used interchangeably and refer to a large group of diseases characterized by the uncontrolled growth of abnormal cells in the body. Unregulated cell division may lead to the formation of malignant tumors or cells that invade adjacent tissues and may metastasize to distal parts of the body through the lymphatic system or bloodstream. Cancers include benign and malignant cancers as well as dormant tumors or micrometastases. Cancers also include hematological malignancies.

As used herein, the term "pharmaceutically acceptable" means that a molecule itself, a molecular fragment, or a composition does not produce unfavorable, allergic, or other adverse reactions when properly administered to an animal or human. Specific examples of some substances that may be pharmaceutically acceptable carriers or components thereof include sugars (e.g. lactose), starches, cellulose and derivatives thereof, vegetable oils, gelatin, polyols (e.g. propylene glycol), alginate, and the like.

In this context, combination therapies includes the use of an anti-CDCP1 antibody or an antigen-binding fragment thereof encompassed by the present disclosure in combination with one or more additional active therapeutic agents of a second therapy (e.g., chemotherapeutic agents) or other prophylactic or therapeutic modality (e.g., radiation therapy).

Exemplary anticancer agents for the second therapy may include chemotherapeutic agents (e.g., mitosis inhibitors), alkylating agents (e.g., Nitrogen Mustard), antimetabolites (e.g., folate analogs), natural products (e.g., Vinca Alkaloid), multiple agents (e.g., platinum coordination complexes), hormones and antagonists (e.g., adrenal corticosteroids), immunomodulators (e.g., Bropirimine, Upjohn), etc. Other anticancer treatments include other antibodies that specifically target cancer cells.

In such combination therapies, various active agents often have different complementary mechanisms of action, and combination therapies may lead to synergistic effects. Combination therapies include therapeutic agents that affect immune responses (e.g., enhance or activate responses) and therapeutic agents that affect (e.g., inhibit or kill) tumors or cancer cells. Combination therapies reduce the likelihood of developing drug-resistant cancer cells. Combination therapies may allow one or more of the reagents to be dosed down to reduce or eliminate adverse effects associated with one or more of the reagents. Such combination therapies can have synergistic therapeutic or prophylactic effects on the underlying diseases, disorders or conditions.

Herein, "combination" includes therapies that can be administered separately, for example, therapies that are separately formulated for separated administration (e.g., can be provided in kits), and therapies that can be administered together as a single formulation (i.e., "co-formulation"). In certain embodiments, the anti-CDCP1 antibody or the antigen-binding fragment thereof of the present disclosure may be administered sequentially. In other embodiments, the anti-CDCP1 antibody or the antigen-binding fragment thereof may be administered simultaneously. The anti-CDCP1 antibody or the antigen-binding fragment thereof of the present disclosure may be used in combination with at least one other (active) agent in any manner.

### Beneficial effects of the invention

Compared to known anti-CDCP1 antibodies, the anti-CDCP1 antibodies provided in the present disclosure have obvious advantages, such as better target-binding capacity, tumor cell binding capacity, internalization capability, better capability to inhibit the migration of tumor cells, and better druggability. The present disclosure further provides an anti-CDCP1 antibody conjugate that can target CDCP1 positive cells. The antibody conjugate of the present disclosure can target CDCP1 positive cells with high affinity and high specificity, which is very beneficial for cancer treatment or *in vivo* diagnosis. Examples also demonstrate that the ADC of the present disclosure has strong killing activity against a variety of tumor cell lines expressing human CDCP1, such as colorectal cancer, breast cancer, and prostate cancer. Still further, the ADC of the present disclosure exhibits significant tumor inhibitory activity and has good safety in a mouse colorectal cancer xenograft tumor model.

Embodiments of the present disclosure will be described in detail below in conjunction with the drawings and examples, but it will be understood by those skilled in the art that the following drawings and examples are intended only to illustrate the present disclosure and not to limit the scope of the present disclosure. The various purposes and advantages of the present disclosure will become practicable to those skilled in the art according to the drawings and the following detailed description of preferred embodiments.

### BRIEF DESCRIPTION OF THE DRAWINGS

FIG. 1: structural schematic diagrams of CDCP1-C, CDCP1-A, and CDCP1-B;
FIG. 2: detection results of binding capacity of anti-CDCP1 antibody to tumor cells;
FIG. 3: laser confocal detection results of antibody internalization in tumor cell lines; in this figure, green indicates anti-CDCP1 antibody, red indicates lysosome, and arrows indicate intracellular colocalization of the antibody after endocytosis;
FIG. 4a: effect of anti-CDCP1 antibody on the proliferation of MDA-MB-231 tumor cells;
FIG. 4b: effect of anti-CDCP1 antibody on the proliferation of PC3 tumor cells;
FIG. 4c: Effect of anti-CDCP1 antibody on migration ability of tumor cells;
FIG. 5: Inhibitory effect of anti-CDCP1 chimeric antibody-vcMMAE conjugate on tumor cell growth;
FIG. 6a: inhibitory effect of anti-CDCP1 humanized antibody-vcMMAE conjugate on the growth of HCC1806 tumor cells;
FIG. 6b: inhibitory effect of anti-CDCP1 humanized antibody-vcMMAE conjugate on the growth of HCT116 tumor cells;
FIG. 7a: effect of anti-CDCP1 chimeric antibody-vcMMAE conjugate on body weight of mice;
FIG. 7b: inhibitory effect of anti-CDCP1 chimeric antibody-vcMMAE conjugate on tumor growth in mice.

### DETAILED DESCRIPTION OF THE PREFERRED EMBODIMENT

The present disclosure is now described with reference to the following examples which are intended to illustrate, but not to limit, the present disclosure.

The information of the sequence to which the present disclosure relates is described in Table 1 below:

**Table 1 Amino acid sequence of antibody**

| SEQ ID NO: | Description | Sequence |
|---|---|---|
| 1 | IMGT 3B12H9/3B12H5 CDR-H1 | GYSFTIYW |
| 2 | IMGT 3C11 CDR-H1 | GYNFISYW |
| 3 | IMGT 5G9 CDR-H1 | GYIFLNYW |
| 4 | IMGT 4E12G10/SB4/SB14 CDR-H1 | GYTFTNYW |
| 5 | IMGT 6A7 CDR-H1 | GYSFTSYW |
| 6 | IMGT 6G9/3F1A11 CDR-H1 | GYSITSDYA |
| 7 | IMGT 20G4/27D11/3F1A11-H-12 CDR-H1 | GYSITSGYY |
| 8 | IMGT | GDSITTGY |
| | 3A9 CDR-H1 | |
| 9 | IMGT 19F7D6 CDR-H1 | GFTFSTYT |
| 10 | IMGT 3E7/SB2/SB15 CDR-H1 | GFTFSSYT |
| 11 | IMGT 5B8/8C9 CDR-H1 | GLTFTHYH |
| 12 | IMGT 4B7 CDR-H1 | GLTFTYYH |
| 13 | IMGT 12E6/G10F6/14A10C5/1C P-064 CDR-H1 | GFSLSTSGMG |
| 14 | IMGT 8E5 CDR-H1 | GYTLTDFS |
| 15 | IMGT 27A3 CDR-H1 | GYTFTYYS |
| 16 | IMGT 10D2 CDR-H1 | GYTFTDYS |
| 17 | IMGT 24G4/1CP-065/1CP-068 CDR-H1 | GFNIKDYY |
| 18 | IMGT 1CP-061 CDR-H1 | GYTFTSYW |
| 19 | IMGT 1CP-063 CDR-H1 | GYTFTSYY |
| 20 | IMGT 1CP-062 CDR-H1 | GFSLTTYG |
| 21 | IMGT 1CP-067/1CP-069 CDR-H1 | GFTFSVYG |
| 22 | IMGT 1CP-066 CDR-H1 | GYAFTKYG |
| 23 | IMGT SB1 CDR-H1 | GYTFTDHA |
| 24 | IMGT 3E7/SB2/SB15 CDR-H1 | GFTFSSYT |
| 25 | IMGT SB3 CDR-H1 | GFPFSSYT |
| 26 | IMGT 4E12G10/SB4/SB14 CDR-H1 | GYTFTNYW |
| 27 | IMGT SB5 CDR-H1 | GFSLTDFG |
| 28 | IMGT SB6 CDR-H1 | GFTFNSSY |
| 29 | IMGT SB7 CDR-H1 | GYTFTDYA |
| 30 | IMGT SB8 CDR-H1 | GFSLSTSGVG |
| 31 | IMGT SB9 CDR-H1 | GFTFSSFT |
| 32 | IMGT SB10/SB17 CDR-H1 | GFSLSTSGM |
| 33 | IMGT SB11 CDR-H1 | GFNIQDTY |
| 34 | IMGT SB12 CDR-H1 | GYTFSRYW |
| 35 | IMGT SB13 CDR-H1 | GFTFSDFY |
| 36 | IMGT SB16 CDR-H1 | GYPFTDYA |
| 37 | IMGT SB18 CDR-H1 | GFSLTTSGM |
| 38 | IMGT 3B12H9/3B12H5 CDR-H2 | VYPGNDDT |
| 39 | IMGT 3C11/5G9 CDR-H2 | IYPGNTDT |
| 40 | IMGT 4E12G10/SB4 CDR-H2 | NYPGDNYR |
| 41 | IMGT 6A7 CDR-H2 | IHPSDSET |
| 42 | IMGT 6G9 CDR-H2 | IIYSGRT |
| 43 | IMGT 3F1A11 CDR-H2 | ISYNTYT |
| 44 | IMGT 20G4/27D11/3F1A11-H-12 CDR-H2 | ITYDDSH |
| 45 | IMGT 3A9 CDR-H2 | ILYSGST |
| 46 | IMGT 19F7D6/SB2/SB3 CDR-H2 | ISSGGIYT |
| 47 | IMGT 3E7 CDR-H2 | ISNGGGNT |
| 48 | IMGT 5B8/4B7/8C9 CDR-H2 | IRNKAYGYTT |
| 49 | IMGT 12E6/1CP-064 CDR-H2 | IWWDDVK |
| 50 | IMGT G10F6/SB10 CDR-H2 | IYWDDDK |
| 51 | IMGT 14A10C5 CDR-H2 | IYWDDEK |
| 52 | IMGT 8E5 CDR-H2 | INTETGAP |
| 53 | IMGT 27A3 CDR-H2 | INTETGEP |
| 54 | IMGT 10D2 CDR-H2 | INTETGQP |
| 55 | IMGT 24G4 CDR-H2 | IDPENGDT |
| 56 | IMGT 1CP-061CDR-H2 | IYPSDSYT |
| 57 | IMGT 1CP-063 CDR-H2 | IYPGNVNS |
| 58 | IMGT 1CP-062CDR-H2 | IWSDGNT |
| 59 | IMGT 1CP-067 CDR-H2 | LSGGSNYT |
| 60 | IMGT 1CP-069 CDR-H2 | LSGGDNYT |
| 61 | IMGT 1CP-066CDR-H2 | INTYTGEA |
| 62 | IMGT 1CP-065CDR-H2 | IDPENDNT |
| 63 | IMGT 1CP-068CDR-H2 | IDPENGNT |
| 64 | IMGT SB1 CDR-H2 | ISFSYDNT |
| 65 | IMGT 19F7D6/SB2/SB3 CDR-H2 | ISSGGIYT |
| 66 | IMGT 4E12G10/SB4 CDR-H2 | NYPGDNYR |
| 67 | IMGT SB5 CDR-H2 | IWANGRI |
| 68 | IMGT SB6 CDR-H2 | IFAGTGGT |
| 69 | IMGT SB7 CDR-H2 | KSVYYGIT |
| 70 | IMGT SB8 CDR-H2 | IWWDDEK |
| 71 | IMGT SB9 CDR-H2 | ISSSGSYT |
| 72 | IMGT G10F6/SB10 CDR-H2 | IYWDDDK |
| 73 | IMGT SB11 CDR-H2 | IDPANDNT |
| 74 | IMGT SB12 CDR-H2 | ILPGSDFT |
| 75 | IMGT SB13 CDR-H2 | SRDKTNDYTT |
| 76 | IMGT SB14 CDR-H2 | IFPGTGTT |
| 77 | IMGT SB15 CDR-H2 | ISGGINYT |
| 78 | IMGT SB16 CDR-H2 | IAPYSDNT |
| 79 | IMGT SB17/SB18 CDR-H2 | IWWNDDK |
| 80 | IMGT 3B12H9/3B12H5 CDR-H3 | TRHTYDEVFDY |
| 81 | IMGT 3C11/5G9 CDR-H3 | IRYDYDRGMDY |
| 82 | IMGT 4E12G10 CDR-H3 | TRSGGTRYYTMDY |
| 83 | IMGT 6A7 CDR-H3 | TINYESFYYAMDY |
| 84 | IMGT 6G9 CDR-H3 | AREDYYGGSYGGLDY |
| 85 | IMGT 3F1A11 CDR-H3 | AGGDYYGTGAVDY |
| 86 | IMGT 20G4/27D11/3F1A11-H-12 CDR-H3 | ARGNYYANYPHAMDY |
| 87 | IMGT 3A9 CDR-H3 | ARGDYGAMDY |
| 88 | IMGT 19F7D6/SB2/SB3 CDR-H3 | SSPHSYAISYGYFDY |
| 89 | IMGT 3E7 CDR-H3 | ASSYGNYRGAWFAY |
| 90 | IMGT 5B8/8C9 CDR-H3 | VREGGYGYDGTFAY |
| 91 | IMGT 4B7 CDR-H3 | AREGGYGYDGTFAY |
| 92 | IMGT 12E6 CDR-H3 | ARIDHGYGSNYWDFDV |
| 93 | IMGT G10F6 CDR-H3 | ARVPGNWDEDAMDY |
| 94 | IMGT 14A10C5 CDR-H3 | AREGSANWEAMDY |
| 95 | IMGT 8E5 CDR-H3 | SRGRDYDPHFDY |
| 96 | IMGT 27A3 CDR-H3 | ANAMDY |
| 97 | IMGT 10D2 CDR-H3 | TRTFYYDYVGDY |
| 98 | IMGT 24G4 CDR-H3 | NPCGTGPWFAY |
| 99 | IMGT 1CP-061 CDR-H3 | TRCLRRRDWYFDV |
| 100 | IMGT 1CP-063 CDR-H3 | AREDYGHYYAMDY |
| 101 | IMGT 1CP-062 CDR-H3 | AKNYRNYDYAMDY |
| 102 | IMGT 1CP-067/1CP-069 CDR-H3 | ATEDGLIGTWGDY |
| 103 | IMGT 1CP-064 CDR-H3 | ARMGGKVPWFAY |
| 104 | IMGT 1CP-066 CDR-H3 | SRGDDYGTGGYVLFY |
| 105 | IMGT 1CP-065 CDR-H3 | ARFYGSYYYAMDY |
| 106 | IMGT 1CP-068 CDR-H3 | AGRLYAMEY |
| 107 | IMGT SB1 CDR-H3 | ARDCYGSNRGYFDY |
| 108 | IMGT 19F7D6/SB2/SB3 CDR-H3 | SSPHSYAISYGYFDY |
| 109 | IMGT SB4 CDR-H3 | TRSGGSRYYTMDY |
| 110 | IMGT SB5 CDR-H3 | ARDHDYDVEAMDY |
| 111 | IMGT SB6 CDR-H3 | ARHGLGYDY |
| 112 | IMGT SB7 CDR-H3 | ARDSYGSSRGYFDY |
| 113 | IMGT SB8 CDR-H3 | VKEGDGMDY |
| 114 | IMGT SB9 CDR-H3 | TSPDYYGRSYGYFDY |
| 115 | IMGT SB10 CDR-H3 | TREGSADWEALDY |
| 116 | IMGT SB11 CDR-H3 | AREDAYPWFAY |
| 117 | IMGT SB12 CDR-H3 | ARKDYYGNYDYAMDY |
| 118 | IMGT SB13 CDR-H3 | ARDGVYYYGSSYTLDY |
| 119 | IMGT SB14 CDR-H3 | ARTNWEGYYFDY |
| 120 | IMGT SB15 CDR-H3 | ARHDRYDGYYYVMDY |
| 121 | IMGT SB16 CDR-H3 | AAYGNYGYYTMDY |
| 122 | IMGT SB17 CDR-H3 | ARIGYYYGTYALDY |
| 123 | IMGT SB18 CDR-H3 | ARLRDGGFAY |
| 124 | IMGT 3B12H9/27A3/SB9 CDR-L1 | QSLLDSDGKTY |
| 125 | IMGT 3B12H5/3C11 CDR-L1 | EDIYYR |
| 126 | IMGT 5G9/24G4/1CP-068/SB 17 CDR-L1 | SSVSY |
| 127 | IMGT 4E12G10/1CP-067/1CP-069 CDR-L1 | KSLLHSNGITY |
| 128 | IMGT 6A7 CDR-L1 | KSVSTSGYSY |
| 129 | IMGT 6G9 CDR-L1 | QSIVHSNGNTY |
| 130 | IMGT 3F1A11/3F1A11-H-12 CDR-L1 | QTLVHTNGNTY |
| 131 | IMGT 20G4 CDR-L1 | ESVDHNGISF |
| 132 | IMGT 27D11 CDR-L1 | ESVDNSGISF |
| 133 | IMGT 3A9 CDR-L1 | QSIVHSNGKTY |
| 134 | IMGT 19F7D6/SB2 CDR-L1 | QSLLDIDGKTY |
| 135 | IMGT 3E7 CDR-L1 | QSLLYSNNQRNY |
| 136 | IMGT 5B8/4B7/8C9 CDR-L1 | ESVDNYGISF |
| 137 | IMGT 12E6 CDR-L1 | KSLLHSDGKTY |
| 138 | IMGT G10F6 CDR-L1 | QNIVHTNGNTY |
| 139 | IMGT 14A10C5 CDR-L1 | QNIVYTNGITY |
| 140 | IMGT 8E5 CDR-L1 | QSISDF |
| 141 | IMGT 10D2/1CP-065 CDR-L1 | QDVSTA |
| 142 | IMGT 1CP-061CDR-L1 | ESVDYSGYSF |
| 143 | IMGT 1CP-063CDR-L1 | QDIKSY |
| 144 | IMGT 1CP-062 /1CP-064 CDR-L1 | ESVDSYGNSF |
| 145 | IMGT 1CP-066 CDR-L1 | ENIYNN |
| 146 | IMGT SB1 CDR-L1 | QDINRF |
| 147 | IMGT 19F7D6/SB2 CDR-L1 | QSLLDIDGKTY |
| 148 | IMGT SB3 CDR-L1 | QSLLDFDGKTY |
| 149 | IMGT SB4 CDR-L1 | RSLLHNNGITY |
| 150 | IMGT SB5 CDR-L1 | SSVIY |
| 151 | IMGT SB6 CDR-L1 | QSVNTD |
| 152 | IMGT SB7 CDR-L1 | QDINSF |
| 153 | IMGT SB8 CDR-L1 | TGAVATSNY |
| 154 | IMGT 3B12H9/27A3/SB9 CDR-L1 | QSLLDSDGKTY |
| 155 | IMGT SB10 CDR-L1 | QTIVYSNGNSY |
| 156 | IMGT SB11 CDR-L1 | QSVSTSSYSY |
| 157 | IMGT SB12 CDR-L1 | KSIGKY |
| 158 | IMGT SB13 CDR-L1 | QDVSSS |
| 159 | IMGT SB14 CDR-L1 | SSVRSSY |
| 160 | IMGT SB15 CDR-L1 | QEISGY |
| 161 | IMGT SB16 CDR-L1 | QDITNY |
| 162 | IMGT 5G9/24G4/1CP-068/SB 17 CDR-L1 | SSVSY |
| 163 | IMGT SB18 CDR-L1 | QAISNY |
| 164 | IMGT 3B12H9/19F7D6/27A3/SB 2/SB3/SB9 CDR-L2 | LVS |
| 165 | IMGT 3B12H5 CDR-L2 | GGT |
| 166 | IMGT 3C11 CDR-L2 | GAT |
| 167 | IMGT 5G9 CDR-L2 | RTS |
| 168 | IMGT 4E12G10/1CP-067/1CP-069/SB4 CDR-L2 | QMS |
| 169 | IMGT 6A7/1CP-064 CDR-L2 | LAS |
| 170 | IMGT 6G9/3F1A11/3F1A11-H-12/3A9/G10F6/14A10C5/S B10 CDR-L2 | KVS |
| 171 | IMGT 20G4/27D 1 1/5B8/4B7/8C9 /SB15 CDR-L2 | AAS |
| 172 | IMGT 3E7/10D2/1CP-065/SB13 CDR-L2 | WAS |
| 173 | IMGT 12E6 CDR-L2 | RMS |
| 174 | IMGT 24G4 CDR-L2 | ATS |
| 175 | IMGT 8E5/SB11 CDR-L2 | YAS |
| 176 | IMGT 1CP-061/1CP-062 CDR-L2 | RAS |
| 177 | IMGT 1CP-063CDR-L2 | YAT |
| 178 | IMGT 1CP-066 CDR-L2 | AAT |
| 179 | IMGT 1CP-068 CDR-L2 | LTS |
| 180 | IMGT SB1/SB7 CDR-L2 | RAN |
| 181 | IMGT 3B12H9/19F7D6/27A3/SB 2/SB3/SB9 CDR-L2 | LVS |
| 182 | IMGT 4E12G10/1CP-067/1CP-069/SB4 CDR-L2 | QMS |
| 183 | IMGT SB5 CDR-L2 | DTS |
| 184 | IMGT SB6 CDR-L2 | SAS |
| 185 | IMGT SB1/SB7 CDR-L2 | RAN |
| 186 | IMGT SB8 CDR-L2 | GTN |
| 187 | IMGT 3B12H9/19F7D6/27A3/SB 2/SB3/SB9 CDR-L2 | LVS |
| 188 | IMGT 6G9/3F1A11/3F1A11-H-12/3A9/G10F6/14A10C5/S B10 CDR-L2 | KVS |
| 189 | IMGT 8E5/SB11 CDR-L2 | YAS |
| 190 | IMGT SB12 CDR-L2 | SGS |
| 191 | IMGT 3E7/10D2/1CP-065/SB13 CDR-L2 | WAS |
| 192 | IMGT SB14 CDR-L2 | STS |
| 193 | IMGT 20G4/27D11/5B8/4B7/8C9 /SB15 CDR-L2 | AAS |
| 194 | IMGT SB16/SB18 CDR-L2 | YTS |
| 195 | IMGT SB17 CDR-L2 | DAS |
| 196 | IMGT SB16/SB18 CDR-L2 | YTS |
| 197 | IMGT 3B12H9 CDR-L3 | WQGTHFPWT |
| 198 | IMGT 3B12H5 CDR-L3 | QHYWNTPWT |
| 199 | IMGT/Kabat/AbM/Chothia 3C11 CDR-L3 | QHYWNIPWT |
| 200 | IMGT 5G9 CDR-L3 | QQYHSYPYT |
| 201 | IMGT 4E12G10/SB4 CDR-L3 | AQNLDLPYT |
| 202 | IMGT 6A7 CDR-L3 | LHSRELPYT |
| 203 | IMGT 6G9/3F1A11/3F1A11-H-12/3A9 CDR-L3 | FQGSHVPWT |
| 204 | IMGT 20G4/27D11 CDR-L3 | QQNKEVPWT |
| 205 | IMGT 19F7D6/SB2/SB9 CDR-L3 | WQGTHFPYT |
| 206 | IMGT 3E7 CDR-L3 | QQYYSYPWT |
| 207 | IMGT/Kabat/AbM/Chothia 5B8/4B7/8C9 CDR-L3 | QQSKEVPRT |
| 208 | IMGT 12E6 CDR-L3 | MQHLEYPFT |
| 209 | IMGT G10F6 CDR-L3 | FQGSHVPYT |
| 210 | IMGT 14A10C5/SB10 CDR-L3 | FQSSHVPWT |
| 211 | IMGT/Kabat/AbM/Chothia 8E5 CDR-L3 | QSAHSFPYT |
| 212 | IMGT 27A3 CDR-L3 | WQGTHFPHT |
| 213 | IMGT 10D2 CDR-L3 | QQHSSTPYT |
| 214 | IMGT 24G4 CDR-L3 | QQWNSSPFT |
| 215 | IMGT 1CP-061 /1CP-062 CDR-L3 | QQSNEDPYT |
| 216 | IMGT 1CP-063 CDR-L3 | LQYGESPWT |
| 217 | IMGT 1CP-067/1CP-069 CDR-L3 | AQNLELPFT |
| 218 | IMGT 1CP-064 CDR-L3 | QQNNEDPWT |
| 219 | IMGT 1CP-066 CDR-L3 | QHFWGTPPT |
| 220 | IMGT 1CP-065 CDR-L3 | QQHYSTPYT |
| 221 | IMGT 1CP-068 CDR-L3 | QQWSSDPFT |
| 222 | IMGT SB1/SB7 CDR-L3 | LQYDEFPRT |
| 223 | IMGT 19F7D6/SB2/SB9 CDR-L3 | WQGTHFPYT |
| 224 | IMGT SB3 CDR-L3 | WQGTHFPFT |
| 225 | IMGT 4E12G10/SB4 CDR-L3 | AQNLDLPYT |
| 226 | IMGT SB5 CDR-L3 | QQWSSYPPT |
| 227 | IMGT SB6 CDR-L3 | QQDHSSPPWT |
| 228 | IMGT SB8 CDR-L3 | ALWYSNHWV |
| 229 | IMGT 19F7D6/SB2/SB9 CDR-L3 | WQGTHFPYT |
| 230 | IMGT 14A10C5/SB10 CDR-L3 | FQSSHVPWT |
| 231 | IMGT SB11 CDR-L3 | QHNWEIPWT |
| 232 | IMGT SB12 CDR-L3 | QQHDEYPWT |
| 233 | IMGT SB13 CDR-L3 | QQHYNTPPT |
| 234 | IMGT SB14 CDR-L3 | QQYSGSPSWT |
| 235 | IMGT SB15 CDR-L3 | LQYASYPYT |
| 236 | IMGT SB16 CDR-L3 | QQGNTLPWT |
| 237 | IMGT SB17 CDR-L3 | QQWSSYPLT |
| 238 | IMGT SB18 CDR-L3 | QQYSKIPWT |
| 239 | 3B12H9/3B12H5 heavy chain variable region | EVQLQQSGTVLARPGASVKMSCKASGYSFTIYWVHW VKQRPGQGLEWIGAVYPGNDDTSYNQNFKGKAKLTAV TSATTAYMELSSLTIDDSAVYYCTRHTYDEVFDYWGQG TTVTVSS |
| 240 | 3C11 heavy chain variable region | EVQLQQSGTVLARPGASVKMSCKASGYNFISYWLHWI KQRPGQGLEWIGGIYPGNTDTTYNQKFKGKAKVTAVT SATTAYMELSSLTNEDSAVYYCIRYDYDRGMDYWGQG TTVTVSS |
| 241 | 5G9 heavy chain variable region | EVQLQQSGTVLARPGASVKMSCKASGYIFLNYWMHW VKQRPGQGLEWIGGIYPGNTDTTYNQKFKGKAKVTAV TSATTAYMELSSLTNEDSAVYYCIRYDYDRGMDYWGQ GTTVTVSS |
| 242 | 4E12G10 heavy chain variable region | EVQLQQSGAELVRPGTSVKISCKASGYTFTNYWLGWV KQRPGHGLEWIGDNYPGDNYRNFNEKFKGKATLTTDA SSSTAYLQLSSLISEDSAVYFCTRSGGTRYYTMDYWGQ GTTVTVSS |
| 243 | 6A7 heavy chain variable region | EVQLKESGAELVRPGASVKVSCKASGYSFTSYWMNW VKQRPGQGLEWIGMIHPSDSETKLNQKFRDRASLTVDK SSSTAYMQLSSPTSEDSAVYYCTINYESFYYAMDYWGQ GTTVTVSS |
| 244 | 6G9 heavy chain variable region | EVQLKESGPGLVKPSQSLSLTCTVTGYSITSDYAWNWIR QFPGNKLEWMGYIIYSGRTSYNPSLKSRISITRDTSKNQ FFLQLNSVTTEDTATYYCAREDYYGGSYGGLDYWGQG TTVTVSS |
| 245 | 3F1A11 heavy chain variable region | EVKLVESGPGLVKPSQSLSLTCTVTGYSITSDYAWNWIR QFPGNKLEWMGYISYNTYTSYNPSLKSRISVTRDTSKN QFFLQLKSVTIEDTATYFCAGGDYYGTGAVDYWGQGT TVTVSS |
| 246 | 20G4 heavy chain variable region | EVQLKESGPGLVKPSQSLSLTCSVTGYSITSGYYWNWI RQFPGNKLDWMGYITYDDSHYYNPSLKDRLSITRDTS KNQFFLKLNSVTAEDTATYFCARGNYYANYPHAMDY WGQGTTVTVSS |
| 247 | 27D11 heavy chain variable region | EVTLKESGPGLVKPSQSLSLTCSVTGYSITSGYYWNWIR QFPGNKLDWMGYITYDDSHYYNPSLKHRLSITRDTSK NQFFLKLNSVTAEDTATYFCARGNYYANYPHAMDYW GQGTTVTVSS |
| 248 | 3F1A11-H-12 heavy chain variable region | EVKLVESGPGLVKPSQSLSLTCSVTGYSITSGYYWNWIR QFPGNKLDWMGYITYDDSHYYNPSLKDRLSITRDTSK NQFFLKLNSVTAEDTATYFCARGNYYANYPHAMDYW GQGTTVTVSS |
| 249 | 3A9 heavy chain variable region | EVKLVESGSSLVKPSQTLSLTCSVTGDSITTGYWNWIRK FPGNKLEYMGYILYSGSTYYNPSLKSRISITRDTSKNQY YLQLNSVTTEDTATYYCARGDYGAMDYWGQGTTVTV SS |
| 250 | 19F7D6 heavy chain variable region | EVKLVESGGGLVKPGGSLKLSCAASGFTFSTYTMSWV RQTPEKRLEWVATISSGGIYTYYPDSVKGRFTISRDNAK DTLYLQMSSLRSEDTAVYYCSSPHSYAISYGYFDYWGQ GTTVTVSS |
| 251 | 3E7 heavy chain variable region | EVQLKESGGGLVQPGGSLQLSCAASGFTFSSYTMSWV RQTLEKRLEWVAYISNGGGNTYYPDTLKGRFTISRDNA KNILYLQLSSLKSEDTAMYYCASSYGNYRGAWFAYWG QGTTVTVSS |
| 252 | 5B8 heavy chain variable region | EVQLKESGGGLVQPGGSLRISCATSGLTFTHYHMSWVR QPPGKAPEWLAIIRNKAYGYTTDYSASVRGRFTISRDDS QSILYLQMNTLRAEDSATYYCVREGGYGYDGTFAYWG QGTTVTVSS |
| 253 | 4B7 heavy chain variable region | EVQLQQSGGGLVQPGGSLRLSCATSGLTFTYYHMSWV RQPPGKAPEWLAIIRNKAYGYTTEYSASVKGRFTISRD DSQSILYLQMNTLRTEDSATYYCAREGGYGYDGTFAY WGQGTTVTVSS |
| 254 | 8C9 heavy chain variable region | EVQLKESGGGLVQPGGSLRISCATSGLTFTHYHMSWVR QPPGKAPEWLAIIRNKAYGYTTDYSASVRGRFTISRDDS QSILYLQMNTLRPEDSATYYCVREGGYGYDGTFAYWG QGTTVTVSS |
| 255 | 12E6 heavy chain variable region | EVQLKESGPGILQPSQTLSLTCSFSGFSLSTSGMGVGWI RQPSGKGLEWLAHIWWDDVKLYNPALKSRLTISKETSN NQVFLKIASVDAADTATYYCARIDHGYGSNYWDFDV WGAGTTVTVSS |
| 256 | G10F6 heavy chain variable region | EVKLVESGPGILQPSQTLSLTCSFSGFSLSTSGMGVSWV RQPSGKGLEWLAHIYWDDDKRYNPSLKGRLTISKDTPR NQVFLEITSVDTTDTATYYCARVPGNWDEDAMDYWG QGTTVTVSS |
| 257 | 14A10C5 heavy chain variable region | EVKLVESGPGILQPSQTLSLTCSFSGFSLSTSGMGVSWIR QPSGKGLEWLAHIYWDDEKRYNPSLKSRLTISKDTSSN QVFLKITSVDTADTATYYCAREGSANWEAMDYWGQG TTVTVSS |
| 258 | 8E5 heavy chain variable region | EVQLKESGPELKKPGETVKISCKASGYTLTDFSMHWLK QAPGKGLKWMGWINTETGAPTYADDFKGRFALSLDTS ANTAYLQINNLKNEDTATYFCSRGRDYDPHFDYWGQG TTVTVSS |
| 259 | 27A3 heavy chain variable region | EVQLKESGPELKKPGETVKISCKASGYTFTYYSMHWV KQTPGKGLKWMGWINTETGEPTYADDFKGRFAFSLET SDSTAYLQINNLKNEDTATYFCANAMDYWGQGTTVTV SS |
| 260 | 10D2 heavy chain variable region | EVQLQQSGPELKKPGETVKISCKASGYTFTDYSMHWV KQAPGKGLKWMGWINTETGQPTYADDFKGRFAFSLET SASSAYLQINNLKNEDTATYFCTRTFYYDYVGDYWGQ GTTVTVSS |
| 261 | 24G4 heavy chain variable region | EVQLKESGAELVRSGASVKLSCTASGFNIKDYYIHWVK QRPEQGLEWIGWIDPENGDTEYVPKFQGKATLTADTSS NTAYLQLNSLTSEDTAVYYCNPCGTGPWFAYWGQGTT VTVSS |
| 262 | 1CP-061 heavy chain variable region | EVQLKESGAELVRPGASVKLSCKASGYTFTSYWINWV RQRPGQGLEWIGNIYPSDSYTNYNQKFKDKATLTVDKS SSTAYMQLSSPTSEDSAVYYCTRCLRRRDWYFDVWGQ GTTVTVSS |
| 263 | 1CP-063 heavy chain variable region | EVNLKESGPELVKPGASVRISCKASGYTFTSYYIHWVK QRPGQGLEWIGWIYPGNVNSKYNEKFKGKVTLTADKS SITAYMQLSSLTSEDSAVYFCAREDYGHYYAMDYWGQ GTTVTVSS |
| 264 | 1CP-062 heavy chain variable region | EVKLVESGPGLVAPSQSLSITCTVSGFSLTTYGVHWVRQ PPGKGLEWLVVIWSDGNTTYNSALKSRLSINKDNSKSQ VFLKMNSLQTDDTAMYYCAKNYRNYDYAMDYWGQG TTVTVSS |
| 265 | 1CP-067 heavy chain variable region | EVQLKESGGGLVKPGGSLKLSCTASGFTFSVYGMSWV RQTPEKRLEWVATLSGGSNYTYYSDSVKGRFTISRDTG KNNLYLQMSSLSSDDTALYYCATEDGLIGTWGDYWGQ GTTVTVSS |
| 266 | 1CP-069 heavy chain variable region | EVQLQQSGGGLVKPGGSLKLSCTASGFTFSVYGMSWV RQTPEKRLEWVATLSGGDNYTYYTDSVKGRFTISRDTA KNNLYLQMSSLRSEDTALYFCATEDGLIGTWGDYWGQ GTTVTVSS |
| 267 | 1CP-064 heavy chain variable region | EVKLVESGPGILQPSQTLSLTCSFSGFSLSTSGMGVGWI RQPSGKGLEWLAHIWWDDVKRYNPALKSRLTISKDTSS SQVFLKIASVDTADTATYYCARMGGKVPWFAYWGQGT TVTVSS |
| 268 | 1CP-066 heavy chain variable region | EVTLKESGPELKKPGETVKISCKASGYAFTKYGMNWV KQAPGKDLKFMGWINTYTGEATYADDFKGRFDFSLEA SASTAFLRINNLKNEDTATYFCSRGDDYGTGGYVLFYW GQGTTVTVSS |
| 269 | 1CP-065 heavy chain variable region | EVTLKESGAELVRPGALVKLSCKASGFNIKDYYMHWV KQRPEQGLEWIGWIDPENDNTIYDPKFQGKASITADTSS NTAYLQLSSLTSEDTAVYYCARFYGSYYYAMDYWGQG TTVTVSS |
| 270 | 1CP-068 heavy chain variable region | EVQLKESGAELVRPGALVKLSCKASGFNIKDYYMYWV KQRPEQGLEWIGWIDPENGNTIYDPKLQGKARITADTS SNTAYLQLSSLTSEDTAVYYCAGRLYAMEYWGQGTTVT VSS |
| 271 | SB1 heavy chain variable region | QVQLQQSGPDLVRPGESVKISCKGSGYTFTDHAIHWVR QSHAKSLEWIGVISFSYDNTKYNQNFKGKATMTVDKS SNTAYMELARLTSEDSAIYYCARDCYGSNRGYFDYWG QGTTLTVSS |
| 272 | SB2 heavy chain variable region | DVKLVESGGGLVKPGGSLKLSCAASGFTFSSYTMSWV RQTPEKRLEWVATISSGGIYTYYPDSVKGRFTISRDNAK DTLYLQMNSLRSEDTAVYYCSSPHSYAISYGYFDYWGQ GTTLTVSS |
| 273 | SB3 heavy chain variable region | DVKLVESGGGLVEPGGSLKLSCAASGFPFSSYTMSWVR QTPEKRLEWVATISSGGIYTYYPDSVKGRFTISRDNAKD TLYLQMSSLRSEDTAVYYCSSPHSYAISYGYFDYWGQG TTLTVSS |
| 274 | SB4 heavy chain variable region | QVQLQQSGAELVRPGTSVKISCKASGYTFTNYWLGWV KQRPGHGLEWIGDNYPGDNYRNFNEKFKGKATLTTDA SSSTAYLQLSSLISEDSAVYFCTRSGGSRYYTMDYWGQ GTSVTVSS |
| 275 | SB5 heavy chain variable region | QVQLRESGPGLVAPSQSLSITCTVSGFSLTDFGVNWVRQ PPGKGLEWLGIIWANGRIEYNSALKSRLSISKDNSRSQV FLKLNSLQTDDTARYYCARDHDYDVEAMDYWGQGTS VTVSA |
| 276 | SB6 heavy chain variable region | QGQMQQSGAELVKPGASVKLSCKTSGFTFNSSYITWLK QKPGQSLEWIAWIFAGTGGTYYNEKFTDKAQLTVDTTS STAYMQFSSLTTEDSAIYYCARHGLGYDYWGQGTTLTV SS |
| 277 | SB7 heavy chain variable region | QVQLQQSGPELVSPGESVKISCKGSGYTFTDYAVHWVK QSHSKSLEWIGVKSVYYGITKYSQNFKDKATMTVDKS SSTAYMELARLTSEDSAIYYCARDSYGSSRGYFDYWGQ GTTLTVSS |
| 278 | SB8 heavy chain variable region | QVTLKESGPGILKPSQTLSLTCSFSGFSLSTSGVGVGWIR QPSGKGLEWLAHIWWDDEKYYNPSLKSHFTISKDTSR NQVFLKITSVDTADTATYYCVKEGDGMDYWGQGTSVT VSS |
| 279 | SB9 heavy chain variable region | DVKLVESGGGLVKPGGSLKLSCAASGFTFSSFTMSWVR QTPEKRLEWVATISSSGSYTYYPDSVKGRFTISRDNAKN TLYLQMSSLKSEDTAMYYCTSPDYYGRSYGYFDYWG QGTTLTVSS |
| 280 | SB10 heavy chain variable region | QVTLKESGPGILQPSQTLSLTCSFSGFSLSTSGMGVSWF RQPSGKGLEWLAHIYWDDDKRYNPSLKSRLTISKDTSS NQVFLRITNVDTSDTATYYCTREGSADWEALDYWGQG TSVTVSS |
| 281 | SB11 heavy chain variable region | EVQLQQSGAELVKPGASVKLSCTASGFNIQDTYILWVK QRPEQGLEWIGGIDPANDNTNYGPNFQGKATITADTSS NTAYLQLSSLTSEDSAVYYCAREDAYPWFAYWGQGTLV TVSS |
| 282 | SB12 heavy chain variable region | QVQLQQSGAELMKPGASVKISCKATGYTFSRYWVEWV KQRPGHGLEWIGDILPGSDFTNYNEKFKGKATFTADSS SNTAYMQLSSLTSEDSAVYYCARKDYYGNYDYAMDY WGQGTSVTVSS |
| 283 | SB13 heavy chain variable region | EVKLVESGGGLVQPGGSLRLSCATSGFTFSDFYMEWVR QPPGKRLEWIAVSRDKTNDYTTEYSASMKGRFIVSRDT SQSILYLQMNALRAEDTAIYYCARDGVYYYGSSYTLD YWGQGTSVTVSS |
| 284 | SB14 heavy chain variable region | QVQLQQSGNEVVKPGASVKLSCKTSGYTFTNYWLQW VKQRPGQGLEWIGEIFPGTGTTYYNEKFKDQATVTIDT SSSTAYIHLSSLTSEDSAVYFCARTNWEGYYFDYWGQG TTLTVSS |
| 285 | SB15 heavy chain variable region | EVKLVESGGGLVKPGGSLKLSCAASGFTFSSYGMSWV RQTPEKRLEWVATISGGINYTYYRDSVEGRFTISRDNAK NNLYLQMSRLRSEDTALYYCARHDRYDGYYYVMDYW GQGTSVTVSS |
| 286 | SB16 heavy chain variable region | QVQLQQSGPELVRPGVSVRISCKGSGYPFTDYAIHWVK QGHGKNLEWIGVIAPYSDNTGYNQRFKGKATMTVDKS SSTAYMELARLTSEDSAIYYCAAYGNYGYYTMDYWGQ GTSVTVSS |
| 287 | SB17 heavy chain variable region | QVTLKESGPGILQPSQTLSLTCSFSGFSLSTSGMSVGWIR QPSGKGLEWLAHIWWNDDKYYNPALKTRLTISKDSSN NQIFLKIASVVTADSATYYCARIGYYYGTYALDYWGPG TSVTVSS |
| 288 | SB18 heavy chain variable region | QVTLKESGPGILQPSQTLSLTCSFSGFSLTTSGMNVGWL RQPSGKGLEWLAHIWWNDDKHSNPVLKSRLTISKDTS NNQAFLKIANVVTADSATYYCARLRDGGFAYWGQGTL VTVSA |
| 289 | 3B12H9 light chain variable region | EIVLTQSPLTLSVTIGQPASISCKSSQSLLDSDGKTYLNW LLQRPGQSPKRLIYLVSKLDSGVPDRFTGSGSGTDFTLK ISRVEAEDLGVYYCWQGTHFPWTFGGGTKLEIK |
| 290 | 3B12H5 light chain variable region | DIVMTQSPSSLSVSLGDRVTITCKASEDIYYRLAWYQQ KPGNAPRLLISGGTNLEAGIPSRFSGSGSGKDYTLSITSL QTEDVAIYYCQHYWNTPWTFGGGTKLEIK |
| 291 | 3C11 light chain variable region | DIQMTQSPSSFSVSLGDRVTITCRASEDIYYRLAWYQQK PGNAPRLLISGATALETGVPSRFSGSGSGKDYTLSITSLQ TEDVATYYCQHYWNIPWTFGGGTKLEIK |
| 292 | 5G9 light chain variable region | DIVMTQAPAIMSASPGEKVTISCSASSSVSYIYWYQQKP GSSPKPWIYRTSNLASGVPARFSGSGSGTSYSLTISSMEA EDAATYYCQQYHSYPYTFGGGTKLEIK |
| 293 | 4E12G10 light chain variable region | DIVMTQAAFSNPVTLGTSASISCRSSKSLLHSNGITYLY WYLQKPGQSPQLLIYQMSNLASGVPDRFSSSGSGTDFT LSISRVEAGDVGVYYCAQNLDLPYTFGGGTKLEIK |
| 294 | 6A7 light chain variable region | DIVMTQSPASLAVSLGQRATISCRASKSVSTSGYSYMH WYQQKPGQPPKLLLYLASNLESGVPARFSGSGSGADFT LNIHPVEEEDAATYYCLHSRELPYTFGGGTKLEIK |
| 295 | 6G9 light chain variable region | DIVMTQSPLSLPVSLGDQASISCRSSQSIVHSNGNTYLE WYLQKPGQSPKLLIYKVSNRFSGVPDRFSGSGSGTDFT LKISRVEAEDLGVYYCFQGSHVPWTFGGGTKLEIK |
| 296 | 3F1A11/3F1A11-H-12 light chain variable region | EIVLTQSPLSLPVSLGDQASISCRSRQTLVHTNGNTYLD WYLQKPGQSPKLLIYKVSIRFSGVPDRFSGSRSGTDFTL KISRVEAEDLGIYYCFQGSHVPWTFGGGTKLEIK |
| 297 | 20G4 light chain variable region | EIVITQSPASLAVSLGQRASISCRASESVDHNGISFMNWF QQKPGQPPKLLIYAASNQGSGVPARFSGSGSRTDFSLSI HPMEEDDTAIYFCQQNKEVPWTFGGGTKLEIK |
| 298 | 27D11 light chain variable region | DIVMTQSPASLAVSLGQRATISCRASESVDNSGISFMNW FQQKPGQPPKLLIYAASNQGSGVPARFSGSGSRTDFSLSI HPMEEDDTAMYFCQQNKEVPWTFGGGTKLEIK |
| 299 | 3A9 light chain variable region | DIQMTQSPLSLPVSLGDQASISCRSGQSIVHSNGKTYLE WYLQKPGQSPKLLIYKVSTRFSGVPDRFSGSGSGTDFT LKITRVEAEDLGVYYCFQGSHVPWTFGGGTKLEIK |
| 300 | 19F7D6 light chain variable region | DIVMTQSPLTLSITIGQPASISCRSSQSLLDIDGKTYLNW LLQRPGQSPKRLIYLVSELDSGVPDRFTGSGSGTDFTLK ISGVEAEDLGIYYCWQGTHFPYTFGGGTKLEIK |
| 301 | 3E7 light chain variable region | DIQMTQSPSSLAVSVGEKVSMSCKSSQSLLYSNNQRNY LAWYQQKPGQSPKLLIYWASTWESGVPDRFTGSGSGT AFTLTISSVKAEDLAVYYCQQYYSYPWTFGGGTKLEIK |
| 302 | 5B8 light chain variable region | EIVLTQSPASLAVSLGQRATISCRASESVDNYGISFMSWF QQKPGQPPKLLIYAASNQGSGVPDRFSGSGSGTDFSLNI HPMEEDDTAMYFCQQSKEVPRTFGGGTKLEIK |
| 303 | 4B7 light chain variable region | DVVMTQTPASLAVSLGQRATISCRASESVDNYGISFMN WFQQKPGQPPKLLIYAASNQGSGVPARFSGSGSGTDFS LNIHPMEEDDTAMYFCQQSKEVPRTFGGGTKLEIK |
| 304 | 8C9 light chain variable region | DIQMTQSPASLAVSLGQRATISCRASESVDNYGISFMNW FQQKPGQPPKLLIYAASNQGSGVPDRFSGSGSGTDFSLN IHPMEEDDTAMYFCQQSKEVPRTFGGGTKLEIK |
| 305 | 12E6 light chain variable region | DIVMTQAAPSVPVTPGGSVSISCRSTKSLLHSDGKTYLY WFLQRPGQSPQLLIYRMSNLASGVPDRFSGSGSGTAFT LRISRVEAEDVGVYYCMQHLEYPFTFGSGTKLEIK |
| 306 | G10F6 light chain variable region | DIQMTQSPLSLPVSLGDQASISCRSSQNIVHTNGNTYLE WYLQKVGQSPKLLIYKVSNRFSGVPDRFSGSGSGTDFT LKISRVEAEDLGVYYCFQGSHVPYTFGGGTKLEIK |
| 307 | 14A10C5 light chain variable region | DIVMTQAALSLPVSLGDQASISCRSSQNIVYTNGITYLE WYLQKPGQSPKLLIYKVSNRFSGVPDRFSGSGSATDFT LKISRVEAEDLGVYYCFQSSHVPWTFGGGTKLEIK |
| 308 | 8E5 light chain variable region | EIVLTQSPATLSVTPGDRVSLSCRASQSISDFLHWYQQRS HESPRLLIKYASQSISGIPSRFGGSGSGSDFTLNINTVEPE DVGMYYCQSAHSFPYTFGGGTKLEIK |
| 309 | 27A3 light chain variable region | DIVMTQTPLTLSVTIGQPASISCKSSQSLLDSDGKTYLN WLLQRPGQSPKRLIYLVSKLDSGVPDRFTGSGSGTDFT LKISRVEAEDLGVYYCWQGTHFPHTFGGGTKLEIK |
| 310 | 10D2 light chain variable region | DIVMTQSHKFMSTSVGDRVSITCKASQDVSTAVAWYRQ KPGQSPKLLIYWASTRHTGVPDRLTGSGSGTDFALTINS VQAEDLALYYCQQHSSTPYTFGGGTKLEIK |
| 311 | 24G4 light chain variable region | DIQMTQSPAILSASPGEKVTMTCRASSSVSYIHWYQQK PGSSPDPWIYATSFLASGVPGRFSGSGSGTSYSLTISRLE AEDAATYYCQQWNSSPFTFGGGTKLEIK |
| 312 | 1CP-061 light chain variable region | DVVMTQTPASLAVSLGQRATISCRASESVDYSGYSFMH WYQQKPGQPPKLLIYRASNLESGIPARFSGSGSRTDFTL TINPVEADDVATYYCQQSNEDPYTFGGGTKLEIK |
| 313 | 1CP-063 light chain variable region | DIVMTQSPSSMYASLGERVTITCKASQDIKSYLSWYQQ KPWKSPKTLIYYATSLADGVPSRFSGSGSGQDYSLTISSL ESDDTATYYCLQYGESPWTFGGGTKLEIK |
| 314 | 1CP-062 light chain variable region | DIVMTQTPASLAVSLGQRATISCRASESVDSYGNSFMH WYQQKPGQPPKLLIYRASNLESGIPARFSGSGSRTDFTL TINPVEADDVATYYCQQSNEDPYTFGGGTKLEIK |
| 315 | 1CP-067 light chain variable region | DIVMTQAAFSNPVTLGTSASISCRSTKSLLHSNGITYLY WFLQKPGQSPQLLIYQMSNLASGVPDRFSSSGSGTDFT LRISRVEAEDVGVYYCAQNLELPFTFGGGTKLEIK |
| 316 | 1CP-069 light chain variable region | DIVMTQAAFSNPVTLGTSASISCRSTKSLLHSNGITYLY WYLQKPGQSPQLLIYQMSNLASGVPDRFSSSGSGTDFT LRISRVEAEDVGVYYCAQNLELPFTFGGGTKLEIK |
| 317 | 1CP-064 light chain variable region | DVVMTQTPASLAVSLGQRATISCRASESVDSYGNSFMH WYQQKPGQPPKLLIYLASNLESGVPARFSGSGSRTDFTL TIDPVEADDAATYYCQQNNEDPWTFGGGTKLEIK |
| 318 | 1CP-066 light chain variable region | DIVMTQAAASLSVTVGETVTITCRASENIYNNLAWYQQ QQGKSPQLLVYAATNLADGVPSRFSGSGSGTHYSLKIN SLQSEDFGFYYCQHFWGTPPTFGGGTKLEIK |
| 319 | 1CP-065 light chain variable region | DIVMTQSPKFMSTSVGDRVSITCKASQDVSTAVTWYQQ KPGQSPKLLIYWASTRHTGVPDRFTGSGSGTDYTLTISS VQAEDLALYYCQQHYSTPYTFGGGTKLEIK |
| 320 | 1CP-068 light chain variable region | DVVMTQTPALMSASPGEKVTMTCSASSSVSYMYWYQ QKPRSSPKPWIYLTSNLASGVPARFSGSGSGTSYSLTISS MEAEDAATYYCQQWSSDPFTFGGGTKLEIK |
| 321 | SB1 light chain variable region | DIRMTQSPSSMYASLGERVTITCKASQDINRFLSWFLQR PGKAPKTLIYRANNLVEGVPSRFSGSGSGQDYSLIISNL EYEDMGIYYCLQYDEFPRTFGGGTKLEIK |
| 322 | SB2 light chain variable region | DVVMTQTPLTLSITIGQPASISCRSSQSLLDIDGKTYLNW LLQRPGQSPKRLIYLVSELDSGVPDRFTGSGSGTDFTLRI SGVEAEDLGIYYCWQGTHFPYTFGGGTKLEIK |
| 323 | SB3 light chain variable region | DVVMTQTPLTLSITIGQPASISCRSSQSLLDFDGKTYLN WLLQRPGQSPKRLIYLVSELDSGVPDRFTGSGSGTDFTL KISGVEAEDLGIYYCWQGTHFPFTFGGGTKLEIK |
| 324 | SB4 light chain variable region | DIVMTQAAFSNPVTLGTSASISCRSSRSLLHNNGITYLY WYLQKPGQSPQLLIYQMSNLASGVPDRFHSSGSGTDFT LSISRVEAEDVGVYYCAQNLDLPYTFGGGTKLEIK |
| 325 | SB5 light chain variable region | QIVLTQSPAIKSASPGEKVTMTCSASSSVIYMHWYQQK SGSSPKRWIYDTSKLASGVPVRFSGSGSGTSYSLTISTM EAEDAATYYCQQWSSYPPTFGGGTKVEIK |
| 326 | SB6 light chain variable region | SIVMTQTPKFLLVSAGDRVTITCKASQSVNTDVAWYQQ KSGQSPKLLIYSASNRYTGVPDRFTGSGYGTDFTFTIST VQAEDLAVYFCQQDHSSPPWTFGGGTKLEIK |
| 327 | SB7 light chain variable region | DIKMTQSPSSIYVSPGERVTITCKASQDINSFLSWFQQKP GKSPKTLIYRANRLVDGVPSRFSGSGSGQDYSLTISSLE YEDMGIYYCLQYDEFPRTFGGGTKLEIK |
| 328 | SB8 light chain variable region | QAVVTQESALTTSPGETVTLTCRSSTGAVATSNYANWV QEKPDHLFTGLIGGTNNRVPGVPARFSGSLIGDKAALTI TGAQTEDEAIYFCALWYSNHWVFGGGTKLTVL |
| 329 | SB9 light chain variable region | DVVMTQTPLTLSITIGQPASISCKSSQSLLDSDGKTYLT WLLQRPGQSPKRLIFLVSKLDSGVPDRFTGSGSGTDFTL KISRVEAEDLGVYYCWQGTHFPYTLGGGTKLEIK |
| 330 | SB10 light chain variable region | DVLMTQTPLSLSVSLGDQASISCRSSQTIVYSNGNSYLE WYLQKPGQSPKLLIYKVSNRFSGIPDRFSGSGSGTDFTL KISRVEAEDLGIYYCFQSSHVPWTFGGGTKLEIK |
| 331 | SB11 light chain variable region | DIVLTQSPASLAVSLGQRATISCRASQSVSTSSYSYMHW YQLKPGQPPKLLIKYASNLESGVPARFSGSGSGTDFTLN IHPVEEEDAAIYYCQHNWEIPWTFGGGTRLEIK |
| 332 | SB12 light chain variable region | DVQITQSPSYLAASPGEAITINCRTSKSIGKYLAWYQEK PGKTNKLLIYSGSTLQSGIPSRFSGSGSGTDFTLTITSLEP EDFAMYYCQQHDEYPWTFGGGTKLDIK |
| 333 | SB13 light chain variable region | DIVMTQSHKFMSTSVGDRVSITCKASQDVSSSVAWYQQ KPGQSPKLLIYWASTRHTGVPDRFTGSGSGTDYTLTISS VQAEDLALYYCQQHYNTPPTFGGGTKLEIK |
| 334 | SB14 light chain variable region | ENVLTQSPAIMSASSGEKVTMTCRASSSVRSSYLHWYQ QKSGASPKVWIYSTSNLASGVPVRFSGSGSGTSYSLTIS SVEAEDAATYYCQQYSGSPSWTFGGGTKLEIK |
| 335 | SB15 light chain variable region | DIQMTQSPSSLSASLGERVSLTCRASQEISGYLTWLQQR PDGTIKRLIYAASTLDSGVPKRFSGSRSGSDYSLTISSLES EDFADYYCLQYASYPYTFGGGTKLEIK |
| 336 | SB16 light chain variable region | DIQMTQSTSSLSASLGDRVTITCRASQDITNYLNWYQQ KPDGTVKLLIYYTSRLHSGVPSRFSGSGSGTDFSLTISNL EQEDIATYFCQQGNTLPWTFGGGTKLEII |
| 337 | SB17 light chain variable region | QIVLTQSPALMTASPGEQVTMTCSASSSVSYIHWYQQK SGTSPKRWIYDASKLASGVPARFSGSGSGTSYSLTISSM EAEDAATYYCQQWSSYPLTFGGGTKLELK |
| 338 | SB18 light chain variable region | DIQMTQTTSSLSASLGDRVTISCSASQAISNYLNWYQQ KPDGTVKLLIYYTSHLHSGVPSRFSGSGSGTDYSLTISN LEPEDIATYYCQQYSKIPWTFGGGTKLEII |
| 339 | Humanized antibody 3C11 heavy chain variable region 01 | QVQLVQSGAEVKKPGASVKVSCKASGYNFISYWLHW VRQAPGQGLEWMGGIYPGNTDTTYNQKFKGRVTMTR DTSTSTVYMELSSLRSEDTAVYYCIRYDYDRGMDYWG QGTLVTVSS |
| 340 | Humanized antibody 3C11 heavy chain variable region 02 | QVQLVQSGAEVKKPGASVKVSCKASGYNFISYWLHW VRQAPGQGLEWMGGIYPGNTDTTYNQKFKGRVTMTA DTSTSTVYMELSSLRSEDTAVYYCIRYDYDRGMDYWG QGTLVTVSS |
| 341 | Humanized antibody 3C11 heavy chain variable region 03 | QVQLVQSGAEVKKPGASVKVSCKASGYNFISYWLHW VRQAPGQGLEWMGGIYPGNTDTTYNQKFKGRVTMTA DTSTSTAYMELSSLRSEDTAVYYCIRYDYDRGMDYWG QGTLVTVSS |
| 342 | Humanized antibody 3C11 heavy chain variable region 04 | QVQLVQSGAEVKKPGASVKVSCKASGYNFISYWLHW VRQAPGQGLEWMGGIYPGNTDTTYNQKFKGRVTVTA VTSTSTVYMELSSLRSEDTAVYYCIRYDYDRGMDYWG QGTLVTVSS |
| 343 | Humanized antibody 8C9 heavy chain variable region 01 | EVQLVESGGGLVQPGRSLRLSCTASGLTFTHYHMSWFR QAPGKGLEWVAIIRNKAYGYTTDYSASVRGRFTISRDD SKSILYLQMNSLKTEDTAVYYCVREGGYGYDGTFAYW GQGTLVTVSS |
| 344 | Humanized antibody 8C9 heavy chain variable region 02 | EVQLVESGGGLVQPGRSLRLSCTTSGLTFTHYHMSWVR QAPGKGLEWVAIIRNKAYGYTTDYSASVRGRFTISRDD SKSILYLQMNSLKTEDTAVYYCVREGGYGYDGTFAYW GQGTLVTVSS |
| 345 | Humanized antibody 8C9 heavy chain variable region 03 | EVQLVESGGGLVQPGRSLRLSCTTSGLTFTHYHMSWVR QAPGKGPEWVAIIRNKAYGYTTDYSASVRGRFTISRDD SKSILYLQMNSLKTEDTAVYYCVREGGYGYDGTFAYW GQGTLVTVSS |
| 346 | Humanized antibody 8C9 heavy chain variable region 04 | EVQLVESGGGLVQPGRSLRLSCTTSGLTFTHYHMSWVR QPPGKAPEWVAIIRNKAYGYTTDYSASVRGRFTISRDDS KSILYLQMNSLKTEDTAVYYCVREGGYGYDGTFAYWG QGTLVTVSS |
| 347 | Humanized antibody 8E5 heavy chain variable region 01 | QVQLVQSGSELKKPGASVKVSCKASGYTLTDFSMHWV RQAPGQGLEWMGWINTETGAPTYADDFKGRFVFSLDT SVSTAYLQISSLKAEDTAVYYCSRGRDYDPHFDYWGQG TLVTVSS |
| 348 | Humanized antibody 8E5 heavy chain variable region 02 | QVQLVQSGSELKKPGASVKVSCKASGYTLTDFSMHWV RQAPGQGLEWMGWINTETGAPTYADDFKGRFVLSLDT SVSTAYLQISSLKAEDTAVYYCSRGRDYDPHFDYWGQG TLVTVSS |
| 349 | Humanized antibody 8E5 heavy chain variable region 03 | QVQLVQSGSELKKPGESVKVSCKASGYTLTDFSMHWV RQAPGQGLEWMGWINTETGAPTYADDFKGRFVLSLDT SVSTAYLQISSLKSEDTAVYYCSRGRDYDPHFDYWGQG TLVTVSS |
| 350 | Humanized antibody 8E5 heavy chain variable region 04 | QVQLQQSGSELKKPGESVKLSCKASGYTLTDFSMHWV RQAPGQGLKWMGWINTETGAPTYADDFKGRFVFSLDT SVSMAYLQISSLKSEDTAVYYCSRGRDYDPHFDYWGQ GTLVTVSS |
| 351 | Humanized antibody 3C11 light chain variable region 01 | DIQMTQSPSSLSASVGDRVTITCRASEDIYYRLAWYQQ KPGKAPKLLISGATALETGVPSRFSGSGSGKDYTFTISSL QPEDIATYYCQHYWNIPWTFGQGTKVEIK |
| 352 | Humanized antibody 3C11 light chain variable region 02 | DIQMTQSPSSLSASVGDRVTITCRASEDIYYRLAWYQQ KPGKAPKLLISGATALETGVPSRFSGSGSGKDYTLTISSL QPEDIATYYCQHYWNIPWTFGQGTKVEIK |
| 353 | Humanized antibody 3C11 light chain variable region 03 | DIQMTQSPSSLSASVGDRVTITCRASEDIYYRLAWYQQ KPGNAPKLLISGATALETGVPSRFSGSGSGKDYTLTISSL QPEDIATYYCQHYWNIPWTFGQGTKVEIK |
| 354 | Humanized antibody 8C9 light chain variable region 01 | DIQMTQSPSSLSASVGDRVTITCRASESVDNYGISFMN WFQQKPGQAPKLLIYAASNQGSGVPSRFSGSGSGTDFT LTISSMQPEDFATYYCQQSKEVPRTFGGGTKVEIK |
| 355 | Humanized antibody 8C9 light chain variable region 02 | DIQMTQSPSSLSASVGDRVTITCRASESVDNYGISFMN WFQQKPGQPPKLLIYAASNQGSGVPSRFSGSGSGTDFT LTISSMQPEDFATYYCQQSKEVPRTFGGGTKVEIK |
| 356 | Humanized antibody 8C9 light chain variable region 03 | DIQMTQSPSSLSASVGDRVTITCRASESVDNYGISFMN WFQQKPGQPPKLLIYAASNQGSGVPSRFSGSGSGTDFT LTISSMQPEDFATYFCQQSKEVPRTFGGGTKVEIK |
| 357 | Humanized antibody 8E5 light chain variable region 01 | EIVLTQSPATLSLSPGERATLSCRASQSISDFLHWYQQKP GQAPRLLIKYASQSISGIPARFSGSGPGSDFTLTISSVEPE DFAVYYCQSAHSFPYTFGQGTKLEIK |
| 358 | Humanized antibody 8E5 light chain variable region 02 | EIVLTQSPATLSLSPGERATLSCRASQSISDFLHWYQQKP GQSPRLLIKYASQSISGIPARFSGSGSGSDFTLTISSVEPE DFAVYYCQSAHSFPYTFGQGTKLEIK |
| 359 | Humanized antibody 8E5 light Chain Variable Region 03 | EIVLTQSPATLSLSPGERATLSCRASQSISDFLHWYQQKS GQSPRLLIKYASQSISGIPARFSGSGSGSDFTLTISSVEPE DFAVYYCQSAHSFPYTFGQGTKLEIK |
| 360 | Human IgG1 heavy chain constant region | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKVEPKSCDKTHTCPPCPAPE LLGGPSVFLFPPKPKDTLMISRTPEVTCVVVDVSHEDPE VKFNWYVDGVEVHNAKTKPREEQYNSTYRVVSVLTV LHQDWLNGKEYKCKVSNKALPAPIEKTISKAKGQPREP QVYTLPPSREEMTKNQVSLTCLVKGFYPSDIAVEWESN GQPENNYKTTPPVLDSDGSFFLYSKLTVDKSRWQQGN VFSCSVMHEALHNHYTQKSLSLSPGK |
| 361 | Human light chain constant region (Kappa chain constant region) | |
| 362 | Kabat 3C11 CDR-H1 | SYWLH |
| 363 | Kabat 3C11 CDR-H2 | GIYPGNTDTTYNQKFKG |
| 364 | Kabat/AbM/Chothia 3C11 CDR-H3 | YDYDRGMDY |
| 365 | Kabat/AbM/Chothia 3C11 CDR-L1 | RASEDIYYRLA |
| 366 | Kabat/AbM/Chothia 3C11 CDR-L2 | GATALET |
| 367 | AbM 3C11 CDR-H1 | GYNFISYWLH |
| 368 | AbM 3C11 CDR-H2 | GIYPGNTDTT |
| 369 | Chothia 3C11 CDR-H1 | GYNFISY |
| 370 | Chothia 3C11 CDR-H2 | YPGNTD |
| 371 | Contact 3C11 CDR-H1 | ISYWLH |
| 372 | Contact 3C11 CDR-H2 | WIGGIYPGNTDTT |
| 373 | Contact 3C11 CDR-H3 | IRYDYDRGMD |
| 374 | Contact 3C11 CDR-L1 | YYRLAWY |
| 375 | Contact 3C11 CDR-L2 | LLISGATALE |
| 376 | Contact 3C11 CDR-L3 | QHYWNIPW |
| 377 | Kabat 8E5 CDR-H1 | DFSMH |
| 378 | Kabat 8E5 CDR-H2 | WINTETGAPTYADDFKG |
| 379 | Kabat/AbM/Chothia 8E5 CDR-H3 | GRDYDPHFDY |
| 380 | Kabat/AbM/Chothia 8E5 CDR-L1 | RASQSISDFLH |
| 381 | Kabat/AbM/Chothia 8E5 CDR-L2 | YASQSIS |
| 382 | AbM 8E5 CDR-H1 | GYTLTDFSMH |
| 383 | AbM 8E5 CDR-H2 | WINTETGAPT |
| 384 | Chothia 8E5 CDR-H1 | GYTLTDF |
| 385 | Chothia 8E5 CDR-H2 | NTETGA |
| 386 | Contact 8E5 CDR-H1 | TDFSMH |
| 387 | Contact 8E5 CDR-H2 | WMGWINTETGAPT |
| 388 | Contact 8E5 CDR-H3 | SRGRDYDPHFD |
| 389 | Contact 8E5 CDR-L1 | SDFLHWY |
| 390 | Contact 8E5 CDR-L2 | LLIKYASQSI |
| 391 | Contact 8E5 CDR-L3 | QSAHSFPY |
| 392 | Kabat 8C9 CDR-H1 | HYHMS |
| 393 | Kabat 8C9 CDR-H2 | IIRNKAYGYTTDYSASVRG |
| 394 | Kabat/AbM/Chothia 8C9 CDR-H3 | EGGYGYDGTFAY |
| 395 | Kabat/AbM/Chothia 8C9 CDR-L1 | RASESVDNYGISFMN |
| 396 | Kabat/AbM/Chothia 8C9 CDR-L2 | AASNQGS |
| 397 | AbM 8C9 CDR-H1 | GLTFTHYHMS |
| 398 | AbM 8C9 CDR-H2 | IIRNKAYGYTTD |
| 399 | Chothia 8C9 CDR-H1 | GLTFTHY |
| 400 | Chothia 8C9 CDR-H2 | RNKAYGYT |
| 401 | Contact 8C9 CDR-H1 | THYHMS |
| 402 | Contact 8C9 CDR-H2 | WLAIIRNKAYGYTTD |
| 403 | Contact 8C9 CDR-H3 | VREGGYGYDGTFA |
| 404 | Contact 8C9 CDR-L1 | DNYGISFMNWF |
| 405 | Contact 8C9 CDR-L2 | LLIYAASNQG |
| 406 | Contact 8C9 CDR-L3 | QQSKEVPR |

Unless otherwise specified, the molecular biology experimental methods and immunoassays used in the present disclosure refer essentially to the methods described in J. Sambrook et al. Molecular cloning: Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995. Restriction enzymes are used in accordance with the conditions recommended by the manufacturer of the product. Those skilled in the art are aware that examples describe the present disclosure by way of example and are not intended to limit the scope of protection claimed by the present disclosure.

### Example 1: Preparation of full-length antigens for human, Macaca fascicularis, rat and mouse CDCP1-C extracellular domains and antigens for human CDCP1-A and CDCP1-B.

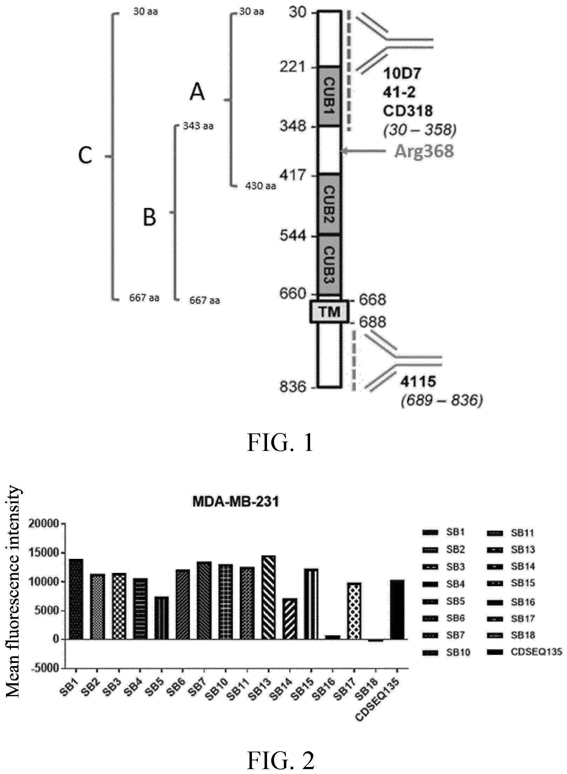

CDCP1-C of this example refers to the full-length extracellular domain(taking human CDCP1 as an example, the amino acid sequence of positions 30 to 667 from N-terminus to C-terminus); CDCP1-A refers to the membrane-distal region of CDCP1, that is, the N-terminus (taking human CDCP1 as an example, the amino acid sequence of positions 30 to 430 from N-terminus to C-terminus). CDCP1-B refers to the membrane-proximal region of CDCP1, that is, the C-terminus (taking human CDCP1 as an example, the amino acid sequence of positions 343 to 667 from N-terminus to C-terminus), and the specific structure is shown in FIG. 1.

CDCP1 proteins of human, Macaca fascicularis, rat and mouse were expressed *in vitro.* The gene number of human CDCP1 protein coding sequence was NP_073753.3, the gene number of Macaca fascicularis CDCP1 protein coding sequence was XP_001114659.2, the gene number of rat CDCP1 protein coding sequence was NP_001100339, and the gene number of mouse CDCP1 protein coding sequence was AAH85253. By means of molecular cloning, the corresponding full-length extracellular domain, membrane-distal region or membrane-proximal region fragments were cloned into an expression vector pTT5, transfected and expressed in HEK-293T cells, and then purified with Fc/his tag. The purity and molecular weight were determined by SEC or SDS PAGE. The purity was greater than 95%, and the endotoxin content was less than 1 EU/mg. HuCDCP1-Fc, HuCDCP1-A, HuCDCP1-B, HuCDCP1-C, CynoCDCP1, RatCDCP1 and MsCDCP1 were prepared for later animal immunization or screening experiments.

### Example 2: Preparation of anti-CDCP1 murine monoclonal antibody

BALB/c mice were immunized with the human CDCP1-Fc (HuCDCP1-Fc) expressed in Example 1, once every two weeks, and mouse serum was collected each time to determine the titer of anti-CDCP1 antibody in the serum. After the fourth immunization, mouse splenocytes and SP2/0 cells were taken for hybridoma fusion, the hybridoma cells were plated into a 96-well plate, supernatants were collected after 7 to 10 days, and ELISA was carried out by coating with human CDCP1-his (Kactus Biosystems, CDC-HM101). The supernatants of the clones with OD > 0.5 were selected for incubation with MDA-MB-231 cells (breast cancer cells with high expression of CDCP1, Nanjing Cobioer, CBP60382) at 4 °C for 30 mins, followed by incubation with anti-mFc secondary antibody (PE Goat anti-mouse IgG (minimal x-reactivity) Antibody, Biolegend, 405307). Detection was performed using a flow cytometer. The cells without hybridoma supernatant and only with secondary antibody were used as control, and Geo-MFI (test sample)/Geo-MFI (control) was used as the detection index. 298 hybridoma monoclonal antibodies with a magnification greater than 100 times were selected. Then, the screened hybridoma monoclonal antibodies were subjected to an antibody internalization assay, hybridoma monoclonal antibodies with an internalization rate greater than 50% within 1 h were selected, and molecules were ranked by subcloning, affinity testing, sequencing and other works. Finally, 33 hybridoma monoclonal antibodies were selected as candidate antibodies, and they are numbered respectively: 3B12H9, 3B12H5, 3C11, 5G9, 4E12G10, 6A7, 6G9, 3F1A11, 20G4, 27D11, 3F1A11-H-12, 3A9, 19F7D6, 3E7, 5B8, 4B7, 8C9, 12E6, G10F6, 14A10C5, 8E5, 27A3, 10D2, 24G4, 1CP-061, 1CP-063, 1CP-062, 1CP-067, 1CP-069, 1CP-064, 1CP-066, 1CP-065, 1CP-068.

### Example 3: Detection of affinity of anti-CDCP1 murine monoclonal antibody

Antigens HuCDCP1-A, HuCDCP1-B, HuCDCP1-C, CynoCDCP1, MsCDCP1, and RatCDCP1 (2 µg/ml) prepared in Example 1 were respectively immobilized to assay the binding of the candidate antibodies obtained in Example 2 and the control antibody CDSEQ135 (which is from the sequence No. 135 in Patent WO 2011023389A1 and the SEQ135 and antibody 135 in the present disclosure, unless otherwise specified, are CDSEQ135), antibodies to be tested (antibodies were diluted to the highest concentration of 150 nM, with a 3-fold dilution across 5 concentration points, which were respectively 1.85, 5.55, 16.67, 50, and 150 nM) were allowed to flow through the detection chip respectively by Caterra or Biacore, and data analysis was performed with the corresponding software.

The detection results are shown in Table 2. 13 anti-CDCP1 murine monoclonal antibodies had good affinities with HuCDCP1-A, HuCDCP1-B, HuCDCP1-C, CynoCDCP1, MsCDCP 1 or RatCDCP1.

**Table 2 Detection of affinity of anti-CDCP1 murine monoclonal antibody**

| Antibody No. | HuCDCP1-A EC₅₀ (M) | HuCDCP1-B EC₅₀ (M) | HuCDCP1-C EC₅₀ (M) | CynoCDCP1 EC₅₀ (M) | MsCDCP1 EC₅₀ (M) | RatCDCP1 EC₅₀ (M) |
|---|---|---|---|---|---|---|
| 12E6 | 1.67E-09 | 1.62E-09 | 6.34E-11 | 1.45E-09 | 4.01E-06 | / |
| 10D2 | 7.47E-10 | / | 7.05E-10 | 1.61E-08 | / | 4.11E-08 |
| 8E5 | 7.93E-08 | / | 1.05E-10 | 5.16E-11 | / | 5.05E-08 |
| G10F6 | / | / | 5.63E-09 | 4.47E-09 | / | / |
| 3C11 | / | 3.78E-09 | 3.96E-09 | 2.36E-08 | / | / |
| 6A7 | / | / | 9.65E-09 | 9.96E-09 | 2.75E-08 | 9.14E-08 |
| 3B12H5 | 5.23E-08 | / | 7.23E-10 | 4.50E-09 | / | / |
| 5B8 | / | / | 1.19E-09 | 1.36E-10 | / | 1.80E-08 |
| 8C9 | / | / | 1.72E-09 | 5.64E-10 | / | 3.05E-08 |
| 3F1A11 | 1.27E-07 | / | 3.79E-08 | 1.15E-07 | / | 1.70E-08 |
| 14A10C5 | / | / | 2.02E-09 | 9.83E-08 | / | / |
| CDSEQ1 35 (positive control) | / | / | 1.54E-08 | 2.00E-08 | / | 1.39E-08 |

| | | | | | | |
|---|---|---|---|---|---|---|
| Note: "/" in table means not detected | | | | | | |

### Example 4: Detection of binding capacity of anti-CDCP1 murine monoclonal antibody to tumor cells

Flow cytometry was used to detect the binding capacity of antibody to tumor cells. MDA-MB-231 (a breast cancer cell with high expression of CDCP1, Nanjing Cobioer, CBP60382), PC3 (a prostate cancer cell with high expression of CDCP1, Nanjing Cobioer, CBP60343), MCF7 (a breast cancer cell that does not express CDCP1, Nanjing Cobioer, CBP60380), A2780 (a human ovarian cancer cell that does not express CDCP1, Nanjing Cobioer, CBP60283) were subjected to digestion with 5 mM EDTA, and centrifuged. 2.0 × 10⁴ tumor cells were added per well, respectively, and 50 µL (PBS + 2% FBS) were added, and after blocking at room temperature with an Fc blocker (Human TruStain FcX ^{™}, Biolegend, 422302) for 10 mins, an anti-CDCP1 murine monoclonal antibody at a final concentration of 200 nM was added. The obtained mixture was subjected to a 3-fold serial dilution across 8 concentration points, and incubated at 4 °C for 30 mins, and then incubated with a secondary antibody (PE anti-human IgG Fc Antibody, Biolegend, 410708), detection was performed using a flow cytometer.

The detection results are shown in Table 3. 11 anti-CDCP1 murine monoclonal antibodies showed good affinity with MDA-MB-231 and PC3 tumor cells with high expression of CDCP1, and do not bind to MCF7 and A2780 tumor cells that do not express CDCP1.

**Table 3 Detection of affinity of anti-CDCP1 murine monoclonal antibody**

| Antibody number | MDA-MB-231 EC₅₀ (nM) | PC3 EC₅₀ (nM) | MCF7 EC₅₀ (nM) | A2780 EC₅₀ (nM) |
|---|---|---|---|---|
| 12E6 | 85.45 | 10.09 | / | / |
| 10D2 | 0.49 | 2.54 | / | / |
| 8E5 | 0.67 | 0.63 | / | / |
| G10F6 | 0.19 | 0.23 | / | / |
| 3C11 | 0.27 | 0.23 | / | / |
| 6A7 | 1.72 | 1.35 | / | / |
| 3B12H5 | 0.47 | 0.55 | / | / |
| 5B8 | 0.93 | 0.91 | / | / |
| 8C9 | 0.47 | 0.40 | / | / |
| 3F1A11 | / | 0.41 | / | / |
| 14A10C5 | / | 0.10 | / | / |
| CDSEQ135 (positive control) | 0.03 | 0.26 | / | / |

| | | | | |
|---|---|---|---|---|
| Note: "/" in table means not detected | | | | |

### Example 5: Detection of endocytosis capacity of anti-CDCP1 murine monoclonal antibody

The endocytosis capacity of the antibody was detected for 0.5 to 2 hours. MDA-MB-231 and PC3 cells were subjected to digestion with 5 mM EDTA, and plated at 4 × 10⁴ cells/well after cell counting. After adding an Fc blocker (Human TruStain FcX ^{™}, Biolegend, 422302) and blocking at room temperature for 10 mins, a test antibody (1.5 µg/mL) was added, and the mixture was incubated at 4 °C for 30 mins, centrifuged, and washed twice with PBS + 2% FBS, then the cells were divided into four equal portions (labeled as 0 h, 0.5 h, 1 h, 2 h), cells labeled as 0.5 h, 1 h, 2 h time points were added to a cell culture medium at 200 µL/well, and cultured at 37 °C for 0.5 h, 1 h, 2 h, respectively. Cells at 0 h point were centrifuged to remove supernatant, a secondary antibody (PE anti-human IgG Fc Antibody, Biolegend, 410708) was added, and the mixture was incubated at 4 °C for 30 mins, centrifuged, and washed twice with PBS + 2% FBS, and detection was performed using a flow cytometer. After incubation of cells labeled as 0.5 h, 1 h, and 2 h points at 37 °C was completed, a secondary antibody was added (in the same steps as those at 0 h point), Geo-MFI of the signal under the same voltage was collected as detection signal at 0 h, and antibody internalization rate was calculated according to the following equation: Internalization (%)= (Geo-MFI (0 h)- Geo-MFI (0.5, 1, 2 h))/ Geo-MFI (0 h) × 100%.

The detection results are shown in Table 4. The murine monoclonal antibodies in the table all showed good endocytic effects when using MDA-MB-231 and PC3.

**Table 4 Detection of endocytosis capacity of anti-CDCP1 murine monoclonal antibody**

| Antibody number | MDA-MB-231 (%) | | | PC3 (%) | | |
|---|---|---|---|---|---|---|
| | 0.5 h | 1 h | 2 h | 0.5 h | 1 h | 2 h |
| 27A3 | 93.40 | 90.57 | 98.74 | 90.84 | 94.04 | 96.12 |
| 12E6 | 10.62 | 7.08 | 27.65 | 0.00 | 0.00 | 7.99 |
| 10D2 | 46.38 | 57.85 | 70.77 | 21.48 | 41.99 | 64.19 |
| 20G4 | 84.77 | 91.47 | 95.13 | 78.02 | 87.36 | 94.46 |
| 24G4 | 55.98 | 70.68 | 83.23 | 46.97 | 67.07 | 82.61 |
| 8E5 | 24.32 | 38.20 | 53.26 | 6.54 | 17.17 | 42.94 |
| 27D11 | 76.41 | 82.73 | 89.16 | 76.08 | 83.52 | 88.25 |
| G10F6 | 34.49 | 50.70 | 67.02 | 3.36 | 20.68 | 54.36 |
| 6G9 | 75.76 | 86.81 | 88.89 | 60.58 | 77.88 | 87.67 |
| 3C11 | 28.50 | 36.16 | 52.23 | 28.45 | 39.70 | 59.90 |
| 5G9 | 0.00 | 0.00 | 0.00 | 0.00 | 0.00 | 0.00 |
| 6A7 | 28.97 | 43.77 | 58.78 | 22.99 | 35.14 | 49.86 |
| 3B12H9 | 0.00 | 0.00 | 0.00 | 0.00 | 0.00 | 0.00 |
| 3B12H5 | 20.76 | 29.93 | 49.69 | 16.54 | 30.28 | 50.50 |
| 5B8 | 24.06 | 32.99 | 50.21 | 28.18 | 40.11 | 54.90 |
| 4B7 | 18.01 | 27.41 | 48.49 | 14.05 | 31.25 | 49.70 |
| 8C9 | 26.56 | 37.08 | 51.59 | 24.63 | 39.13 | 56.20 |
| 3F1A11 | / | / | 38.10 | / | / | 51.76 |
| 3F1A11-12 | / | / | 37.57 | / | / | 33.83 |
| 19F7D6 | / | / | 63.32 | / | / | 64.51 |
| 14A10C5 | / | / | 39.23 | / | / | 51.73 |
| 1CP-061 | / | / | 42.68 | / | / | 28.00 |
| 1CP-063 | / | / | 49.71 | / | / | 62.68 |
| 1CP-062 | / | / | 59.61 | / | / | 66.74 |
| 1CP-067 | / | / | 69.09 | / | / | 72.66 |
| 1CP-069 | / | / | 73.58 | / | / | 73.92 |
| 1CP-064 | / | / | 93.43 | / | / | 93.05 |
| 1CP-066 | / | / | 55.57 | / | / | 33.21 |
| 1CP-065 | / | / | 52.89 | / | / | 68.71 |
| 1CP-068 | / | / | 49.47 | / | / | 66.24 |
| CDSEQ135 | 25.40 | 34.35 | 46.38 | 14.73 | 29.75 | 45.11 |

### Example 6: Preparation of anti-CDCP1 antibody by single B cell technique

In this study, BALBc mice were immunized with the antigen human CDCP1-Fc expressed in Example 1, once every two weeks, mouse serum was collected each time to determine the titer of anti-CDCP1-his antibody in the serum; after the fourth immunization, mouse spleen was harvested and processed with a commercially available kit (EasySep^{™} Mouse Pan-B Cell Isolation Kit, Stem Cell, 19844), cells was counted, and subjected to *in vitro* B220 (a B cell label, PE anti-mouse/human CD45R/B220 Antibody, BioLegend, 103207) and CDCP1 fluorescent labeling, and B220+CDCP1+ cells were sorted by flow cytometry technology, and then labeled with 10 x Genomics kit (Chromium Next GEM Single Cell 5 'Kit v2, 16 rxns, 10X GENOMICS, PN-1000263), single cells were subjected to BCR sequencing, *in vitro* data analysis was performed, VH and VL sequences were classified, family assignment was performed, and representative sequences of each family were selected for synthesis. Finally, 18 candidate antibodies were selected, and their numbers are SB1, SB2, SB3, SB4, SB5, SB6, SB7, SB8, SB9, SB10, SB11, SB12, SB13, SB14, SB15, SB16, SB17, SB18, respectively.

### Example 7: Detection of affinity of anti-CDCP1 antibody by single B cell technique

The candidate antibodies obtained from Example 6 were subjected to gene cloning, constructed into an expression vector for a small amount of expression in CHO cells, and purified with an Fc affinity column (AT Protein A Diamond Plus. No. AA402312). Antibody purity was analyzed by HPLC and determined by Nanodrop. High-throughput affinity assay was carried out for a small amount of antibodies by Caterra. Specifically, human CDCP1 antigen (2 µg/ml) was immobilized to assay antibody binding, and antibodies to be tested (antibodies diluted to the highest concentration of 150 nM, with a 3-fold dilution across 5 concentration points, which were respectively 1.85, 5.55, 16.67, 50, 150 nM) were allowed to flow through the detection chip respectively by Caterra, and data analysis was performed with Kinetics software. At the same time, 2 µg/ml antigen human CDCP1-C and 2 µg/ml human CDCP1-B were used for coating, and the antigen-antibody binding capacity was tested by ELISA for verification.

The detection results are shown in Table 5, and the anti-CDCP1 murine monoclonal antibodies in the table showed good affinity with HuCDCP1-C.

**Table 5 Detection of affinity of anti-CDCP1 antibody by single B cell technique**

| Antibody number | ELISA (nM) | | Affinity (Caterra, M) |
|---|---|---|---|
| | HuCDCP1-B | HuCDCP1-C | KD (M)-huCDCP1-his |
| SB1 | / | 0.092 | 1.61E-09 |
| SB2 | / | 0.045 | 1.44E-09 |
| SB3 | / | 0.049 | 2.01E-10 |
| SB4 | / | 0.071 | 7.66E-09 |
| SB5 | 0.208 | 0.413 | 8.35E-10 |
| SB6 | / | 0.027 | 7.22E-10 |
| SB7 | / | 0.034 | 1.74E-09 |
| SB10 | / | 0.030 | 3.68E-09 |
| SB11 | 0.035 | 0.038 | 4.76E-10 |
| SB12 | / | / | 2.38E-07 |
| SB13 | / | 0.048 | 1.24E-08 |
| SB14 | 0.056 | 0.067 | 4.97E-10 |
| SB15 | / | 0.042 | 1.16E-08 |
| SB16 | / | 19.580 | 1.78E-07 |
| SB17 | / | 0.046 | 5.52E-09 |
| SB18 | / | 0.121 | 6.99E-09 |

### Example 8: Detection of binding capacity of anti-CDCP1 antibody to tumor cells by single B cell technique

Flow cytometry was used to detect the binding capacity of antibody to tumor cells. MDA-MB-231 cells were subjected to digestion with 5 mM EDTA, centrifuged, and counted, the cells were resuspended with FACS buffer (PBS + 2% FBS). Cell density was adjusted to 4 × 10⁵/ml, 50 µL (2 × 10⁴ tumor cells) were added per well, an Fc blocker (Human TruStain FcX^{™}, Biolegend, 422302) was added for blocking at room temperature for 10 mins, then the antibody to be tested was added at a final concentration of 1.5 µg/ml. The mixture was incubated out at 4 °C for 30 mins, centrifuged and washed, a secondary antibody (PE anti-human IgG Fc Antibody, Biolegend, 410708) was added for incubation, and then detection was performed using a flow cytometer. Geo-MFI was used as the preliminary criterion for determining antibody-cell binding.

As shown in FIG. 2, SB1-15 and SB17 have good binding capacity to MDA-MB-231 cells.

### Example 9: Detection of endocytosis capacity of anti-CDCP1 antibody by single B cell technique

The endocytosis capacity of the antibody was detected for 0.5 to 2 hours. MDA-MB-231 cells were subjected to digestion with 5 mM EDTA, and plated at 2 × 10⁴ cells/well after cell counting. After adding an Fc blocker (Human TruStain FcX ^{™}, Biolegend, 422302) and blocking at room temperature for 10 mins, a test antibody (1.5 µg/mL) was added, and the mixture was incubated at 4 °C for 30 mins, centrifuged, and washed twice with PBS + 2% FBS, then the cells were divided into four equal portions (labeled as 0 h, 0.5 h, 1 h, 2 h), cells labeled as 0.5 h, 1 h, 2 h time points were added to 200 µL of cell culture medium, and cultured at 37 °C for 0.5 h, 1 h, 2 h, respectively. Cells at 0 h point were centrifuged, a secondary antibody (PE anti-human IgG Fc Antibody, Biolegend, 410708) was added, and the mixture was incubated at 4 °C for 30 mins, centrifuged, and washed twice with PBS + 2% FBS, and detection was performed using a flow cytometer. After incubation of cells labeled as 0.5 h, 1 h, and 2 h points at 37 °C was completed, a secondary antibody was added (in the same steps as those at 0 h point), Geo-MFI of the signal under the same voltage was collected as detection signal at 0 h, and antibody internalization rate was calculated according to the following equation: Internalization (%)= (Geo-MFI (0 h)- Geo-MFI (2 h))/ Geo-MFI (0 h) × 100%.

The detection results are shown in Table 6. MDA-MB-231 showed good endocytosis effect on the antibodies in Table 4.

**Table 6 Detection of endocytosis capacity of anti-CDCP1 antibody by single B cell technique**

| Antibody number | MDA-MB-231 (%) |
|---|---|
| SB1 | 34.65 |
| SB2 | 77.69 |
| SB3 | 78.20 |
| SB4 | 83.16 |
| SB5 | 15.63 |
| SB6 | 63.21 |
| SB7 | 31.67 |
| SB10 | 87.72 |
| SB11 | 18.52 |
| SB13 | 50.12 |
| SB14 | 80.03 |
| SB15 | 80.12 |
| SB17 | 80.07 |
| SB18 | 38.83 |
| CDSEQ135 (control antibody) | 46.38 |

### Example 10: Subtype identification and variable region amplification of anti-CDCP1 murine antibody

The anti-CDCP1 murine monoclonal antibody obtained from Example 2 and the anti-CDCP1 antibody obtained by single B cell technology from Example 6 were subcloned and cultured, 5000-1000 monoclonal cells were taken and subjected to centrifugation, small sample RNA extraction was performed using a TaKaRa MiniBEST Universal RNA Extraction Kit (TAKARA, cat #: 9767), RNA was extracted and reverse transcribed into cDNA by PrimeScript^{™} IV 1st strand cDNA Synthesis Mix (TAKARA, cat # 6215A), the obtained DNA was amplified by PCR with VH and VL primers respectively, and the obtained fragment was ligated to pTT5 vector, and then 5-10 clones were selected for DNA sequencing. The sequencing results were analyzed, the CDR region was defined, the IgG family genes were localized, the antibody families were distinguished, and the antibody classification was carried out. The sequences of the variable regions and CDRs of the anti-CDCP1 murine monoclonal antibodies obtained are shown in Table 7:

**Table 7 Amino acid sequences of variable regions and CDRs of anti-CDCP1 murine antibodies**

| Antibody number | Heavy chain variable region (SEQ ID NO:) | Light chain variable region (SEQ ID NO:) | Definition method | Heavy chain CDR1 (SEQ ID NO:) | Heavy chain CDR2 (SEQ ID NO:) | Heavy chain CDR3 (SEQ ID NO:) | Light chain CDR1 (SEQ ID NO:) | Light chain CDR2 (SEQ ID NO:) | Light chain CDR3 (SEQ ID NO:) |
|---|---|---|---|---|---|---|---|---|---|
| 3B12H9 | 239 | 289 | IMGT | 1 | 38 | 80 | 124 | 164 | 197 |
| 3B12H5 | 239 | 290 | IMGT | 1 | 38 | 80 | 125 | 165 | 198 |
| 3C11 | 240 | 291 | IMGT | 2 | 39 | 81 | 125 | 166 | 199 |
| 5G9 | 241 | 292 | IMGT | 3 | 39 | 81 | 126 | 167 | 200 |
| 4E12G10 | 242 | 293 | IMGT | 4 | 40 | 82 | 127 | 168 | 201 |
| 6A7 | 243 | 294 | IMGT | 5 | 41 | 83 | 128 | 169 | 202 |
| 6G9 | 244 | 295 | IMGT | 6 | 42 | 84 | 129 | 170 | 203 |
| 3F1A11 | 245 | 296 | IMGT | 6 | 43 | 85 | 130 | 170 | 203 |
| 20G4 | 246 | 297 | IMGT | 7 | 44 | 86 | 131 | 171 | 204 |
| 27D11 | 247 | 298 | IMGT | 7 | 44 | 86 | 132 | 171 | 204 |
| 3F1A11 -H-12 | 248 | 296 | IMGT | 7 | 44 | 86 | 130 | 170 | 203 |
| 3A9 | 249 | 299 | IMGT | 8 | 45 | 87 | 133 | 170 | 203 |
| 19F7D6 | 250 | 300 | IMGT | 9 | 46 | 88 | 134 | 164 | 205 |
| 3E7 | 251 | 301 | IMGT | 10 | 47 | 89 | 135 | 172 | 206 |
| 5B8 | 252 | 302 | IMGT | 11 | 48 | 90 | 136 | 171 | 207 |
| 4B7 | 253 | 303 | IMGT | 12 | 48 | 91 | 136 | 171 | 207 |
| 8C9 | 254 | 304 | IMGT | 11 | 48 | 90 | 136 | 171 | 207 |
| 12E6 | 255 | 305 | IMGT | 13 | 49 | 92 | 137 | 173 | 208 |
| G10F6 | 256 | 306 | IMGT | 13 | 50 | 93 | 138 | 170 | 209 |
| 14A10C5 | 257 | 307 | IMGT | 13 | 51 | 94 | 139 | 170 | 210 |
| 8E5 | 258 | 308 | IMGT | 14 | 52 | 95 | 140 | 175 | 211 |
| 27A3 | 259 | 309 | IMGT | 15 | 53 | 96 | 124 | 164 | 212 |
| 10D2 | 260 | 310 | IMGT | 16 | 54 | 97 | 141 | 172 | 213 |
| 24G4 | 261 | 311 | IMGT | 17 | 55 | 98 | 126 | 174 | 214 |
| 1CP-061 | 262 | 312 | IMGT | 18 | 56 | 99 | 142 | 176 | 215 |
| 1CP-063 | 263 | 313 | IMGT | 19 | 57 | 100 | 143 | 177 | 216 |
| 1CP-062 | 264 | 314 | IMGT | 20 | 58 | 101 | 144 | 176 | 215 |
| 1CP-067 | 265 | 315 | IMGT | 21 | 59 | 102 | 127 | 168 | 217 |
| 1CP-069 | 266 | 316 | IMGT | 21 | 60 | 102 | 127 | 168 | 217 |
| 1CP-064 | 267 | 317 | IMGT | 13 | 49 | 103 | 144 | 169 | 218 |
| 1CP-066 | 268 | 318 | IMGT | 22 | 61 | 104 | 145 | 178 | 219 |
| 1CP-065 | 269 | 319 | IMGT | 17 | 62 | 105 | 141 | 172 | 220 |
| 1CP-068 | 270 | 320 | IMGT | 17 | 63 | 106 | 126 | 179 | 221 |
| SB1 | 271 | 321 | IMGT | 23 | 64 | 107 | 146 | 180 | 222 |
| SB2 | 272 | 322 | IMGT | 24 | 65 | 108 | 147 | 181 | 223 |
| SB3 | 273 | 323 | IMGT | 25 | 65 | 108 | 148 | 181 | 224 |
| SB4 | 274 | 324 | IMGT | 26 | 66 | 109 | 149 | 182 | 225 |
| SB5 | 275 | 325 | IMGT | 27 | 67 | 110 | 150 | 183 | 226 |
| SB6 | 276 | 326 | IMGT | 28 | 68 | 111 | 151 | 184 | 227 |
| SB7 | 277 | 327 | IMGT | 29 | 69 | 112 | 152 | 185 | 222 |
| SB8 | 278 | 328 | IMGT | 30 | 70 | 113 | 153 | 186 | 228 |
| SB9 | 279 | 329 | IMGT | 31 | 71 | 114 | 154 | 187 | 229 |
| SB10 | 280 | 330 | IMGT | 32 | 72 | 115 | 155 | 188 | 230 |
| SB11 | 281 | 331 | IMGT | 33 | 73 | 116 | 156 | 189 | 231 |
| SB12 | 282 | 332 | IMGT | 34 | 74 | 117 | 157 | 190 | 232 |
| SB13 | 283 | 333 | IMGT | 35 | 75 | 118 | 158 | 191 | 233 |
| SB14 | 284 | 334 | IMGT | 26 | 76 | 119 | 159 | 192 | 234 |
| SB15 | 285 | 335 | IMGT | 24 | 77 | 120 | 160 | 193 | 235 |
| SB16 | 286 | 336 | IMGT | 36 | 78 | 121 | 161 | 194 | 236 |
| SB17 | 287 | 337 | IMGT | 32 | 79 | 122 | 162 | 195 | 237 |
| SB18 | 288 | 338 | IMGT | 37 | 79 | 123 | 163 | 196 | 238 |

### Example 11: Humanization of anti-CDCP1 murine antibodies

The anti-CDCP1 murine monoclonal antibodies 3C11, 8E5 and 8C9 were humanized. Specifically, murine antibody sequences were aligned with the amino acids of human germline antibody to find out sequences that were highly homologous and had better physical and chemical properties as the humanized antibody framework sequences; The CDR regions of the murine antibodies were defined according to IMGT, and the CDR regions of the murine antibodies were transplanted into the frame sequence of a human antibody. Based on sequence analysis and 3D simulation, some CDR and framework region amino acids, as well as amino acids on the antibody surface that may interact, were restored. Further, by computer calculation of mutation energy, best-single mutation, germline substitution, frequent reside substitution, etc., each antibody produced 12 mutants for preliminary affinity assay, antibodies with high affinity and humanization degrees were further expressed to assay the binding capacity to antigens and cells from different species and the internalization. The 12 humanized antibodies produced by 8C9 were: 1CP-151 to 1CP-162; The 12 humanized antibodies produced by 8E5 were: 1CP-112 to 1CP-123; The 12 humanized antibodies produced by 3C11 were: 1CP-124 to 1CP-135.

The amino acid sequences of the CDRs of murine monoclonal antibodies 3C11, 8E5 and 8C9 are shown in Table 8:

**Table 8 Amino acid sequences of CDRs of 3C11, 8E5 and 8C9**

| **Designation of antibody** | **Definition method** | **VH-CDR1** (SEQ ID NO:) | **VH-CDR2** (SEQ ID NO:) | **VH-CDR3** (SEQ ID NO:) | **VL-CDR1** (SEQ ID NO:) | **VL-CDR2** (SEQ ID NO:) | **VL-CDR3** (SEQ ID NO:) |
|---|---|---|---|---|---|---|---|
| 3C11 | IMGT | 2 | 39 | 81 | 125 | 166 | 199 |
| | Kabat | 362 | 363 | 364 | 365 | 366 | 199 |
| | AbM | 367 | 368 | 364 | 365 | 366 | 199 |
| | Chothia | 369 | 370 | 364 | 365 | 366 | 199 |
| | Contact | 371 | 372 | 373 | 374 | 375 | 376 |
| 8E5 | IMGT | 14 | 52 | 95 | 140 | 175 | 211 |
| | Kabat | 377 | 378 | 379 | 380 | 381 | 211 |
| | AbM | 382 | 383 | 379 | 380 | 381 | 211 |
| | Chothia | 384 | 385 | 379 | 380 | 381 | 211 |
| | Contact | 386 | 387 | 388 | 389 | 390 | 391 |
| 8C9 | IMGT | 11 | 48 | 90 | 136 | 171 | 207 |
| | Kabat | 392 | 393 | 394 | 395 | 396 | 207 |
| | AbM | 397 | 398 | 394 | 395 | 396 | 207 |
| | Chothia | 399 | 400 | 394 | 395 | 396 | 207 |
| | Contact | 401 | 402 | 403 | 404 | 405 | 406 |

The amino acid sequences of the variable regions and constant regions of the humanized antibodies are shown in Table 9:

**Table 9 Amino acid sequences of variable regions and constant regions of humanized anti-CDCP1 antibodies**

| Name | Heavy chain variable region (SEQ ID NO:) | Light chain variable region (SEQ ID NO:) | Heavy chain constant region (SEQ ID NO:) | Light chain constant region (SEQ ID NO:) |
|---|---|---|---|---|
| 1CP-112 | 347 | 357 | 360 | 361 |
| 1CP-113 | 348 | 357 | | |
| 1CP-114 | 349 | 357 | | |
| 1CP-115 | 350 | 357 | | |
| 1CP-116 | 347 | 358 | | |
| 1CP-117 | 348 | 358 | | |
| 1CP-118 | 349 | 358 | | |
| 1CP-119 | 350 | 358 | | |
| 1CP-120 | 347 | 359 | | |
| 1CP-121 | 348 | 359 | | |
| 1CP-122 | 349 | 359 | | |
| 1CP-123 | 350 | 359 | | |
| 1CP-124 | 339 | 351 | | |
| 1CP-125 | 340 | 351 | | |
| 1CP-126 | 341 | 351 | | |
| 1CP-127 | 342 | 351 | | |
| 1CP-128 | 339 | 352 | | |
| 1CP-129 | 340 | 352 | | |
| 1CP-130 | 341 | 352 | | |
| 1CP-131 | 342 | 352 | | |
| 1CP-132 | 339 | 353 | | |
| 1CP-133 | 340 | 353 | | |
| 1CP-134 | 341 | 353 | | |
| 1CP-135 | 342 | 353 | | |
| 1CP-151 | 343 | 354 | | |
| 1CP-152 | 344 | 354 | | |
| 1CP-153 | 345 | 354 | | |
| 1CP-154 | 346 | 354 | | |
| 1CP-155 | 343 | 355 | | |
| 1CP-156 | 344 | 355 | | |
| 1CP-157 | 345 | 355 | | |
| 1CP-158 | 346 | 355 | | |
| 1CP-159 | 343 | 356 | | |
| 1CP-160 | 344 | 356 | | |
| 1CP-161 | 345 | 356 | | |
| 1CP-162 | 346 | 356 | | |

### Example 12: Detection of affinity of humanized anti-CDCP1 antibodies

The humanized anti-CDCP1 antibodies obtained from Example 11 were subjected to gene cloning, constructed into pTT5 expression vector for a small amount of expression in CHO cells (Expi-CHOS: Gibco A29127), and purified with an Fc affinity column (AT Protein A Diamond Plus. No. AA402312). Antibody purity was analyzed by HPLC and determined by Nanodrop. High-throughput affinity assay was carried out for a small amount of antibodies by Caterra. Specifically, HuCDCP1-C antigen (2 µg/ml) was immobilized to assay antibody binding, and antibodies to be tested (antibodies diluted to the highest concentration of 150 nM, with a 3-fold dilution across 5 concentration points, which were respectively 1.85, 5.55, 16.67, 50, 150 nM) were allowed to flow through the detection chip respectively by Caterra, and data analysis was performed with Kinetics software.

The detection results are shown in Table 10, and the anti-CDCP1 humanized antibodies in the table showed good affinity with HuCDCP1-C.

**Table 10 Detection of affinity of anti-CDCP1 humanized antibody with HuCDCP1-C**

| Designation of antibody | ka (1/Ms) | kd (1/s) | KD (M) |
|---|---|---|---|
| 1CP-112 | 1.36E+05 | 5.00E-05 | 3.68E-10 |
| 1CP-113 | 1.43E+05 | 5.90E-05 | 4.11E-10 |
| 1CP-114 | 1.61E+05 | 2.46E-03 | 1.52E-08 |
| 1CP-116 | 1.28E+05 | 4.59E-05 | 3.59E-10 |
| 1CP-117 | 1.52E+05 | 3.93E-05 | 2.59E-10 |
| 1CP-118 | 1.68E+05 | 2.25E-03 | 1.34E-08 |
| 1CP-119 | 2.26E+05 | 1.24E-03 | 5.51E-09 |
| 1CP-120 | 1.39E+05 | 1.70E-05 | 1.22E-10 |
| 1CP-121 | 1.48E+05 | 1.00E-05 | 6.75E-11 |
| 1CP-122 | 1.67E+05 | 2.09E-03 | 1.25E-08 |
| 1CP-123 | 1.99E+05 | 2.27E-03 | 1.14E-08 |
| 1CP-124 | 1.48E+05 | 1.47E-05 | 9.93E-11 |
| 1CP-125 | 1.51E+05 | 2.17E-05 | 1.44E-10 |
| 1CP-126 | 2.11E+05 | 1.43E-03 | 6.78E-09 |
| 1CP-127 | 2.05E+05 | 1.98E-03 | 9.66E-09 |
| 1CP-128 | 1.39E+05 | 1.00E-05 | 7.17E-11 |
| 1CP-129 | 1.60E+05 | 1.00E-05 | 6.23E-11 |
| 1CP-130 | 1.84E+05 | 2.13E-03 | 1.16E-08 |
| 1CP-131 | 1.91E+05 | 2.00E-03 | 1.05E-08 |
| 1CP-132 | 1.40E+05 | 1.00E-05 | 7.13E-11 |
| 1CP-133 | 1.83E+05 | 1.00E-05 | 5.46E-11 |
| 1CP-134 | 1.93E+05 | 2.02E-03 | 1.05E-08 |
| 1CP-135 | 2.49E+05 | 1.20E-03 | 4.80E-09 |
| 1CP-151 | 1.58E+05 | 3.02E-03 | 1.91E-08 |
| 1CP-152 | 1.14E+05 | 4.65E-04 | 4.09E-09 |
| 1CP-153 | 1.08E+05 | 3.88E-04 | 3.58E-09 |
| 1CP-154 | 1.18E+05 | 3.46E-04 | 2.92E-09 |
| 1CP-155 | 1.58E+04 | 5.79E-04 | 3.66E-08 |
| 1CP-156 | 1.23E+05 | 4.41E-04 | 3.60E-09 |
| 1CP-157 | 1.22E+05 | 3.85E-04 | 3.14E-09 |
| 1CP-158 | 1.09E+05 | 3.21E-04 | 2.96E-09 |
| 1CP-159 | 1.80E+04 | 7.67E-04 | 4.25E-08 |
| 1CP-160 | 1.10E+05 | 4.20E-04 | 3.80E-09 |
| 1CP-161 | 1.14E+05 | 3.65E-04 | 3.21E-09 |
| 1CP-162 | 1.13E+05 | 3.05E-04 | 2.70E-09 |

Further, Partial anti-CDCP1 humanized antibodies were selected for expression, and then the affinity of the antibodies to the antigens Hu-CDCP1-A, Hu-CDCP1-B, Cyno-CDCP1, and Ms-CDCP1 was detected according to the above affinity detection method. It can be seen that the antibodies in Table 11 had good affinity with human and monkey CDCP1, and some antibodies showed the binding capacity to mouse CDCP1.

**Table 11 Detection of affinity of humanized anti-CDCP1 antibodies with Hu-CDCP1-A, Hu-CDCP1-B, Cyno-CDCP1, and Ms-CDCP1**

| Name | Hu-CDCP1-A KD (M) | Hu-CDCP1-B KD (M) | Cyno-CDCP1 KD (M) | Ms-CDCP1 KD (M) |
|---|---|---|---|---|
| 1CP-117 | 7.65E-08 | 1.82E-07 | 1.84E-09 | 2.12E-07 |
| 1CP-121 | 9.98E-08 | 1.31E-07 | 1.51E-09 | 1.41E-07 |
| 1CP-124 | 1.32E-07 | 1.00E-07 | 2.42E-08 | 2.74E-07 |
| 1CP-125 | 5.59E-07 | 5.51E-08 | 2.27E-09 | / |
| 1CP-128 | 7.60E-08 | 1.09E-07 | 4.75E-08 | / |
| 1CP-129 | 1.26E-07 | 1.57E-07 | 5.38E-08 | / |
| 1CP-152 | 1.05E-07 | 1.10E-07 | 2.90E-09 | 7.45E-07 |
| 1CP-157 | 2.26E-07 | 3.59E-07 | 2.60E-09 | / |

### Example 13: Detection of binding capacity of anti-CDCP1 humanized antibody to tumor cells

Referring to the method of Example 4, MDA-MB-231 cells were taken and subjected to digestion with 5 mM EDTA, 2 × 10⁴ tumor cells were added respectively per well, and 50 µL (PBS + 2% FBS) were added; after blocking at room temperature with Fc blocker for 10 mins, an anti-CDCP1 humanized antibody at a final concentration of 200 nM was added. The obtained mixture was subjected to a 3-fold serial dilution across 8 concentration points, incubated at 4 °C for 30 mins, centrifuged and washed, and then a secondary antibody (PE anti-human IgG Fc Antibody, Biolegend, 410708) was added, then detection was performed using a flow cytometer. Geo-MFI (geometric mean fluorescence signal value) was used as the preliminary signal of antibody-cell binding, curve fitting was performed using graphpad, and the binding capacity of antibody to cells was calculated. As can be seen from the results in Table 12, the humanized antibodies in Table 12 have good cellular binding capacity to MDA-MB-231.

**Table 12 Detection of affinity of humanized anti-CDCP1 antibodies**

| Humanized clone No. | MDA-MB-231 (EC50, nM) |
|---|---|
| 1CP-117 | 1.30 |
| 1CP-121 | 1.37 |
| 1CP-124 | 0.38 |
| 1CP-125 | 0.95 |
| 1CP-128 | 0.32 |
| 1CP-129 | 0.34 |
| 1CP-152 | 2.82 |
| 1CP-157 | 2.50 |
| CDSEQ135 | 0.41 |

### Example 14: Detection of endocytosis capacity of anti-CDCP1 humanized antibodies

The endocytosis capacity of the anti-CDCP1 humanized antibody was detected with reference to example 5. MDA-MB-231 cells were subjected to digestion with 5 mM EDTA, and plated at 3 × 10⁴ cells/well after cell counting. After adding an Fc blocker and blocking at room temperature for 10 mins, a test antibody (1.5 µg/mL) was added, and the mixture was incubated at 4 °C for 30 mins, centrifuged, and washed twice with PBS + 2% FBS, then the cells were divided into three equal portions (labeled as 0 h, 1 h, 2 h), cells labeled as 1 h and 2 h time points were added to 200 µL of cell culture medium, and cultured at 37 °C for 1 h and 2 h, respectively. Cells at 0 h point were centrifuged, a secondary antibody (PE anti-human IgG Fc Antibody, Biolegend, 410708) was added, and the mixture was incubated at 4 °C for 30 mins, centrifuged, and washed twice with PBS + 2% FBS, and detection was performed using a flow cytometer. After incubation of cells labeled as 1 h and 2 h points at 37 °C was completed, a secondary antibody was added (in the same steps as those at 0 h point), Geo-MFI of the signal under the same voltage was collected as detection signal at 0 h, and antibody internalization rate was calculated according to the following equation: Internalization (%)= (Geo-MFI (0 h)-Geo-MFI (1, 2 h))/ Geo-MFI (0 h) × 100%.

The detection results are shown in Table 13, and the humanized antibody had a good internalization effect at the cellular level.

**Table 13 Detection of endocytosis capacity of humanized anti-CDCP1 antibodies**

| Designation of antibody | 1 h endocytosis (%) | 2 h endocytosis (%) |
|---|---|---|
| 1CP-117 | 45.68 | 68.37 |
| 1CP-121 | 51.49 | 72.98 |
| 1CP-124 | 38.17 | 56.39 |
| 1CP-125 | 79.56 | 88.62 |
| 1CP-128 | 36.87 | 55.68 |
| 1CP-129 | 36.18 | 54.25 |
| 1CP-152 | 54.39 | 69.96 |
| 1CP-157 | 56.06 | 70.74 |

### Example 15: Laser confocal detection of antibody internalization in tumor cell lines

8E5, 10D2, 3B12H5, 3C11, 8C9, G10F6, 5B8, 1CP-063, 1CP-064, 1CP-062, 1CP-065, SBS, SB11, SB14 were directly labeled with green fluorescent dye, and then incubated with HCC1806 (a breast cancer cell with high expression of CDCP1) tumor cells, the binding of antibodies on cell membrane surface was detected under a laser confocal microscope, and the transfer of antibodies to cells was detected at 0, 5, 15, 30, 60, 90 mins, respectively. The lysosomes were labeled with red fluorescence, and the relative position changes and colocalization of green fluorescence and red fluorescence in the cells were detected to determine whether the antibodies were colocalized with lysosomes after endocytosis into the cells.

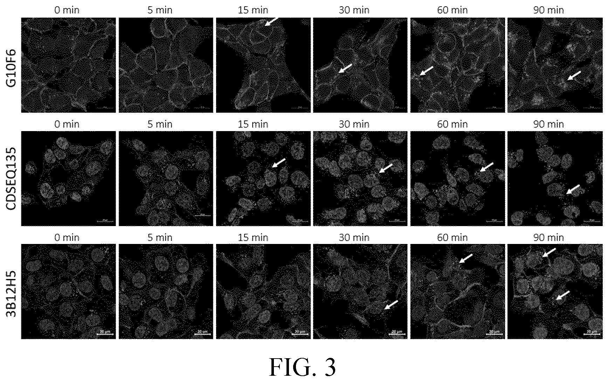

The antibody results detected in this experiment are consistent with the results shown in FIG. 3, indicating that the detected antibodies can obviously bind to the cell surface, which is consistent with the antibody-cell binding detected by flow cytometry. With the increase of time, the intracellular green fluorescence signal gradually strengthened, indicating that the green fluorescence signal on the cell surface gradually transferred into the cell, and the antibody was internalized; In addition, the gradual strengthening of the orange fluorescence signal (the superposition of the green and red signals, indicated by the arrows) indicated that the antibody had entered the lysosome after internalization. Therefore, the antibody provided by the present disclosure can enter lysosomes through tumor cell endocytosis.

### Example 16: Detection of the effect of anti-CDCP1 antibody on proliferation and migration of tumor cell lines

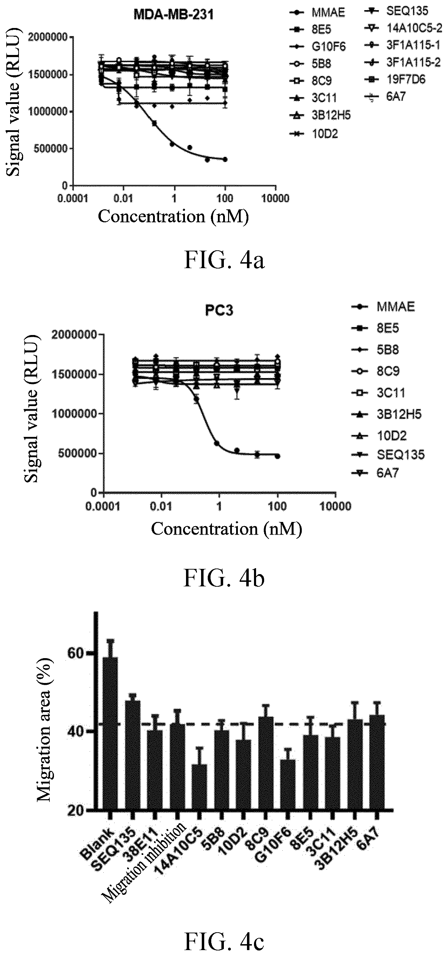

3000 cells (breast cancer MDA-MB-231, prostate cancer PC-3 cells)/well were plated onto 96-well plates overnight, an antibody at a final concentration of 500 nM to 0.00128 nM (100 nM to 0.000256 nM, 100, 20, 4, 0.8, 0.16, 0.032, 0.0064, 0.00128, 0.000256 nM, respectively) was added; after a 5-fold dilution across 9 concentration points and culturing in a CO₂ incubator at 37 °C for 96 h, half of the medium (100 µL) was discarded, and 100 µL of CellTiter-Glo Luminescent Cell Viability assay reagent (Promega, cat # G7570) was added to detect signal values on a BMG microplate reader. The inhibition of antibody on tumor cell growth was calculated by graphpad fitting based on the signal value and antibody concentration. As can be seen from FIGS. 4a and 4b, the detected antibodies 8E5, G10F6, 5B8, 8C9, 3C11, 3B12H5, 10D2, SEQ135, 14A10C5-2, 3F1A115-1, 3F1A115-2, 19F7D6, 6A7 did not exhibit an effect on tumor cell growth.

In order to determine the effect of antibodies on tumor cell metastasis, the migration ability of tumor cells was examined in this study according to a transwell experiment. The specific cell migration experiment involved culturing and collecting MDA-MB-231 cells, washing them once with PBS, adjusting the density to 8 × 10⁵ cells/mL, adding the antibody to be tested to the cells such that the working concentration thereof was 10 µg/ml, co-incubating for 30 mins, adding a cell suspension to the upper chamber of the transwell chamber, placing the transwell chamber in the incubator for 12-48 h, then removing the chamber (transwell chamber, to examine cell migration ability), immobilizing the cells, staining the cells with crystal violet for 15 mins, washing, air drying, mounting, and taking photos of 4-5 fields of view under a microscope, and counting the number or area of the stained cells using imageJ analysis software, the number or area was used as the basis for determining tumor cell migration.

The results are shown in FIG. 4c. The detected antibodies 38E11, 14A10C5, 5B8, 10D2, 8C9, G10F6, 8E5, 3C11, 3B12H5 and 6A7 had a better capability to inhibit tumor cell migration than SEQ135, and thus resulted in weaker potential side effects associated with tumor migration.

The results showed that the detected antibody could not affect the proliferation of tumor cells, but the control antibody SEQ135 could cause the migration of tumor cells. However, the antibody found in this study did not show the ability to promote the migration of tumor cells, on the contrary, it had certain inhibition ability on tumor metastasis.

### Example 17: Coupling of anti-CDCP1 antibody to vc-MMAE

Anti-CDCP1 chimeric antibody was coupled to MMAE via a vc linker. The structure of vc-MMAE is shown below: 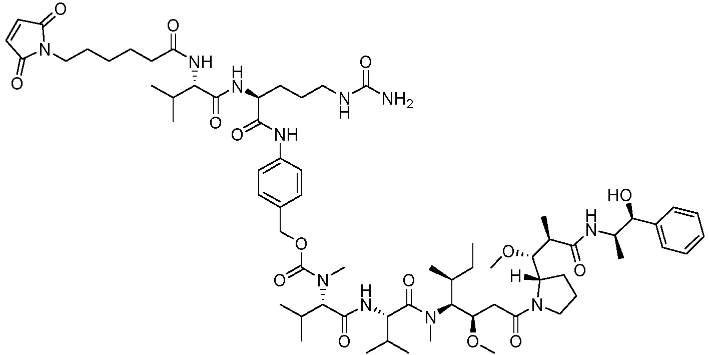 where the structure of vc linker is shown below:

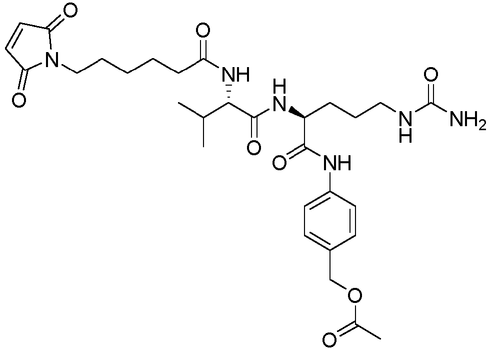

The specific operation was to reduce each antibody and the control antibody CD27H10 (27H10 antibody in WO2018112334A1) with a 2-fold molar amount of P-trichloroethyl phosphate (TECP) at 4 °C for 1 hour. A 7-fold molar amount of vcMMAE was then added for ligation at 4-25 °C for 1 h, and then the reaction mixture was dialyzed against 1 × PBS pH 8.0 and filtered for sterilization. Drug : antibody ratio (DAR) was detected by hydrophobic kurtosis with HLPC, and the molecular coupling results are shown in Table 14.

**Table 14 DAR values of antibody-drug conjugates**

| ADC | DAR | DARO (%) | DAR2 (%) | DAR4 (%) | DAR6 (%) | DAR8 (%) |
|---|---|---|---|---|---|---|
| 3C11-MMAE | 3.48 | 9.01 | 33.89 | 28.50 | 8.28 | 9.09 |
| 3B 12H5-MMAE | 3.2 | 5.752 | 29.966 | 37.81 | 7.324 | 1.741 |
| 5B8-MMAE | 3.85 | 6.56 | 30.01 | 38.18 | 12.42 | 12.16 |
| 10D2-MMAE | 3.5 | 8.28 | 34.10 | 39.38 | 10.24 | 8.00 |
| 8E5-MMAE | 3.65 | 7.97 | 32.01 | 37.12 | 11.15 | 10.61 |
| 8C9-MMAE | 3.44 | 9.52 | 34.27 | 34.56 | 12.32 | 7.73 |
| G10F6-MMAE | 3.6 | 8.34 | 32.59 | 35.66 | 12.02 | 10.04 |
| 6A7-MMAE | 3.6 | 4.87 | 24.99 | 38.25 | 12.28 | 5.30 |
| 14A10C5-MMAE | 4.3 | 3.92 | 17.62 | 38.37 | 14.63 | 17.44 |
| SB11-MMAE | 4 | 5.512 | 12.686 | 63.822 | 10.043 | 3.223 |
| SB 14-MMAE | 3.3 | 12.187 | 35.306 | 24.545 | 13.283 | 8.006 |
| CD27H10-MMAE | 3.87 | 3.187 | 26.84 | 50.60 | 12.80 | 4.11 |
| IgG1-MMAE | 3.9 | 4.187 | 23.713 | 40.963 | 12.978 | 6.485 |

### Example 18: Killing analysis of chimeric antibody-vc-MMAE conjugate on cancer cells in vitro

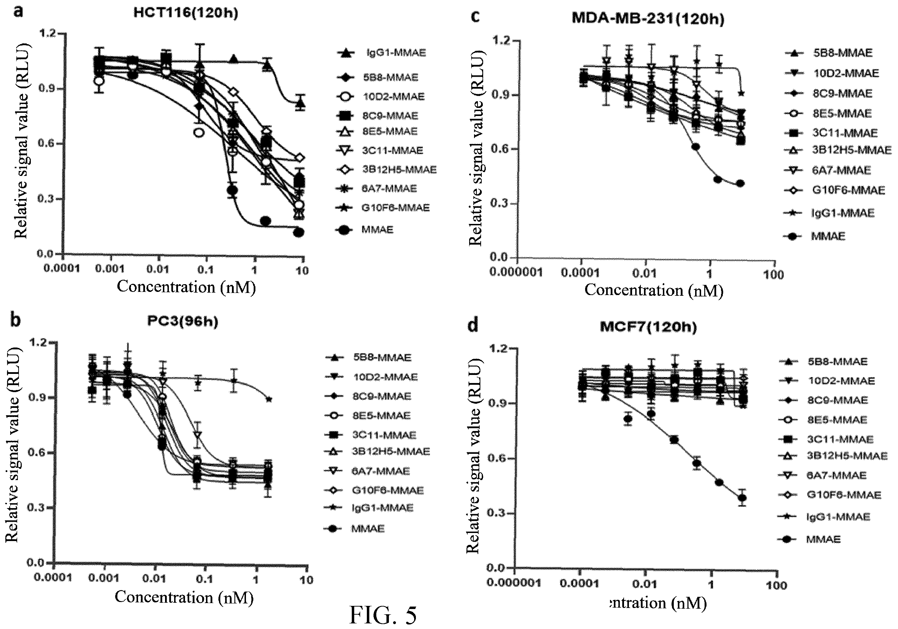

Colorectal cancer cells HCT116 (Nanjing Cobioer, CBP60028), breast cancer cells MDA-MB-231 (Nanjing Cobioer, CBP60382), prostate cancer cells PC3 (Nanjing Cobioer, CBP60343) that express CDCP1, and MCF7 cells that do not express CDCP1 (Nanjing Cobioer, CBP60380) were used to detect the cell killing activity of the chimeric antibody ADC molecule. The specific steps were: Colorectal cancer cells HCT116, breast cancer cells MDA-MB-231, prostate cancer cells PC3, MCF7 cells were respectively counted, adjusted to 3000 cells/well, and plated onto 96-well plates overnight; Conjugates were added at a final concentration of 8 nM to 0.000512 nM (a 5-fold dilution across 7 concentration points), totaling 200 µL, cultured at 37 °C in a CO₂ incubator for 120 h, half of the medium (100 µL) was discarded, and 100 µL of CellTiter-Glo Luminescent Cell Viability assay reagent (Promega, cat # G7570) was added to detect signal values on a BMG microplate reader. The inhibition activity of antibody on tumor cell growth was calculated by graphpad fitting based on the signal value and antibody concentration, and the results are shown in FIG. 5 and Table 15.

**Table 15 IC₅₀ value of anti-CDCP1 chimeric antibody -vcMMAE conjugate on tumor cells**

| IC50 (nM) | 5B8-MMAE | 10D2-MMAE | 8C9-MMAE | 8E5-MMAE | 3C11-MMAE | 3B12H5-MMAE | 6A7-MMAE | G10F6-MMAE | IgG1-MMAE | MMAE |
|---|---|---|---|---|---|---|---|---|---|---|
| HCT116 (120h) | 11.73 | 11.39 | 13.03 | 10.59 | 2.329 | 6.867 | 5.844 | 1.289 | 17.01 | 0.108 |
| PC3 (96 h) | 0.01403 | 0.0164 | 0.01839 | 0.02048 | 0.01188 | 0.02249 | 0.05275 | 0.01058 | 3.903 | 0.00101 |
| MDA-MB-231 (120 h) | >200 | >200 | >40 | >40 | >40 | >40 | >40 | >40 | >40 | 0.1676 |
| MCF7 (120 h) | >200 | >200 | >200 | ~200 | >40 | >40 | >40 | >200 | >40 | 0.07771 |

The results showed that the detected chimeric antibody ADC molecules could effectively kill PC3, HCT116 and MDA-MB-231 tumor cells expressing CDCP1, but had little killing effect on MCF7 cells not expressing CDCP1.

### Example 19: Inhibition of chimeric antibody-vc-MMAE conjugate on the tumor growth of human colorectal cancer cell HCT116 in mouse model in vivo.

20.1 Cell culture: HCT116 cells were cultured with McCoy's 5a + 10% FBS + 1% PS (Penicillin Streptomycin) liquid medium. HCT116 cells in logarithmic growth phase were collected and resuspended to 5 × 10⁷ cells/mL in McCoy's 5a medium, and then used for subcutaneous tumor inoculation in BALB/c nude mice (purchased from Jiangsu Gempharmatech Co., Ltd.).

20.2 Animal modeling: 150 BALB/c nude mice were acclimated to a laboratory environment for 3-7 days. HCT116 cells were resuspended in McCoy's 5a medium, and then resuspended into 5×10⁶ cells, and then subcutaneously inoculated into the right back of experimental mice according to the amount of 0.1 mL per mouse, and the tumor growth was observed regularly. When the tumor of mice grew to an average volume of about 150-200 mm³, the mice were randomly divided into groups according to the tumor size, with 8 mice in each group, so as to ensure that the tumor volume of different groups was similar and the weight of mice was similar. The grouping day was defined as Day 1.

20.3 Administration and observation: CD27H10-MMAE, IgG1-MMAE, 5B8-MMAE, 10D2-MMAE, 8C9-MMAE, G10F6-MMAE, 8E5-MMAE, 3C11-MMAE, 3B12H5-MMAE, 6A7-MMAE and 14A10C5-MMAE were diluted with normal saline on the day of grouping, administered via tail vein at a dose of 1.5 mg/kg, with dosing volume adjusted based on body weight (dosing volume = 5 µl/g), twice a week for a total of 4 doses, and normal saline served as a vehicle group. After tumor inoculation, routine monitoring included tumor growth and the impact of drugs on the normal behavior of animals, such as the mobility, food and water intake, weight gain or loss, eyes, fur, feces, and other abnormalities of experimental animals. Clinical symptoms observed during experiment were recorded in the raw data. After initiation of administration, mice were measured twice a week for body weight and tumor size.

The calculation formula of tumor volume (mm³) was: V = 1/2 × (a × b2), where a = long diameter and b = short diameter.

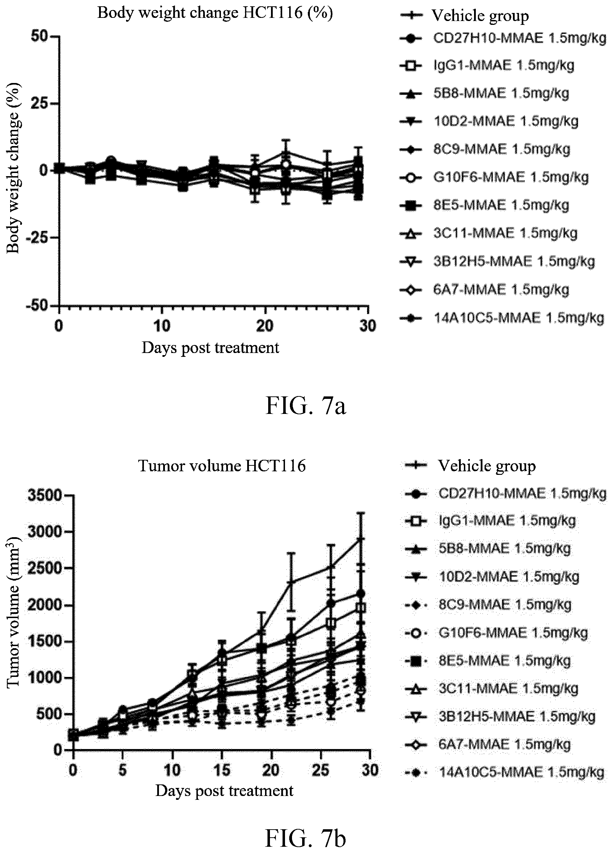

The results are shown in FIG. 7a. There was no significant change in mouse body weight after administration. As shown in FIG. 7b, the antibody-vc-MMAE conjugate of the present disclosure exhibits a better *in vivo* tumor growth inhibition effect than the control CD27H10-MMAE.

### Example 20: Comparison of druggability of anti-CDCP1 antibody liked with vc-MMAE

The 1CP-152 antibody and the control antibody CD27H10 were respectively linked to the vc-MMAE with reference to the method of example 18, and the target DAR was 4. After that, the molecular physical and chemical properties were detected and analyzed, and the specific analysis method was as follows:

### Thermal stability detection:

The denaturation temperature (Tm value), aggregation temperature (Tagg value) and hydration kinetic diameter of ADC molecules were measured by UNcle multifunctional protein stability analysis system. Wherein, the Tm value was based on endogenous fluorescence spectroscopy method, and was fitted and calculated according to BCM algorithm, the Tagg value was measured by static light scattering (SLS) technology, and the hydration dynamics diameter was measured by dynamic light scattering (DLS) technology.
SEC purity detection: The purity of ADC molecules was analyzed by high performance liquid chromatography (HPLC).
Liquid chromatograph: Shimadzu LC-2030C Plus
chromatographic column: TSKgel G3000SWXL, 7.8× 300 mm, 5 µm, TOSOH
mobile phase: 61 mmol/L Na2HPO4, 39 mmol/L NaH2PO4, 200 mmol/L NaCl, pH6.8
flow rate: 1.0 ml/min
Detection wavelength: UV 220 nm
column temperature: 30 °C
Sample chamber temperature: 10 °C
Injection volume: 20 µg
Isocratic running time: 30 min

Quantitative analysis of the results was performed using peak area normalization method. If automatic integration failed due to reasons such as baseline drift, retention time variation, peak shape change or other reasons, integration parameters should be adjusted or the spectrum should be manually integrated. The main peak (MP) was the monomer peak, the peak with a retention time before the main peak was the aggregate peak (HMW), and the peak with a retention time after the main peak was the low molecular weight impurity peak (LMW).

Tm represents the temperature at which 50% of the protein molecules unfold during the thermal denaturation process, and characterizes the conformational stability of the molecule. From the comparison of the results, it can be seen that the Tm value of 1CP-152-MMAE is similar to that of CD27H10-MMAE. It shows that the conformational stability of the two methods is comparable. Tagg represents the initial temperature at which the protein molecules aggregate, and characterizes the colloidal stability of the protein. It can be learned from the data in Table 16 that the Tagg value of 1CP-152-MMAE was 64.1°C, and the Tagg value of CD27H10-MMAE was 60.1°C. The difference in Tagg values between the two was 4°C, which is a significant difference, indicating that during heating process, CD27H10-MMAE aggregated before 1CP-152-MMAE, that is, 1CP-152-MMAE had better colloidal stability; In addition, comparing the hydration kinetic diameters of the two molecules, it can be seen that 1CP-152-MMAE has a lower particle size. The results indicate that 1CP-152-MMAE diffuses faster in solution and has stronger mutual repulsion, indicating that 1CP-152-MMAE has better long-term storage stability.

**Table 16 Thermal stability of ADC molecules**

| Sample | Tm/°C | Tagg/°C | Mean particle size/nm |
|---|---|---|---|
| 1CP-152-MMAE | 76.6 | 64.1 | 12.8 |
| CD27H10-MMAE | 76.1 | 60.1 | 13.9 |

In addition, 1CP-152-MMAE and CD27H10-MMAE were placed at 2 to 8°C for different time (0 days, 3 days, 5 days) to investigate the change in purity of the two molecules. As shown in Table 17, the purity of 1CP-152-MMAE did not change after leaving at 2-8°C for 5 days and the purity of CD27H10-MMAE decreased by 0.5% after leaving at 2-8°C for 5 days, indicating that 1CP-152-MMAE had better long-term storage stability.

**Table 17 Long-term stability of ADC molecules**

| Sample | D0 | | | D3 | | | D5 | | |
|---|---|---|---|---|---|---|---|---|---|
| | HMW | MP | LMW | HMW | MP | LMW | HMW | MP | LMW |
| 1CP-152-MMAE | 1.9 | 97.2 | 0.9 | 1.9 | 97.3 | 0.8 | 1.9 | 97.3 | 0.8 |
| CD27H10-MMAE | 2.6 | 96.8 | 0.6 | 2.9 | 96.4 | 1.7 | 3 | 96.3 | 0.8 |

### Example 21: Killing analysis of anti-CDCP1 humanized antibody-vc-MMAE conjugate on cancer cells in vitro

The humanized antibodies 1CP-125, 1CP-117 and 1CP-152 were subjected to linking to form ADC, and the linking process was the same as in example 18. The molecular coupling results are shown in Table 18.

**Table 18 DAR values of antibody-drug conjugates**

| ADC | DAR | DAR0 (%) | DAR2 (%) | DAR4 (%) | DAR6 (%) | DAR8 (%) |
|---|---|---|---|---|---|---|
| 1CP-125-MMAE | 4.3 | 4.27 | 9.52 | 64.95 | 15.96 | 5.31 |
| 1CP-117-MMAE | 4.8 | 4.78 | 7.01 | 50.02 | 23.01 | 15.19 |
| 1CP-152-MMAE | 4.5 | 3.33 | 11.10 | 53.89 | 22.11 | 9.57 |

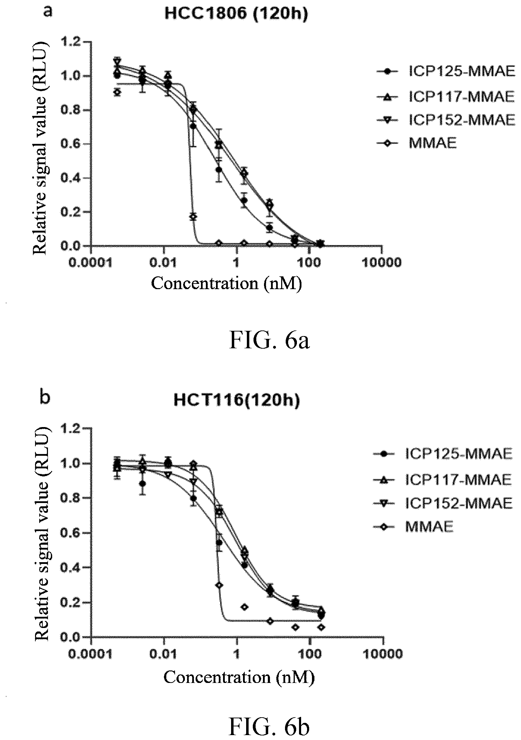

The colorectal cancer cells HCT116 and breast cancer cells HCC1806 were counted separately, adjusted to 3000 cells/well and plated in 96-well plates overnight. Conjugates were added at a final concentration of 200 nM to 0.000512 nM (a 5-fold dilution across 9 concentration points), totaling 200 µL, cultured at 37°C in a CO₂ incubator for 120 h, half of the medium (100 µL) was discarded, and 100 µL of CellTiter-Glo Luminescent Cell Viability assay reagent (Promega, cat # G7570) was added to detect signal values on a BMG microplate reader. The inhibition activity of antibody on tumor cell growth was calculated by graphpad fitting based on the signal value and antibody concentration, and the results are shown in FIGS. 6a and 6b and Tables 19 and 20.

**Table 19 DAR value of anti-CDCP1 humanized antibody-vc-MMAE conjugate and its IC50 value for HCC1806**

| HCC1806 | 1CP-125-MMAE | 1CP-117-MMAE | 1CP-152-MMAE | MMAE |
|---|---|---|---|---|
| IC50 (nM) | 0.272 | 0.894 | 0.734 | 0.053 |
| DAR | 4.3 | 4.8 | 4.5 | / |

**Table 20 DAR value of anti-CDCP1 humanized antibody-vcMMAE conjugate and its IC50 value for HCT116**

| HCT116 | 1CP-125-MMAE | 1CP-117-MMAE | 1CP-152-MMAE | MMAE |
|---|---|---|---|---|
| IC50 (nM) | 0.668 | 1.508 | 1.274 | 0.283 |
| DAR | 4.3 | 4.8 | 4.5 | / |

The results showed that the detected humanized ADC molecules had significant tumor cell killing effects on both HCC1806 and HCT116 cells.

Although specific embodiments of the present disclosure have been described in detail, those skilled in the art will understand that: in accordance with all the published teachings, various modifications and changes to the details may be made, and these changes are within the scope of protection of the present disclosure. The entirety of the present disclosure is given by the following claims and any equivalents thereof.

## Claims

1. An antibody or an antigen-binding fragment thereof that specifically binds to CDCP1, wherein,
the antibody or the antigen-binding fragment thereof comprises the following heavy chain variable region (VH) and light chain variable region (VL), wherein CDRs are defined according to the IMGT numbering system:
(1) VH comprising the following 3 CDRs: CDR-H1 comprising the sequence as set forth in SEQ ID NO: 11, CDR-H2 comprising the sequence as set forth in SEQ ID NO: 48, and CDR-H3 comprising the sequence as set forth in SEQ ID NO: 90; and
VL comprising the following 3 CDRs: CDR-L1 comprising the sequence as set forth in SEQ ID NO: 136, CDR-L2 comprising the sequence as set forth in SEQ ID NO: 171, and CDR-L3 comprising the sequence as set forth in SEQ ID NO: 207;
or
(2) VH comprising the following 3 CDRs: CDR-H1 comprising the sequence as set forth in SEQ ID NO: 14, CDR-H2 comprising the sequence as set forth in SEQ ID NO: 52, and CDR-H3 comprising the sequence as set forth in SEQ ID NO: 95; and
VL comprising the following 3 CDRs: CDR-L1 comprising the sequence as set forth in SEQ ID NO: 140, CDR-L2 comprising the sequence as set forth in SEQ ID NO: 175, and CDR-L3 comprising the sequence as set forth in SEQ ID NO: 211;
or
(3) VH comprising the following 3 CDRs: CDR-H1 comprising the sequence as set forth in SEQ ID NO: 2, CDR-H2 comprising the sequence as set forth in SEQ ID NO: 39, and CDR-H3 comprising the sequence as set forth in SEQ ID NO: 81; and
VL comprising the following 3 CDRs: CDR-L1 comprising the sequence as set forth in SEQ ID NO: 125, CDR-L2 comprising the sequence as set forth in SEQ ID NO: 166, and CDR-L3 comprising the sequence as set forth in SEQ ID NO: 199;
or
(4) VH comprising the following 3 CDRs: CDR-H1 comprising the sequence as set forth in SEQ ID NO: 1, CDR-H2 comprising the sequence as set forth in SEQ ID NO: 38, and CDR-H3 comprising the sequence as set forth in SEQ ID NO: 80; and
VL comprising the following 3 CDRs: CDR-L1 comprising the sequence as set forth in SEQ ID NO: 124, CDR-L2 comprising the sequence as set forth in SEQ ID NO: 164, and CDR-L3 comprising the sequence as set forth in SEQ ID NO: 197;
or
(5) VH comprising the following 3 CDRs: CDR-H1 comprising the sequence as set forth in SEQ ID NO: 1, CDR-H2 comprising the sequence as set forth in SEQ ID NO: 38, and CDR-H3 comprising the sequence as set forth in SEQ ID NO: 80; and
VL comprising the following 3 CDRs: CDR-L1 comprising the sequence as set forth in SEQ ID NO: 125, CDR-L2 comprising the sequence as set forth in SEQ ID NO: 165, and CDR-L3 comprising the sequence as set forth in SEQ ID NO: 198;
or
(6) VH comprising the following 3 CDRs: CDR-H1 comprising the sequence as set forth in SEQ ID NO: 3, CDR-H2 comprising the sequence as set forth in SEQ ID NO: 39, and CDR-H3 comprising the sequence as set forth in SEQ ID NO: 81; and
VL comprising the following 3 CDRs: CDR-L1 comprising the sequence as set forth in SEQ ID NO: 126, CDR-L2 comprising the sequence as set forth in SEQ ID NO: 167, and CDR-L3 comprising the sequence as set forth in SEQ ID NO: 200;
or
(7) VH comprising the following 3 CDRs: CDR-H1 comprising the sequence as set forth in SEQ ID NO: 4, CDR-H2 comprising the sequence as set forth in SEQ ID NO: 40, and CDR-H3 comprising the sequence as set forth in SEQ ID NO: 82; and
VL comprising the following 3 CDRs: CDR-L1 comprising the sequence as set forth in SEQ ID NO: 127, CDR-L2 comprising the sequence as set forth in SEQ ID NO: 168, and CDR-L3 comprising the sequence as set forth in SEQ ID NO: 201;
or
(8) VH comprising the following 3 CDRs: CDR-H1 comprising the sequence as set forth in SEQ ID NO: 5, CDR-H2 comprising the sequence as set forth in SEQ ID NO: 41, and CDR-H3 comprising the sequence as set forth in SEQ ID NO: 83; and
VL comprising the following 3 CDRs: CDR-L1 comprising the sequence as set forth in SEQ ID NO: 128, CDR-L2 comprising the sequence as set forth in SEQ ID NO: 169, and CDR-L3 comprising the sequence as set forth in SEQ ID NO: 202;
or
(9) VH comprising the following 3 CDRs: CDR-H1 comprising the sequence as set forth in SEQ ID NO: 6, CDR-H2 comprising the sequence as set forth in SEQ ID NO: 42, and CDR-H3 comprising the sequence as set forth in SEQ ID NO: 84; and
VL comprising the following 3 CDRs: CDR-L1 comprising the sequence as set forth in SEQ ID NO: 129, CDR-L2 comprising the sequence as set forth in SEQ ID NO: 170, and CDR-L3 comprising the sequence as set forth in SEQ ID NO: 203;
or
(10) VH comprising the following 3 CDRs: CDR-H1 comprising the sequence as set forth in SEQ ID NO: 6, CDR-H2 comprising the sequence as set forth in SEQ ID NO: 43, and CDR-H3 comprising the sequence as set forth in SEQ ID NO: 85; and
VL comprising the following 3 CDRs: CDR-L1 comprising the sequence as set forth in SEQ ID NO: 130, CDR-L2 comprising the sequence as set forth in SEQ ID NO: 170, and CDR-L3 comprising the sequence as set forth in SEQ ID NO: 203;
or
(11) VH comprising the following 3 CDRs: CDR-H1 comprising the sequence as set forth in SEQ ID NO: 7, CDR-H2 comprising the sequence as set forth in SEQ ID NO: 44, and CDR-H3 comprising the sequence as set forth in SEQ ID NO: 86; and
VL comprising the following 3 CDRs: CDR-L1 comprising the sequence as set forth in SEQ ID NO: 131, CDR-L2 comprising the sequence as set forth in SEQ ID NO: 171, and CDR-L3 comprising the sequence as set forth in SEQ ID NO: 204;
or
(12) VH comprising the following 3 CDRs: CDR-H1 comprising the sequence as set forth in SEQ ID NO: 7, CDR-H2 comprising the sequence as set forth in SEQ ID NO: 44, and CDR-H3 comprising the sequence as set forth in SEQ ID NO: 86; and
VL comprising the following 3 CDRs: CDR-L1 comprising the sequence as set forth in SEQ ID NO: 132, CDR-L2 comprising the sequence as set forth in SEQ ID NO: 171, and CDR-L3 comprising the sequence as set forth in SEQ ID NO: 204;
or
(13) VH comprising the following 3 CDRs: CDR-H1 comprising the sequence as set forth in SEQ ID NO: 7, CDR-H2 comprising the sequence as set forth in SEQ ID NO: 44, and CDR-H3 comprising the sequence as set forth in SEQ ID NO: 86; and
VL comprising the following 3 CDRs: CDR-L1 comprising the sequence as set forth in SEQ ID NO: 130, CDR-L2 comprising the sequence as set forth in SEQ ID NO: 170, and CDR-L3 comprising the sequence as set forth in SEQ ID NO: 203;
or
(14) VH comprising the following 3 CDRs: CDR-H1 comprising the sequence as set forth in SEQ ID NO: 8, CDR-H2 comprising the sequence as set forth in SEQ ID NO: 45, and CDR-H3 comprising the sequence as set forth in SEQ ID NO: 87; and
VL comprising the following 3 CDRs: CDR-L1 comprising the sequence as set forth in SEQ ID NO: 133, CDR-L2 comprising the sequence as set forth in SEQ ID NO: 170, and CDR-L3 comprising the sequence as set forth in SEQ ID NO: 203;
or
(15) VH comprising the following 3 CDRs: CDR-H1 comprising the sequence as set forth in SEQ ID NO: 9, CDR-H2 comprising the sequence as set forth in SEQ ID NO: 46, and CDR-H3 comprising the sequence as set forth in SEQ ID NO: 88; and
VL comprising the following 3 CDRs: CDR-L1 comprising the sequence as set forth in SEQ ID NO: 134, CDR-L2 comprising the sequence as set forth in SEQ ID NO: 164, and CDR-L3 comprising the sequence as set forth in SEQ ID NO: 205;
or
(16) VH comprising the following 3 CDRs: CDR-H1 comprising the sequence as set forth in SEQ ID NO: 10, CDR-H2 comprising the sequence as set forth in SEQ ID NO: 47, and CDR-H3 comprising the sequence as set forth in SEQ ID NO: 89; and
VL comprising the following 3 CDRs: CDR-L1 comprising the sequence as set forth in SEQ ID NO: 135, CDR-L2 comprising the sequence as set forth in SEQ ID NO: 172, and CDR-L3 comprising the sequence as set forth in SEQ ID NO: 206;
or
(17) VH comprising the following 3 CDRs: CDR-H1 comprising the sequence as set forth in SEQ ID NO: 12, CDR-H2 comprising the sequence as set forth in SEQ ID NO: 48, and CDR-H3 comprising the sequence as set forth in SEQ ID NO: 91; and
VL comprising the following 3 CDRs: CDR-L1 comprising the sequence as set forth in SEQ ID NO: 136, CDR-L2 comprising the sequence as set forth in SEQ ID NO: 171, and CDR-L3 comprising the sequence as set forth in SEQ ID NO: 207;
or
(18) VH comprising the following 3 CDRs: CDR-H1 comprising the sequence as set forth in SEQ ID NO: 11, CDR-H2 comprising the sequence as set forth in SEQ ID NO: 48, and CDR-H3 comprising the sequence as set forth in SEQ ID NO: 90; and
VL comprising the following 3 CDRs: CDR-L1 comprising the sequence as set forth in SEQ ID NO: 136, CDR-L2 comprising the sequence as set forth in SEQ ID NO: 171, and CDR-L3 comprising the sequence as set forth in SEQ ID NO: 207;
or
(19) VH comprising the following 3 CDRs: CDR-H1 comprising the sequence as set forth in SEQ ID NO: 13, CDR-H2 comprising the sequence as set forth in SEQ ID NO: 49, and CDR-H3 comprising the sequence as set forth in SEQ ID NO: 92; and
VL comprising the following 3 CDRs: CDR-L1 comprising the sequence as set forth in SEQ ID NO: 137, CDR-L2 comprising the sequence as set forth in SEQ ID NO: 173, and CDR-L3 comprising the sequence as set forth in SEQ ID NO: 208;
or
(20) VH comprising the following 3 CDRs: CDR-H1 comprising the sequence as set forth in SEQ ID NO: 13, CDR-H2 comprising the sequence as set forth in SEQ ID NO: 50, and CDR-H3 comprising the sequence as set forth in SEQ ID NO: 93; and
VL comprising the following 3 CDRs: CDR-L1 comprising the sequence as set forth in SEQ ID NO: 138, CDR-L2 comprising the sequence as set forth in SEQ ID NO: 170, and CDR-L3 comprising the sequence as set forth in SEQ ID NO: 209;
or
(21) VH comprising the following 3 CDRs: CDR-H1 comprising the sequence as set forth in SEQ ID NO: 13, CDR-H2 comprising the sequence as set forth in SEQ ID NO: 51, and CDR-H3 comprising the sequence as set forth in SEQ ID NO: 94; and
VL comprising the following 3 CDRs: CDR-L1 comprising the sequence as set forth in SEQ ID NO: 139, CDR-L2 comprising the sequence as set forth in SEQ ID NO: 170, and CDR-L3 comprising the sequence as set forth in SEQ ID NO: 210;
or
(22) VH comprising the following 3 CDRs: CDR-H1 comprising the sequence as set forth in SEQ ID NO: 15, CDR-H2 comprising the sequence as set forth in SEQ ID NO: 53, and CDR-H3 comprising the sequence as set forth in SEQ ID NO: 96; and
VL comprising the following 3 CDRs: CDR-L1 comprising the sequence as set forth in SEQ ID NO: 124, CDR-L2 comprising the sequence as set forth in SEQ ID NO: 164, and CDR-L3 comprising the sequence as set forth in SEQ ID NO: 212;
or
(23) VH comprising the following 3 CDRs: CDR-H1 comprising the sequence as set forth in SEQ ID NO: 16, CDR-H2 comprising the sequence as set forth in SEQ ID NO: 54, and CDR-H3 comprising the sequence as set forth in SEQ ID NO: 97; and
VL comprising the following 3 CDRs: CDR-L1 comprising the sequence as set forth in SEQ ID NO: 141, CDR-L2 comprising the sequence as set forth in SEQ ID NO: 172, and CDR-L3 comprising the sequence as set forth in SEQ ID NO: 213;
or
(24) VH comprising the following 3 CDRs: CDR-H1 comprising the sequence as set forth in SEQ ID NO: 17, CDR-H2 comprising the sequence as set forth in SEQ ID NO: 55, and CDR-H3 comprising the sequence as set forth in SEQ ID NO: 98; and
VL comprising the following 3 CDRs: CDR-L1 comprising the sequence as set forth in SEQ ID NO: 126, CDR-L2 comprising the sequence as set forth in SEQ ID NO: 174, and CDR-L3 comprising the sequence as set forth in SEQ ID NO: 214;
or
(25) VH comprising the following 3 CDRs: CDR-H1 comprising the sequence as set forth in SEQ ID NO: 18, CDR-H2 comprising the sequence as set forth in SEQ ID NO: 56, and CDR-H3 comprising the sequence as set forth in SEQ ID NO: 99; and
VL comprising the following 3 CDRs: CDR-L1 comprising the sequence as set forth in SEQ ID NO: 142, CDR-L2 comprising the sequence as set forth in SEQ ID NO: 176, and CDR-L3 comprising the sequence as set forth in SEQ ID NO: 215;
or
(26) VH comprising the following 3 CDRs: CDR-H1 comprising the sequence as set forth in SEQ ID NO: 19, CDR-H2 comprising the sequence as set forth in SEQ ID NO: 57, and CDR-H3 comprising the sequence as set forth in SEQ ID NO: 100; and
VL comprising the following 3 CDRs: CDR-L1 comprising the sequence as set forth in SEQ ID NO: 143, CDR-L2 comprising the sequence as set forth in SEQ ID NO: 177, and CDR-L3 comprising the sequence as set forth in SEQ ID NO: 216;
or
(27) VH comprising the following 3 CDRs: CDR-H1 comprising the sequence as set forth in SEQ ID NO: 20, CDR-H2 comprising the sequence as set forth in SEQ ID NO: 58, and CDR-H3 comprising the sequence as set forth in SEQ ID NO: 101; and
VL comprising the following 3 CDRs: CDR-L1 comprising the sequence as set forth in SEQ ID NO: 144, CDR-L2 comprising the sequence as set forth in SEQ ID NO: 176, and CDR-L3 comprising the sequence as set forth in SEQ ID NO: 215;
or
(28) VH comprising the following 3 CDRs: CDR-H1 comprising the sequence as set forth in SEQ ID NO: 21, CDR-H2 comprising the sequence as set forth in SEQ ID NO: 59, and CDR-H3 comprising the sequence as set forth in SEQ ID NO: 102; and
VL comprising the following 3 CDRs: CDR-L1 comprising the sequence as set forth in SEQ ID NO: 127, CDR-L2 comprising the sequence as set forth in SEQ ID NO: 168, and CDR-L3 comprising the sequence as set forth in SEQ ID NO: 217;
or
(29) VH comprising the following 3 CDRs: CDR-H1 comprising the sequence as set forth in SEQ ID NO: 21, CDR-H2 comprising the sequence as set forth in SEQ ID NO: 60, and CDR-H3 comprising the sequence as set forth in SEQ ID NO: 102; and
VL comprising the following 3 CDRs: CDR-L1 comprising the sequence as set forth in SEQ ID NO: 127, CDR-L2 comprising the sequence as set forth in SEQ ID NO: 168, and CDR-L3 comprising the sequence as set forth in SEQ ID NO: 217;
or
(30) VH comprising the following 3 CDRs: CDR-H1 comprising the sequence as set forth in SEQ ID NO: 13, CDR-H2 comprising the sequence as set forth in SEQ ID NO: 49, and CDR-H3 comprising the sequence as set forth in SEQ ID NO: 103; and
VL comprising the following 3 CDRs: CDR-L1 comprising the sequence as set forth in SEQ ID NO: 144, CDR-L2 comprising the sequence as set forth in SEQ ID NO: 169, and CDR-L3 comprising the sequence as set forth in SEQ ID NO: 218;
or
(31) VH comprising the following 3 CDRs: CDR-H1 comprising the sequence as set forth in SEQ ID NO: 22, CDR-H2 comprising the sequence as set forth in SEQ ID NO: 61, and CDR-H3 comprising the sequence as set forth in SEQ ID NO: 104; and
VL comprising the following 3 CDRs: CDR-L1 comprising the sequence as set forth in SEQ ID NO: 145, CDR-L2 comprising the sequence as set forth in SEQ ID NO: 178, and CDR-L3 comprising the sequence as set forth in SEQ ID NO: 219;
or
(32) VH comprising the following 3 CDRs: CDR-H1 comprising the sequence as set forth in SEQ ID NO: 17, CDR-H2 comprising the sequence as set forth in SEQ ID NO: 62, and CDR-H3 comprising the sequence as set forth in SEQ ID NO: 105; and
VL comprising the following 3 CDRs: CDR-L1 comprising the sequence as set forth in SEQ ID NO: 141, CDR-L2 comprising the sequence as set forth in SEQ ID NO: 172, and CDR-L3 comprising the sequence as set forth in SEQ ID NO: 220;
or
(33) VH comprising the following 3 CDRs: CDR-H1 comprising the sequence as set forth in SEQ ID NO: 17, CDR-H2 comprising the sequence as set forth in SEQ ID NO: 63, and CDR-H3 comprising the sequence as set forth in SEQ ID NO: 106; and
VL comprising the following 3 CDRs: CDR-L1 comprising the sequence as set forth in SEQ ID NO: 126, CDR-L2 comprising the sequence as set forth in SEQ ID NO: 179, and CDR-L3 comprising the sequence as set forth in SEQ ID NO: 221;
or
(34) VH comprising the following 3 CDRs: CDR-H1 comprising the sequence as set forth in SEQ ID NO: 23, CDR-H2 comprising the sequence as set forth in SEQ ID NO: 64, and CDR-H3 comprising the sequence as set forth in SEQ ID NO: 107; and
VL comprising the following 3 CDRs: CDR-L1 comprising the sequence as set forth in SEQ ID NO: 146, CDR-L2 comprising the sequence as set forth in SEQ ID NO: 180, and CDR-L3 comprising the sequence as set forth in SEQ ID NO: 222;
or
(35) VH comprising the following 3 CDRs: CDR-H1 comprising the sequence as set forth in SEQ ID NO: 24, CDR-H2 comprising the sequence as set forth in SEQ ID NO: 65, and CDR-H3 comprising the sequence as set forth in SEQ ID NO: 108; and
VL comprising the following 3 CDRs: CDR-L1 comprising the sequence as set forth in SEQ ID NO: 147, CDR-L2 comprising the sequence as set forth in SEQ ID NO: 181, and CDR-L3 comprising the sequence as set forth in SEQ ID NO: 223;
or
(36) VH comprising the following 3 CDRs: CDR-H1 comprising the sequence as set forth in SEQ ID NO: 25, CDR-H2 comprising the sequence as set forth in SEQ ID NO: 65, and CDR-H3 comprising the sequence as set forth in SEQ ID NO: 108; and
VL comprising the following 3 CDRs: CDR-L1 comprising the sequence as set forth in SEQ ID NO: 148, CDR-L2 comprising the sequence as set forth in SEQ ID NO: 181, and CDR-L3 comprising the sequence as set forth in SEQ ID NO: 224;
or
(37) VH comprising the following 3 CDRs: CDR-H1 comprising the sequence as set forth in SEQ ID NO: 26, CDR-H2 comprising the sequence as set forth in SEQ ID NO: 66, and CDR-H3 comprising the sequence as set forth in SEQ ID NO: 109; and
VL comprising the following 3 CDRs: CDR-L1 comprising the sequence as set forth in SEQ ID NO: 149, CDR-L2 comprising the sequence as set forth in SEQ ID NO: 182, and CDR-L3 comprising the sequence as set forth in SEQ ID NO: 225;
or
(38) VH comprising the following 3 CDRs: CDR-H1 comprising the sequence as set forth in SEQ ID NO: 27, CDR-H2 comprising the sequence as set forth in SEQ ID NO: 67, and CDR-H3 comprising the sequence as set forth in SEQ ID NO: 110; and
VL comprising the following 3 CDRs: CDR-L1 comprising the sequence as set forth in SEQ ID NO: 150, CDR-L2 comprising the sequence as set forth in SEQ ID NO: 183, and CDR-L3 comprising the sequence as set forth in SEQ ID NO: 226;
or
(39) VH comprising the following 3 CDRs: CDR-H1 comprising the sequence as set forth in SEQ ID NO: 28, CDR-H2 comprising the sequence as set forth in SEQ ID NO: 68, and CDR-H3 comprising the sequence as set forth in SEQ ID NO: 111; and
VL comprising the following 3 CDRs: CDR-L1 comprising the sequence as set forth in SEQ ID NO: 151, CDR-L2 comprising the sequence as set forth in SEQ ID NO: 184, and CDR-L3 comprising the sequence as set forth in SEQ ID NO: 227;
or
(40) VH comprising the following 3 CDRs: CDR-H1 comprising the sequence as set forth in SEQ ID NO: 29, CDR-H2 comprising the sequence as set forth in SEQ ID NO: 69, and CDR-H3 comprising the sequence as set forth in SEQ ID NO: 112; and
VL comprising the following 3 CDRs: CDR-L1 comprising the sequence as set forth in SEQ ID NO: 152, CDR-L2 comprising the sequence as set forth in SEQ ID NO: 185, and CDR-L3 comprising the sequence as set forth in SEQ ID NO: 222;
or
(41) VH comprising the following 3 CDRs: CDR-H1 comprising the sequence as set forth in SEQ ID NO: 30, CDR-H2 comprising the sequence as set forth in SEQ ID NO: 70, and CDR-H3 comprising the sequence as set forth in SEQ ID NO: 113; and
VL comprising the following 3 CDRs: CDR-L1 comprising the sequence as set forth in SEQ ID NO: 153, CDR-L2 comprising the sequence as set forth in SEQ ID NO: 186, and CDR-L3 comprising the sequence as set forth in SEQ ID NO: 228;
or
(42) VH comprising the following 3 CDRs: CDR-H1 comprising the sequence as set forth in SEQ ID NO: 31, CDR-H2 comprising the sequence as set forth in SEQ ID NO: 71, and CDR-H3 comprising the sequence as set forth in SEQ ID NO: 114; and
VL comprising the following 3 CDRs: CDR-L1 comprising the sequence as set forth in SEQ ID NO: 154, CDR-L2 comprising the sequence as set forth in SEQ ID NO: 187, and CDR-L3 comprising the sequence as set forth in SEQ ID NO: 229;
or
(43) VH comprising the following 3 CDRs: CDR-H1 comprising the sequence as set forth in SEQ ID NO: 32, CDR-H2 comprising the sequence as set forth in SEQ ID NO: 72, and CDR-H3 comprising the sequence as set forth in SEQ ID NO: 115; and
VL comprising the following 3 CDRs: CDR-L1 comprising the sequence as set forth in SEQ ID NO: 155, CDR-L2 comprising the sequence as set forth in SEQ ID NO: 188, and CDR-L3 comprising the sequence as set forth in SEQ ID NO: 230;
or
(44) VH comprising the following 3 CDRs: CDR-H1 comprising the sequence as set forth in SEQ ID NO: 33, CDR-H2 comprising the sequence as set forth in SEQ ID NO: 73, and CDR-H3 comprising the sequence as set forth in SEQ ID NO: 116; and
VL comprising the following 3 CDRs: CDR-L1 comprising the sequence as set forth in SEQ ID NO: 156, CDR-L2 comprising the sequence as set forth in SEQ ID NO: 189, and CDR-L3 comprising the sequence as set forth in SEQ ID NO: 231;
or
(45) VH comprising the following 3 CDRs: CDR-H1 comprising the sequence as set forth in SEQ ID NO: 34, CDR-H2 comprising the sequence as set forth in SEQ ID NO: 74, and CDR-H3 comprising the sequence as set forth in SEQ ID NO: 117; and
VL comprising the following 3 CDRs: CDR-L1 comprising the sequence as set forth in SEQ ID NO: 157, CDR-L2 comprising the sequence as set forth in SEQ ID NO: 190, and CDR-L3 comprising the sequence as set forth in SEQ ID NO: 232;
or
(46) VH comprising the following 3 CDRs: CDR-H1 comprising the sequence as set forth in SEQ ID NO: 35, CDR-H2 comprising the sequence as set forth in SEQ ID NO: 75, and CDR-H3 comprising the sequence as set forth in SEQ ID NO: 118; and
VL comprising the following 3 CDRs: CDR-L1 comprising the sequence as set forth in SEQ ID NO: 158, CDR-L2 comprising the sequence as set forth in SEQ ID NO: 191, and CDR-L3 comprising the sequence as set forth in SEQ ID NO: 233;
or
(47) VH comprising the following 3 CDRs: CDR-H1 comprising the sequence as set forth in SEQ ID NO: 26, CDR-H2 comprising the sequence as set forth in SEQ ID NO: 76, and CDR-H3 comprising the sequence as set forth in SEQ ID NO: 119; and
VL comprising the following 3 CDRs: CDR-L1 comprising the sequence as set forth in SEQ ID NO: 159, CDR-L2 comprising the sequence as set forth in SEQ ID NO: 192, and CDR-L3 comprising the sequence as set forth in SEQ ID NO: 234;
or
(48) VH comprising the following 3 CDRs: CDR-H1 comprising the sequence as set forth in SEQ ID NO: 24, CDR-H2 comprising the sequence as set forth in SEQ ID NO: 77, and CDR-H3 comprising the sequence as set forth in SEQ ID NO: 120; and
VL comprising the following 3 CDRs: CDR-L1 comprising the sequence as set forth in SEQ ID NO: 160, CDR-L2 comprising the sequence as set forth in SEQ ID NO: 193, and CDR-L3 comprising the sequence as set forth in SEQ ID NO: 235;
or
(49) VH comprising the following 3 CDRs: CDR-H1 comprising the sequence as set forth in SEQ ID NO: 36, CDR-H2 comprising the sequence as set forth in SEQ ID NO: 78, and CDR-H3 comprising the sequence as set forth in SEQ ID NO: 121; and
VL comprising the following 3 CDRs: CDR-L1 comprising the sequence as set forth in SEQ ID NO: 161, CDR-L2 comprising the sequence as set forth in SEQ ID NO: 194, and CDR-L3 comprising the sequence as set forth in SEQ ID NO: 236;
or
(50) VH comprising the following 3 CDRs: CDR-H1 comprising the sequence as set forth in SEQ ID NO: 32, CDR-H2 comprising the sequence as set forth in SEQ ID NO: 79, and CDR-H3 comprising the sequence as set forth in SEQ ID NO: 122; and
VL comprising the following 3 CDRs: CDR-L1 comprising the sequence as set forth in SEQ ID NO: 162, CDR-L2 comprising the sequence as set forth in SEQ ID NO: 195, and CDR-L3 comprising the sequence as set forth in SEQ ID NO: 237;
or
(51) VH comprising the following 3 CDRs: CDR-H1 comprising the sequence as set forth in SEQ ID NO: 37, CDR-H2 comprising the sequence as set forth in SEQ ID NO: 79, and CDR-H3 comprising the sequence as set forth in SEQ ID NO: 123; and
VL comprising the following 3 CDRs: CDR-L1 comprising the sequence as set forth in SEQ ID NO: 163, CDR-L2 comprising the sequence as set forth in SEQ ID NO: 196, and CDR-L3 comprising the sequence as set forth in SEQ ID NO: 238; preferably:
the antibody or the antigen-binding fragment thereof comprises the following heavy chain variable region (VH) and light chain variable region (VL), wherein CDRs are defined according to the Kabat numbering system:
(1) VH comprising the following 3 CDRs: CDR-H1 comprising the sequence as set forth in SEQ ID NO: 392, CDR-H2 comprising the sequence as set forth in SEQ ID NO: 393, and CDR-H3 comprising the sequence as set forth in SEQ ID NO: 394; and
VL comprising the following 3 CDRs: CDR-L1 comprising the sequence as set forth in SEQ ID NO: 395, CDR-L2 comprising the sequence as set forth in SEQ ID NO: 396, and CDR-L3 comprising the sequence as set forth in SEQ ID NO: 207;
or
(2) VH comprising the following 3 CDRs: CDR-H1 comprising the sequence as set forth in SEQ ID NO: 377, CDR-H2 comprising the sequence as set forth in SEQ ID NO: 378, and CDR-H3 comprising the sequence as set forth in SEQ ID NO: 379; and
VL comprising the following 3 CDRs: CDR-L1 comprising the sequence as set forth in SEQ ID NO: 380, CDR-L2 comprising the sequence as set forth in SEQ ID NO: 381, and CDR-L3 comprising the sequence as set forth in SEQ ID NO: 211;
or
(3) VH comprising the following 3 CDRs: CDR-H1 comprising the sequence as set forth in SEQ ID NO: 362, CDR-H2 comprising the sequence as set forth in SEQ ID NO: 363, and CDR-H3 comprising the sequence as set forth in SEQ ID NO: 364; and
VL comprising the following 3 CDRs: CDR-L1 comprising the sequence as set forth in SEQ ID NO: 365, CDR-L2 comprising the sequence as set forth in SEQ ID NO: 366, and CDR-L3 comprising the sequence as set forth in SEQ ID NO: 199;
or, the CDRs are defined according to the AbM numbering system:
(1) VH comprising the following 3 CDRs: CDR-H1 comprising the sequence as set forth in SEQ ID NO: 397, CDR-H2 comprising the sequence as set forth in SEQ ID NO: 398, and CDR-H3 comprising the sequence as set forth in SEQ ID NO: 394; and
VL comprising the following 3 CDRs: CDR-L1 comprising the sequence as set forth in SEQ ID NO: 395, CDR-L2 comprising the sequence as set forth in SEQ ID NO: 396, and CDR-L3 comprising the sequence as set forth in SEQ ID NO: 207;
or
(2) VH comprising the following 3 CDRs: CDR-H1 comprising the sequence as set forth in SEQ ID NO: 382, CDR-H2 comprising the sequence as set forth in SEQ ID NO: 383, and CDR-H3 comprising the sequence as set forth in SEQ ID NO: 379; and
VL comprising the following 3 CDRs: CDR-L1 comprising the sequence as set forth in SEQ ID NO: 380, CDR-L2 comprising the sequence as set forth in SEQ ID NO: 381, and CDR-L3 comprising the sequence as set forth in SEQ ID NO: 211;
or
(3) VH comprising the following 3 CDRs: CDR-H1 comprising the sequence as set forth in SEQ ID NO: 367, CDR-H2 comprising the sequence as set forth in SEQ ID NO: 368, and CDR-H3 comprising the sequence as set forth in SEQ ID NO: 364; and
VL comprising the following 3 CDRs: CDR-L1 comprising the sequence as set forth in SEQ ID NO: 365, CDR-L2 comprising the sequence as set forth in SEQ ID NO: 366, and CDR-L3 comprising the sequence as set forth in SEQ ID NO: 199;
or, the CDRs are defined according to the Chothia numbering system:
(1) VH comprising the following 3 CDRs: CDR-H1 comprising the sequence as set forth in SEQ ID NO: 399, CDR-H2 comprising the sequence as set forth in SEQ ID NO: 400, and CDR-H3 comprising the sequence as set forth in SEQ ID NO: 394; and
VL comprising the following 3 CDRs: CDR-L1 comprising the sequence as set forth in SEQ ID NO: 395, CDR-L2 comprising the sequence as set forth in SEQ ID NO: 396, and CDR-L3 comprising the sequence as set forth in SEQ ID NO: 207;
or
(2) VH comprising the following 3 CDRs: CDR-H1 comprising the sequence as set forth in SEQ ID NO: 384, CDR-H2 comprising the sequence as set forth in SEQ ID NO: 385, and CDR-H3 comprising the sequence as set forth in SEQ ID NO: 379; and
VL comprising the following 3 CDRs: CDR-L1 comprising the sequence as set forth in SEQ ID NO: 380, CDR-L2 comprising the sequence as set forth in SEQ ID NO: 381, and CDR-L3 comprising the sequence as set forth in SEQ ID NO: 211;
or
(3) VH comprising the following 3 CDRs: CDR-H1 comprising the sequence as set forth in SEQ ID NO: 369, CDR-H2 comprising the sequence as set forth in SEQ ID NO: 370, and CDR-H3 comprising the sequence as set forth in SEQ ID NO: 364; and
VL comprising the following 3 CDRs: CDR-L1 comprising the sequence as set forth in SEQ ID NO: 365, CDR-L2 comprising the sequence as set forth in SEQ ID NO: 366, and CDR-L3 comprising the sequence as set forth in SEQ ID NO: 199;
or, the CDRs are defined according to the Contact numbering system:
(1) VH comprising the following 3 CDRs: CDR-H1 comprising the sequence as set forth in SEQ ID NO: 401, CDR-H2 comprising the sequence as set forth in SEQ ID NO: 402, and CDR-H3 comprising the sequence as set forth in SEQ ID NO: 403; and
VL comprising the following 3 CDRs: CDR-L1 comprising the sequence as set forth in SEQ ID NO: 404, CDR-L2 comprising the sequence as set forth in SEQ ID NO: 405, and CDR-L3 comprising the sequence as set forth in SEQ ID NO: 406;
or
(2) VH comprising the following 3 CDRs: CDR-H1 comprising the sequence as set forth in SEQ ID NO: 386, CDR-H2 comprising the sequence as set forth in SEQ ID NO: 387, and CDR-H3 comprising the sequence as set forth in SEQ ID NO: 388; and
VL comprising the following 3 CDRs: CDR-L1 comprising the sequence as set forth in SEQ ID NO: 389, CDR-L2 comprising the sequence as set forth in SEQ ID NO: 390, and CDR-L3 comprising the sequence as set forth in SEQ ID NO: 391;
or
(3) VH comprising the following 3 CDRs: CDR-H1 comprising the sequence as set forth in SEQ ID NO: 371, CDR-H2 comprising the sequence as set forth in SEQ ID NO: 372, and CDR-H3 comprising the sequence as set forth in SEQ ID NO: 373; and
VL comprising the following 3 CDRs: CDR-L1 comprising the sequence as set forth in SEQ ID NO: 374, CDR-L2 comprising the sequence as set forth in SEQ ID NO: 375, and CDR-L3 comprising the sequence as set forth in SEQ ID NO: 376.

2. The antibody or the antigen-binding fragment thereof that specifically binds to CDCP1 according to claim 1, wherein,
the antibody or the antigen-binding fragment thereof comprises a heavy chain variable region (VH) and a light chain variable region (VL) selected from any one of the following groups:
(1) VH of the sequence as set forth in SEQ ID NO: 239 and VL of the sequence as set forth in SEQ ID NO: 289;
(2) VH of the sequence as set forth in SEQ ID NO: 239 and VL of the sequence as set forth in SEQ ID NO: 290;
(3) VH of the sequence as set forth in SEQ ID NO: 240 and VL of the sequence as set forth in SEQ ID NO: 291;
(4) VH of the sequence as set forth in SEQ ID NO: 241 and VL of the sequence as set forth in SEQ ID NO: 292;
(5) VH of the sequence as set forth in SEQ ID NO: 242 and VL of the sequence as set forth in SEQ ID NO: 293;
(6) VH of the sequence as set forth in SEQ ID NO: 243 and VL of the sequence as set forth in SEQ ID NO: 294;
(7) VH of the sequence as set forth in SEQ ID NO: 244 and VL of the sequence as set forth in SEQ ID NO: 295;
(8) VH of the sequence as set forth in SEQ ID NO: 245 and VL of the sequence as set forth in SEQ ID NO: 296;
(9) VH of the sequence as set forth in SEQ ID NO: 246 and VL of the sequence as set forth in SEQ ID NO: 297;
(10) VH of the sequence as set forth in SEQ ID NO: 247 and VL of the sequence as set forth in SEQ ID NO: 298;
(11) VH of the sequence as set forth in SEQ ID NO: 248 and VL of the sequence as set forth in SEQ ID NO: 296;
(12) VH of the sequence as set forth in SEQ ID NO: 249 and VL of the sequence as set forth in SEQ ID NO: 299;
(13) VH of the sequence as set forth in SEQ ID NO: 250 and VL of the sequence as set forth in SEQ ID NO: 300;
(14) VH of the sequence as set forth in SEQ ID NO: 251 and VL of the sequence as set forth in SEQ ID NO: 301;
(15) VH of the sequence as set forth in SEQ ID NO: 252 and VL of the sequence as set forth in SEQ ID NO: 302;
(16) VH of the sequence as set forth in SEQ ID NO: 253 and VL of the sequence as set forth in SEQ ID NO: 303;
(17) VH of the sequence as set forth in SEQ ID NO: 254 and VL of the sequence as set forth in SEQ ID NO: 304;
(18) VH of the sequence as set forth in SEQ ID NO: 255 and VL of the sequence as set forth in SEQ ID NO: 305;
(19) VH of the sequence as set forth in SEQ ID NO: 256 and VL of the sequence as set forth in SEQ ID NO: 306;
(20) VH of the sequence as set forth in SEQ ID NO: 257 and VL of the sequence as set forth in SEQ ID NO: 307;
(21) VH of the sequence as set forth in SEQ ID NO: 258 and VL of the sequence as set forth in SEQ ID NO: 308;
(22) VH of the sequence as set forth in SEQ ID NO: 259 and VL of the sequence as set forth in SEQ ID NO: 309;
(23) VH of the sequence as set forth in SEQ ID NO: 260 and VL of the sequence as set forth in SEQ ID NO: 310;
(24) VH of the sequence as set forth in SEQ ID NO: 261 and VL of the sequence as set forth in SEQ ID NO: 311;
(25) VH of the sequence as set forth in SEQ ID NO: 262 and VL of the sequence as set forth in SEQ ID NO: 312;
(26) VH of the sequence as set forth in SEQ ID NO: 263 and VL of the sequence as set forth in SEQ ID NO: 313;
(27) VH of the sequence as set forth in SEQ ID NO: 264 and VL of the sequence as set forth in SEQ ID NO: 314;
(28) VH of the sequence as set forth in SEQ ID NO: 265 and VL of the sequence as set forth in SEQ ID NO: 315;
(29) VH of the sequence as set forth in SEQ ID NO: 266 and VL of the sequence as set forth in SEQ ID NO: 316;
(30) VH of the sequence as set forth in SEQ ID NO: 267 and VL of the sequence as set forth in SEQ ID NO: 317;
(31) VH of the sequence as set forth in SEQ ID NO: 268 and VL of the sequence as set forth in SEQ ID NO: 318;
(32) VH of the sequence as set forth in SEQ ID NO: 269 and VL of the sequence as set forth in SEQ ID NO: 319;
(33) VH of the sequence as set forth in SEQ ID NO: 270 and VL of the sequence as set forth in SEQ ID NO: 320;
(34) VH of the sequence as set forth in SEQ ID NO: 271 and VL of the sequence as set forth in SEQ ID NO: 321;
(35) VH of the sequence as set forth in SEQ ID NO: 272 and VL of the sequence as set forth in SEQ ID NO: 322;
(36) VH of the sequence as set forth in SEQ ID NO: 273 and VL of the sequence as set forth in SEQ ID NO: 323;
(37) VH of the sequence as set forth in SEQ ID NO: 274 and VL of the sequence as set forth in SEQ ID NO: 324;
(38) VH of the sequence as set forth in SEQ ID NO: 275 and VL of the sequence as set forth in SEQ ID NO: 325;
(39) VH of the sequence as set forth in SEQ ID NO: 276 and VL of the sequence as set forth in SEQ ID NO: 326;
(40) VH of the sequence as set forth in SEQ ID NO: 277 and VL of the sequence as set forth in SEQ ID NO: 327;
(41) VH of the sequence as set forth in SEQ ID NO: 278 and VL of the sequence as set forth in SEQ ID NO: 328;
(42) VH of the sequence as set forth in SEQ ID NO: 279 and VL of the sequence as set forth in SEQ ID NO: 329;
(43) VH of the sequence as set forth in SEQ ID NO: 280 and VL of the sequence as set forth in SEQ ID NO: 330;
(44) VH of the sequence as set forth in SEQ ID NO: 281 and VL of the sequence as set forth in SEQ ID NO: 331;
(45) VH of the sequence as set forth in SEQ ID NO: 282 and VL of the sequence as set forth in SEQ ID NO: 332;
(46) VH of the sequence as set forth in SEQ ID NO: 283 and VL of the sequence as set forth in SEQ ID NO: 333;
(47) VH of the sequence as set forth in SEQ ID NO: 284 and VL of the sequence as set forth in SEQ ID NO: 334;
(48) VH of the sequence as set forth in SEQ ID NO: 285 and VL of the sequence as set forth in SEQ ID NO: 335;
(49) VH of the sequence as set forth in SEQ ID NO: 286 and VL of the sequence as set forth in SEQ ID NO: 336;
(50) VH of the sequence as set forth in SEQ ID NO: 287 and VL of the sequence as set forth in SEQ ID NO: 337;
(51) VH of the sequence as set forth in SEQ ID NO: 288 and VL of the sequence as set forth in SEQ ID NO: 338;
(52) VH of the sequence as set forth in SEQ ID NO: 339 and VL of the sequence as set forth in SEQ ID NO: 351;
(53) VH of the sequence as set forth in SEQ ID NO: 339 and VL of the sequence as set forth in SEQ ID NO: 352;
(54) VH of the sequence as set forth in SEQ ID NO: 339 and VL of the sequence as set forth in SEQ ID NO: 353;
(55) VH of the sequence as set forth in SEQ ID NO: 340 and VL of the sequence as set forth in SEQ ID NO: 351;
(56) VH of the sequence as set forth in SEQ ID NO: 340 and VL of the sequence as set forth in SEQ ID NO: 352;
(57) VH of the sequence as set forth in SEQ ID NO: 340 and VL of the sequence as set forth in SEQ ID NO: 353;
(58) VH of the sequence as set forth in SEQ ID NO: 341 and VL of the sequence as set forth in SEQ ID NO: 351;
(59) VH of the sequence as set forth in SEQ ID NO: 341 and VL of the sequence as set forth in SEQ ID NO: 352;
(60) VH of the sequence as set forth in SEQ ID NO: 341 and VL of the sequence as set forth in SEQ ID NO: 353;
(61) VH of the sequence as set forth in SEQ ID NO: 342 and VL of the sequence as set forth in SEQ ID NO: 351;
(62) VH of the sequence as set forth in SEQ ID NO: 342 and VL of the sequence as set forth in SEQ ID NO: 352;
(63) VH of the sequence as set forth in SEQ ID NO: 342 and VL of the sequence as set forth in SEQ ID NO: 353;
(64) VH of the sequence as set forth in SEQ ID NO: 343 and VL of the sequence as set forth in SEQ ID NO: 354;
(65) VH of the sequence as set forth in SEQ ID NO: 343 and VL of the sequence as set forth in SEQ ID NO: 355;
(66) VH of the sequence as set forth in SEQ ID NO: 343 and VL of the sequence as set forth in SEQ ID NO: 356;
(67) VH of the sequence as set forth in SEQ ID NO: 344 and VL of the sequence as set forth in SEQ ID NO: 354;
(68) VH of the sequence as set forth in SEQ ID NO: 344 and VL of the sequence as set forth in SEQ ID NO: 355;
(69) VH of the sequence as set forth in SEQ ID NO: 344 and VL of the sequence as set forth in SEQ ID NO: 356;
(70) VH of the sequence as set forth in SEQ ID NO: 345 and VL of the sequence as set forth in SEQ ID NO: 354;
(71) VH of the sequence as set forth in SEQ ID NO: 345 and VL of the sequence as set forth in SEQ ID NO: 355;
(72) VH of the sequence as set forth in SEQ ID NO: 345 and VL of the sequence as set forth in SEQ ID NO: 356;
(73) VH of the sequence as set forth in SEQ ID NO: 346 and VL of the sequence as set forth in SEQ ID NO: 354;
(74) VH of the sequence as set forth in SEQ ID NO: 346 and VL of the sequence as set forth in SEQ ID NO: 355;
(75) VH of the sequence as set forth in SEQ ID NO: 346 and VL of the sequence as set forth in SEQ ID NO: 356;
(76) VH of the sequence as set forth in SEQ ID NO: 347 and VL of the sequence as set forth in SEQ ID NO: 357;
(77) VH of the sequence as set forth in SEQ ID NO: 347 and VL of the sequence as set forth in SEQ ID NO: 358;
(78) VH of the sequence as set forth in SEQ ID NO: 347 and VL of the sequence as set forth in SEQ ID NO: 359;
(79) VH of the sequence as set forth in SEQ ID NO: 348 and VL of the sequence as set forth in SEQ ID NO: 357;
(80) VH of the sequence as set forth in SEQ ID NO: 348 and VL of the sequence as set forth in SEQ ID NO: 358;
(81) VH of the sequence as set forth in SEQ ID NO: 348 and VL of the sequence as set forth in SEQ ID NO: 359;
(82) VH of the sequence as set forth in SEQ ID NO: 349 and VL of the sequence as set forth in SEQ ID NO: 357;
(83) VH of the sequence as set forth in SEQ ID NO: 349 and VL of the sequence as set forth in SEQ ID NO: 358;
(84) VH of the sequence as set forth in SEQ ID NO: 349 and VL of the sequence as set forth in SEQ ID NO: 359;
(85) VH of the sequence as set forth in SEQ ID NO: 350 and VL of the sequence as set forth in SEQ ID NO: 357;
(86) VH of the sequence as set forth in SEQ ID NO: 350 and VL of the sequence as set forth in SEQ ID NO: 358;
(87) VH of the sequence as set forth in SEQ ID NO: 350 and VL of the sequence as set forth in SEQ ID NO: 359;
optionally, for the VH and the VL when compared to any one of groups from (1) to (87), the VH has at least 70%, at least 80%, at least 85%, at least 90%, at least 91%, at least 92%, at least 93%, at least 94%, at least 95%, at least 96%, at least 97%, at least 98%, at least 99%, or 100% sequence identity with VH in any one of groups from (1) to (87); and, the VL has at least 70%, at least 80%, at least 85%, at least 90%, at least 91%, at least 92%, at least 93%, at least 94%, at least 95%, at least 96%, at least 97%, at least 98%, at least 99%, or 100% sequence identity with VL in any one of groups from (1) to (87).

3. The antibody or the antigen-binding fragment thereof that specifically binds to CDCP1 according to claim 1 or 2, wherein the antibody or the antigen-binding fragment thereof is selected from an Fab fragment, an Fab' fragment, an F(ab)'₂ fragment, a single chain antibody, a disulfide-stabilized Fv protein (dsFv);
preferably, the single chain antibody is selected from scFv, di-scFv or (scFv)₂.

4. The antibody or the antigen-binding fragment thereof that specifically binds to CDCP1 according to any one of claims 1 to 3, wherein, the antibody or the antigen-binding fragment thereof is a murine antibody, a chimeric antibody, a humanized antibody, or an antibody derived from other species (e.g., rabbits, camels, or sharks).

5. The antibody or the antigen-binding fragment thereof that specifically binds to CDCP1 according to any one of claims 1 to 4, wherein,
the antibody or the antigen-binding fragment thereof further comprises a heavy chain constant region (CH) and a light chain constant region (CL);
preferably, the heavy chain constant region is selected from the heavy chain constant region or a variant thereof of IgG, IgM, IgE, IgD or IgA; and/or,
the light chain constant region is selected from a light chain constant region or a variant thereof of kappa or lambda;
the variant has a substitution, deletion, or addition of one or more amino acid(s) (e.g., a substitution, deletion, or addition of up to 20, up to 15, up to 10, or up to 5 amino acids; e.g., a substitution, deletion, or addition of 1, 2, 3, 4, or 5 amino acid(s)) compared to the wild-type sequence derived from;
more preferably, the antibody or the antigen-binding fragment thereof comprises a heavy chain constant region and a light chain constant region selected from the following:
(1) the heavy chain constant region of human IgG1;
(2) the light chain constant region of human IgG1;
further more preferably, the heavy chain constant region (CH) of the antibody or the antigen-binding fragment thereof comprises the amino acid sequence as set forth in SEQ ID NO: 360; and/or,
the light chain constant region is the kappa light chain constant region of human IgG1; the light chain constant region (CL) comprises the amino acid as set forth in SEQ ID NO: 361.

6. The antibody or the antigen-binding fragment thereof that specifically binds to CDCP1 according to any one of claims 1 to 5, wherein,
the antibody or the antigen-binding fragment thereof:
binds to CDCP1 (e.g., human CDCP1) at a KD of less than about 500 nM, such as less than about 100 nM, 50 nM, 40 nM, 40 nM, 20 nM, 10 nM, 1 nM, 0.1 nM, 0.01 nM or less; and/or;
binds to a tumor cell expressing CDCP1 at an EC₅₀ of less than about 500 nM, such as less than about 100 nM, 10 nM, 1 nM, 0.9 nM, 0.8 nM, 0.7 nM, 0.6 nM, 0.5 nM, 0.4 nM, 0.3 nM, 0.2 nM, 0.1 nM, 0.01 nM or less; preferably, the EC₅₀ is measured by flow cytometry or cell competitive ELISA;
preferably, the antibody or the antigen-binding fragment thereof can or cannot induce endocytosis; and/or,
the antibody or the antigen-binding fragment thereof cannot promote tumor cell migration; and/or,
the antibody or the antigen-binding fragment thereof can inhibit tumor metastasis.

7. A multispecific antibody, comprising: the antibody or the antigen-binding fragment thereof according to any one of claims 1 to 6; preferably, the multispecific antibody is a bispecific antibody or a trispecific antibody or a tetraspecific antibody.

8. An isolated nucleic acid molecule encoding the antibody or the antigen-binding fragment thereof according to any one of claims 1 to 6 or the multispecific antibody according to claim 7.

9. A vector comprising the nucleic acid molecule according to claim 8.

10. A host cell comprising the nucleic acid molecule according to claim 8 or the vector according to claim 9.

11. A method of preparing an antibody or an antigen-binding fragment thereof or a multispecific antibody, that specifically binds to CDCP1, wherein the method comprises culturing the host cell according to claim 10 under a condition that allows expression of the antibody or the antigen-binding fragment thereof or the multispecific antibody, and recovering the antibody or the antigen-binding fragment thereof or the multispecific antibody from the cultured host cell culture.

12. A conjugate or a pharmaceutically acceptable salt thereof, wherein, the conjugate comprises the antibody or the antigen-binding fragment thereof according to any one of claims 1 to 6, or the multispecific antibody according to claim 7, and a coupling moiety; the antibody or the antigen-binding fragment thereof, or the multispecific antibody is linked to the coupling moiety directly or by a linker;
preferably, the coupling moiety is selected from: a detectable label (e.g., radioisotope, fluorescent substance, luminescent substance, colored substances or enzyme) or therapeutic agent (e.g., nuclide, immune agonist, immunosuppressant, cytokine, toxin, and other active substance that inhibits tumor cell growth, promotes tumor cell apoptosis or necrosis).

13. The conjugate according to claim 12, the structure of the conjugate is as shown in formula (I):
A-(L-D)ₚ (I),
L is present or absent;
wherein,
A is the antibody or the antigen-binding fragment thereof according to any one of claims 1 to 6, or the multispecific antibody according to claim 7;
L is the linker, preferably the linker is a vc linker, the structure of which is: 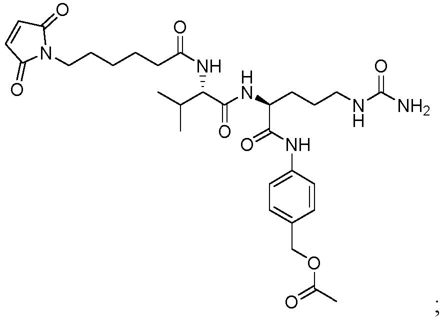
D is the coupling moiety;
preferably, the coupling moiety is an immune activator comprising a TLR, STING, ONDs, CpG, LPS, SEB, SEA, or SEC activator;
preferably, the coupling moiety is a cytokine comprising IL-6, IL-15, IL-12, IL-1b or IL-23;
preferably, the coupling moiety is a toxin comprising a cytotoxic agent; more preferably, the cytotoxic agent is selected from camptothecins (e.g., SN-38), maytansines (e.g., maytansine DM1/4), pyrrolozobenzodiazepines (PBDs), or auristatin (e.g., Monomethyl auristatin E/F);
the value of p is more than or equal to 0; preferably, p is 0-8.

14. A pharmaceutical composition comprising the antibody or the antigen-binding fragment thereof according to any one of claims 1 to 6, or the multispecific antibody according to claim 7, or the conjugate according to claim 12 or 13, and a pharmaceutically acceptable carrier and/or excipient;
preferably, the pharmaceutical composition further comprises an additional pharmaceutically active agent;
more preferably, the additional pharmaceutically active agent is a drug having anti-tumor activity, such as a chemotherapeutic drug (e.g., a platinum-based drug), a small molecule inhibitor; and/or,
the additional pharmaceutically active agent is an immunotherapy-related drug, such as an immune checkpoint inhibitor (e.g., a PD-1, PD-L1 or CTLA-4 inhibitor), an immunity-enhancing drug (e.g., interferon, interleukin) or an immune system activator (e.g., a TLR, ONDs, CpG, LPS, SEB, SEA, STING or SEC activator); and/or,
the additional pharmaceutically active agent is an oncolytic virus, an immune cell, or an engineered immune cell; and/or,
the additional pharmaceutically active agent is a radioactive substance or a chromogenic agent.

15. A kit comprising the antibody or the antigen-binding fragment thereof according to any one of claims 1 to 6, or the multispecific antibody according to claim 7, or the conjugate according to claim 12 or 13, or the pharmaceutical composition according to claim 14.

16. A use of the antibody or the antigen-binding fragment thereof according to any one of claims 1 to 6, or the multispecific antibody according to claim 7, or the conjugate according to claim 12 or 13, or the pharmaceutical composition according to claim 14 in the preparation of a medicament for the prevention and/or treatment and/or adjuvant treatment and/or neoadjuvant treatment of a disease related to CDCP1.

17. The use according to claim 16, wherein the disease related to CDCP1 is a tumor;
preferably, the tumor is selected from a solid tumor or a hematological tumor;
more preferably, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; the hematological tumor is selected from lymphoma, myeloma (e.g., multiple myeloma) or leukemia.

18. The use according to claim 16 or 17, wherein the antibody or the antigen-binding fragment thereof, the conjugate, the multispecific antibody or the pharmaceutical composition is administered separately, in combination, simultaneously or sequentially with an additional pharmaceutically active agent;
preferably, the additional pharmaceutically active agent is a drug having anti-tumor activity (e.g., a platinum-based drug, a small molecule inhibitor); or, the additional pharmaceutically active agent is an immunity-enhancing drug (e.g. interferon, interleukin); alternatively, the additional pharmaceutically active agent is an immune system activator (e.g., a TLR, ONDs, CpG, LPS, SEB, SEA, STING or SEC activator); or, the additional pharmaceutically active agent is an immune checkpoint inhibitor (e.g., a PD-1, PD-L1 or CTLA-4 inhibitor); or, the additional pharmaceutically active agent is an oncolytic virus, an immune cell, or an engineered immune cell.

19. A method of prevention and/or treatment and/or adjuvant treatment and/or neoadjuvant treatment of a disease related to CDCP1 in a subject, wherein the method comprises administering an effective amount of the antibody or the antigen-binding fragment thereof according to any one of claims 1 to 6, or the multispecific antibody according to claim 7, or the conjugate according to claim 12 or 13, or the pharmaceutical composition according to claim 14 to a subject in need thereof.

20. The method according to claim 19, further comprising administering to the subject a second therapy selected from a surgery, a chemotherapy, a radiotherapy, an immunotherapy, a gene therapy, a DNA therapy, a RNA therapy, a nanotherapy, a viral therapy, an adjuvant therapy, a cell therapy, and any combination thereof;
optionally, the second therapy can be applied separately or in combination with the method according to claim 19.

21. The method according to claim 19 or 20, wherein the disease related to CDCP1 is a tumor; preferably, the tumor is selected from a solid tumor or a hematological tumor;
more preferably, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; the hematological tumor is selected from lymphoma, myeloma (e.g., multiple myeloma) or leukemia.

22. A method of detecting the presence or level of CDCP1 in a sample, wherein the method comprises contacting the sample with the antibody or the antigen-binding fragment thereof according to any one of claims 1 to 6, or the multispecific antibody according to claim 7, or the conjugate according to claim 12 or 13, or the pharmaceutical composition according to claim 14 under a condition that allows formation of a complex between the antibody or the antigen-binding fragment thereof, the multispecific antibody, the conjugate, or the pharmaceutical composition and CDCP1, and detecting the formation of the complex.

23. A use of the antibody or the antigen-binding fragment thereof according to any one of claims 1 to 6, or the multispecific antibody according to claim 7, or the conjugate according to claim 12 or 13, or the pharmaceutical composition according to claim 14 in preparation of a diagnostic kit for diagnosis or differential diagnosis of a disease related to CDCP1; preferably, the disease related to CDCP1 is a tumor; more preferably, the tumor is selected from a solid tumor or a hematological tumor; further more preferably, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; the hematological tumor is selected from lymphoma, myeloma (e.g., multiple myeloma) or leukemia.

24. The antibody or the antigen-binding fragment thereof according to any one of claims 1 to 6, or the multispecific antibody according to claim 7, or the conjugate according to claim 12 or 13, or the pharmaceutical composition according to claim 14, for use in the prevention and/or treatment and/or adjuvant treatment and/or neoadjuvant treatment of a disease related to CDCP1;
preferably, the disease related to CDCP1 is a tumor; more preferably, the tumor is selected from a solid tumor or a hematological tumor;
further more preferably, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; the hematological tumor is selected from lymphoma, myeloma (e.g., multiple myeloma) or leukemia.

25. The antibody or the antigen-binding fragment thereof according to any one of claims 1 to 6, or the multispecific antibody according to claim 7, or the conjugate according to claim 12 or 13, or the pharmaceutical composition according to claim 14, for use in the diagnosis or differential diagnosis of a disease related to CDCP1;
preferably, the disease related to CDCP1 is a tumor; more preferably, the tumor is selected from a solid tumor or a hematological tumor; further more preferably, the solid tumor is selected from esophageal cancer, gastrointestinal cancer, pancreatic cancer, thyroid cancer, colorectal cancer, kidney cancer, lung cancer (e.g., lung adenocarcinoma, lung squamous cell cancer, or small cell lung cancer), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell cancer, bone cancer, skin cancer, thymic cancer, cholangiocarcinoma, gallbladder cancer, melanoma, mesothelioma, sarcoma, or glioblastoma; the hematological tumor is selected from lymphoma, myeloma (e.g., multiple myeloma) or leukemia.
